# EUROPEAN PATENT SPECIFICATION

(11) **EP 4 480 545 B1**
(45) Date of publication and mention of the grant of the patent: **04.11.2026**
(21) Application number: 24192826.6
(22) Date of filing: 27.04.2023
(51) Int. Cl.: A61K 9/20, A61P 43/00

(54) **FORMULATIONS OF A PLASMA KALLIKREIN INHIBITOR**
FORMULIERUNGEN EINES PLASMAKALLIKREINHEMMERS
FORMULATIONS D'UN INHIBITEUR DE KALLICRÉINE PLASMATIQUE

(30) Priority: 27.04.2022 US 202263335387 P
(43) Date of publication of application: 25.12.2024
(62) Document number(s) of the earlier application(s) in accordance with Art. 76 EPC: 23722929.9 / 4 288 036
(73) Proprietor: Kalvista Pharmaceuticals Limited, Porton Down, Salisbury SP4 0BF Wiltshire (GB)
(72) Inventor: COOPER, John Alexander, Salisbury SP4 0BF (GB); CROADSHAW, Oliver William, Salisbury SP4 0BF (GB); IVERSON, Matthew Scott, Cottonwood Heights 84093 (US); SCHIANO, Serena, Salisbury SP4 0BF (GB); SMITH, Michael David, Salt Lake City 84109 (US); ARROYO, Ana Catalina Ferreira, Lawrenceville 08648 (US)
(74) Representative: Carpmaels & Ransford LLP

(56) References cited:
- WO-A1-2022/079446
- MAETZEL ANDREAS ET AL: "KVD900, an oral on-demand treatment for hereditary angioedema: Phase 1 study results", JOURNAL OF ALLERGY AND CLINICAL IMMUNOLOGY, ELSEVIER, AMSTERDAM, NL, vol. 149, no. 6, 24 January 2022 (2022-01-24), pages 2034 - 2042, XP087084685, ISSN: 0091-6749, [retrieved on 20220124], DOI: 10.1016/J.JACI.2021.10.038
- HAMPTON SALLY L ET AL: "16 KVD900 as a Single Dose, Rapid, Oral Plasma Kallikrein Inhibitor for the On-Demand Treatment of Hereditary Angioedema Attacks: Pharmacokinetic and Pharmacodynamic results from a Phase 1 Single Ascending Dose Study", 28 February 2019 (2019-02-28), XP093058918, Retrieved from the Internet <URL:https://www.sciencedirect.com/science/article/pii/S0091674918318608/pdfft?md5=93eaffb89be7a60010ba60f8a0727597&pid=1-s2.0-S0091674918318608-main.pdf> [retrieved on 20230628]
- VISHALI T ET AL: "Orodispersible Tablets: A Review", RESEARCH JOURNAL OF PHARMACY AND TECHNOLOGY, vol. 13, no. 5, 1 January 2020 (2020-01-01), India, pages 2522, XP093110679, ISSN: 0974-3618, DOI: 10.5958/0974-360X.2020.00449.7

## Description

The present invention relates to orodispersible tablets as set out in the appended claims, and their use in treatments of bradykinin-mediated angioedema with a plasma kallikrein inhibitor. In particular, the present invention provides pharmaceutical dosage forms in the form of an orodispersible tablet comprising a plasma kallikrein inhibitor, mixtures for use in manufacturing processes for producing the orodispersible tablets of the invention and the uses of such orodispersible tablets. The orodispersible tablets of the present invention are particularly suitable for patients who may struggle to swallow tablets. The present invention provides orodispersible tablets as set out in the appended claims, and their use in on-demand treatments of bradykinin-mediated angioedema by orally administering a plasma kallikrein inhibitor to a patient in need thereof in the form of an orodispersible tablet.

### BACKGROUND TO THE INVENTION

Inhibitors of plasma kallikrein have a number of therapeutic applications, particularly in the treatment of bradykinin-mediated angioedema such as hereditary angioedema and bradykinin-mediated angioedema non-hereditary (BK-AEnH).

Plasma kallikrein is a trypsin-like serine protease that can liberate kinins from kininogens (see K. D. Bhoola et al., "Kallikrein-Kinin Cascade", Encyclopedia of Respiratory Medicine, p483-493; J. W. Bryant et al., "Human plasma kallikrein-kinin system: physiological and biochemical parameters" Cardiovascular and haematological agents in medicinal chemistry, 7, p234-250, 2009; K. D. Bhoola et al., Pharmacological Rev., 1992, 44, 1; and D. J. Campbell, "Towards understanding the kallikrein-kinin system: insights from the measurement of kinin peptides", Brazilian Journal of Medical and Biological Research 2000, 33, 665-677). It is an essential member of the intrinsic blood coagulation cascade, although its role in this cascade does not involve the release of bradykinin or enzymatic cleavage. Plasma prekallikrein is encoded by a single gene and can be synthesized in the liver, as well as other tissues. It is secreted by hepatocytes as an inactive plasma prekallikrein that circulates in plasma as a heterodimer complex bound to high molecular weight kininogen (HK) which is activated to give the active plasma kallikrein. This contact activation system (or contact system) can be activated by negatively charged surfaces that activate Factor XII (FXII) to Factor Xlla (FXlla), by certain proteases e.g. plasmin (Hofman et al., Clin Rev Allergy Immunol 2016), which may not require negative surfaces, or by misfolded proteins (Maas et al., J Clinical Invest 2008). FXlla mediates conversion of plasma prekallikrein to plasma kallikrein and the subsequent cleavage of high molecular weight kininogen (HK) to generate bradykinin, a potent inflammatory hormone. Kinins are potent mediators of inflammation that act through G protein-coupled receptors and antagonists of kinins (such as bradykinin receptor antagonists) have previously been investigated as potential therapeutic agents for the treatment of a number of disorders (F. Marceau and D. Regoli, Nature Rev., Drug Discovery, 2004, 3, 845-852).

Plasma kallikrein is thought to play a role in a number of inflammatory disorders. The major inhibitor of plasma kallikrein is the serpin C1 esterase inhibitor. Patients who present with a genetic deficiency in C1 esterase inhibitor suffer from hereditary angioedema (HAE) which results in intermittent swelling of face, hands, throat, gastro-intestinal tract and genitals. Blisters formed during acute episodes contain high levels of plasma kallikrein which cleaves high molecular weight kininogen (HK) liberating bradykinin leading to increased vascular permeability. Treatment with a large protein plasma kallikrein inhibitor has been shown to effectively treat HAE by preventing the release of bradykinin which causes increased vascular permeability (A. Lehmann "Ecallantide (DX-88), a plasma kallikrein inhibitor for the treatment of hereditary angioedema and the prevention of blood loss in on-pump cardiothoracic surgery" Expert Opin. Biol. Ther. 8, p1187-99). However, risks of anaphylactic reactions have been reported for Ecallantide.

Hereditary angioedema is a rare inherited disorder characterised by recurrent acute attacks where fluids accumulate outside of the blood vessels, blocking the normal flow of blood or lymphatic fluid and causing rapid swelling of tissues such as in the hands, feet, limbs, face, intestinal tract, or airway. "Hereditary angioedema" can thus be defined as any disorder characterised by recurrent episodes of bradykinin-mediated angioedema (e.g. severe swelling) caused by an inherited dysfunction/fault/mutation. There are currently three known categories of HAE: (i) HAE type 1, (ii) HAE type 2, and (iii) normal C1 inhibitor HAE (normal C1-Inh HAE). However, the HAE field is developing quickly and further types of HAE might be defined in the future.

Without wishing to be bound by theory, it is thought that HAE type 1 is caused by mutations in the *SERPING1* gene that lead to reduced levels of C1 inhibitor in the blood. Without wishing to be bound by theory, it is thought that HAE type 2 is caused by mutations in the *SERPING1* gene that lead to dysfunction of the C1 inhibitor in the blood. Without wishing to be bound by theory, the cause of normal C1-Inh HAE is less well defined and the underlying genetic dysfunction/fault/mutation can sometimes remain unknown. What is known is that the cause of normal C1-Inh HAE is not related to reduced levels or dysfunction of the C1 inhibitor (in contrast to HAE types 1 and 2). Normal C1-Inh HAE can be diagnosed by reviewing the family history and noting that angioedema has been inherited from a previous generation (and thus it is hereditary angioedema). Normal C1-Inh HAE can also be diagnosed by determining that there is a dysfunction/fault/mutation in a gene other than those related to C1 inhibitor. For example, it has been reported that dysfunction/fault/mutation with plasminogen can cause normal C1-Inh HAE (see e.g. Veronez et al., Front Med (Lausanne). 2019 Feb 21;6:28. doi: 10.3389/fmed.2019.00028; or Recke et al., Clin Transl Allergy. 2019 Feb 14;9:9. doi: 10.1186/s13601-019-0247-x.). It has also been reported that dysfunction/fault/mutation with Factor XII can cause normal C1-Inh HAE (see e.g. Mansi et al., The Association for the Publication of the Journal of Internal Medicine Journal of Internal Medicine, 2015, 277; 585-593; or Maat et al. J Thromb Haemost. 2019 Jan;17(1):183-194. doi: 10.1111/jth.14325).

Acute HAE attacks normally progress through three key clinically distinct stages: an initial prodromal stage (that can typically last for up to 12 hours), followed by a swelling stage, and then an absorption stage. A majority of HAE attacks announce themselves with prodromal symptoms. Two thirds of prodromes appeared less than 6 hours before a HAE attack and no prodromes occur more than 24 hours before a HAE attack (Magerl et al., Clinical and Experimental Dermatology 2014, 39, 298-303). For example, the following prodromal symptoms may start to be observed: a slight swelling (particularly affecting the face and neck), a typical type of abdominal pain, a typical reddening of the skin called "erythema marginatum". An attack is fully developed when it has reached maximum swelling and maximum expression of pain (e.g. abdominal attack), discomfort (e.g. peripheral attack) or threat to life (e.g. laryngeal attack). Once the attack has reached its peak, the subsequent time period to normalization is determined by the time it takes for the swelling to disappear and the liquid that has penetrated the tissues to be reabsorbed.

However, angioedemas are not necessarily inherited. Indeed, another class of angioedema is bradykinin-mediated angioedema non-hereditary (BK-AEnH), which is not caused by an inherited genetic dysfunction/fault/mutation. Often the underlying cause of BK-AEnH is unknown and/or undefined. However, the signs and symptoms of BK-AEnH are similar to those of HAE, which without being bound by theory, is thought to be on account of the shared bradykinin-mediated pathway between HAE and BK-AEnH. Specifically, BK-AEnH is characterised by recurrent acute attacks where fluids accumulate outside of the blood vessels, blocking the normal flow of blood or lymphatic fluid and causing rapid swelling of tissues such as in the hands, feet, limbs, face, intestinal tract, airway or genitals. BK-AEnH attacks are acute and normally progress through three key clinically distinct stages: an initial prodromal stage (that can typically last for up to 12 hours), followed by a swelling stage, and then an absorption stage. A majority of attacks announce themselves with prodromal symptoms. Two thirds of prodromes appeared less than 6 hours before an attack and no prodromes occur more than 24 hours before an attack (Magerl et al. Clinical and Experimental Dermatology (2014) 39, pp298-303). For example, the following prodromal symptoms may start to be observed: a slight swelling (particularly affecting the face and neck), a typical type of abdominal pain, a typical reddening of the skin called "erythema marginatum". An attack is fully developed when it has reached maximum swelling and maximum expression of pain (e.g. abdominal attack), discomfort (e.g. peripheral attack) or threat to life (e.g. laryngeal attack). Once the attack has reached its peak, the subsequent time period to normalization is determined by the time it takes for the swelling to disappear and the liquid that has penetrated the tissues to be reabsorbed.

Specific types of BK-AEnH include: non-hereditary angioedema with normal C1 Inhibitor (AE-nC1 Inh), which can be environmental, hormonal, or drug-induced; acquired angioedema; anaphylaxis associated angioedema; angiotensin converting enzyme (ACE) inhibitor-induced angioedema; dipeptidyl peptidase-4 inhibitor-induced angioedema; and tPA-induced angioedema (tissue plasminogen activator-induced angioedema). However, reasons why these factors and conditions cause angioedema in only a relatively small proportion of individuals are unknown.

Environmental factors that can induce AE-nC1 Inh include air pollution (Kedarisetty et al., Otolaryngol Head Neck Surg. 2019 Apr 30:194599819846446. doi: 10.1177/0194599819846446) and silver nanoparticles such as those used as antibacterial components in healthcare, biomedical and consumer products (Long et al., Nanotoxicology. 2016;10(4):501-11. doi: 10.3109/17435390.2015.1088589).

Various publications suggest a link between the bradykinin and contact system pathways and BK-AEnHs, and also the potential efficacy of treatments, see e.g.: Bas et al. (N Engl J Med 2015); Leibfried and Kovary. (J Pharm Pract 2017); van den Elzen et al. (Clinic Rev Allerg Immunol 2018); Han et al. (JCI 2002).

tPA-induced angioedema is discussed in various publications as being a potentially life-threatening complication following thrombolytic therapy in acute stroke victims (see e.g. Simão et al., Blood. 2017 Apr 20;129(16):2280-2290. doi: 10.1182/blood-2016-09-740670; Fröhlich et al., Stroke. 2019 Jun 11:STROKEAHA119025260. doi: 10.1161/STROKEAHA.119.025260; Rathbun, Oxf Med Case Reports. 2019 Jan 24;2019(1):omy112. doi: 10.1093/omcr/omy112; Lekoubou et al., Neurol Res. 2014 Jul;36(7):687-94. doi: 10.1179/1743132813Y.0000000302; Hill et al., Neurology. 2003 May 13;60(9):1525-7).

Stone et al. (Immunol Allergy Clin North Am. 2017 Aug;37(3):483-495.) reports that certain drugs can cause angioedema.

Scott et al. (Curr Diabetes Rev. 2018;14(4):327-333. doi: 10.2174/1573399813666170214113856) reports cases of dipeptidyl Peptidase-4 Inhibitor induced angioedema.

Hermanrud et al. (BMJ Case Rep. 2017 Jan 10;2017. pii: bcr2016217802) reports recurrent angioedema associated with pharmacological inhibition of dipeptidyl peptidase IV and also discusses acquired angioedema related to angiotensin-converting enzyme inhibitors (ACEI-AAE). Kim et al. (Basic Clin Pharmacol Toxicol. 2019 Jan;124(1):115-122. doi: 10.1111/bcpt.13097) reports angiotensin II receptor blocker (ARB)-related angioedema. Reichman et al., (Pharmacoepidemiol Drug Saf. 2017 Oct;26(10):1190-1196. doi: 10.1002/pds.4260) also reports angioedema risk for patients taking ACE inhibitors, ARB inhibitors and beta blockers. Diestro et al. (J Stroke Cerebrovasc Dis. 2019 May;28(5):e44-e45. doi: 10.1016/j.jstrokecerebrovasdis.2019.01.030) also reports a possible association between certain angioedemas and ARBs.

Giard et al. (Dermatology. 2012;225(1):62-9. doi: 10.1159/000340029) reports that bradykinin-mediated angioedema can be precipitated by oestrogen contraception.

Synthetic and small molecule plasma kallikrein inhibitors have been described previously, for example by Garrett et al. ("Peptide aldehyde...." J. Peptide Res. 52, 62-71 (1998)), T. Griesbacher et al. ("Involvement of tissue kallikrein but not plasma kallikrein in the development of symptoms mediated by endogenous kinins in acute pancreatitis in rats" British Journal of Pharmacology 137, 692-700 (2002)), Evans ("Selective dipeptide inhibitors of kallikrein" WO03/076458), Szelke et al. ("Kininogenase inhibitors" WO92/04371), D. M. Evans et al. (Immunolpharmacology, 32, p115-116 (1996)), Szelke et al. ("Kininogen inhibitors" WO95/07921), Antonsson et al. ("New peptides derivatives" WO94/29335), J. Corte et al. ("Six membered heterocycles useful as serine protease inhibitors" WO2005/123680), J. Stürzbecher et al. (Brazilian J. Med. Biol. Res 27, p1929-34 (1994)), Kettner et al. (US 5,187,157), N. Teno et al. (Chem. Pharm. Bull. 41, p1079-1090 (1993)), W. B. Young et al. ("Small molecule inhibitors of plasma kallikrein" Bioorg. Med. Chem. Letts. 16, p2034-2036 (2006)), Okada et al. ("Development of potent and selective plasmin and plasma kallikrein inhibitors and studies on the structure-activity relationship" Chem. Pharm. Bull. 48, p1964-72 (2000)), Steinmetzer et al. ("Trypsin-like serine protease inhibitors and their preparation and use" WO08/049595), Zhang et al. ("Discovery of highly potent small molecule kallikrein inhibitors" Medicinal Chemistry 2, p545-553 (2006)), Sinha *et al.* ("Inhibitors of plasma kallikrein" WO08/016883), Shigenaga et al. ("Plasma Kallikrein Inhibitors" WO2011/118672), and Kolte et al. ("Biochemical characterization of a novel high-affinity and specific kallikrein inhibitor", British Journal of Pharmacology (2011), 162(7), 1639-1649). Also, Steinmetzer et al. ("Serine protease inhibitors" WO2012/004678) describes cyclized peptide analogs which are inhibitors of human plasmin and plasma kallikrein.

As explained above, HAE can manifest in patients who present with a genetic deficiency or dysfunction in C1 esterase inhibitor. Thus, some of the current treatments of HAE involve administering a C1 esterase inhibitor to normalise the deficiency or dysfunction in C1 esterase inhibitor. Such treatments can be prophylactic (*i.e.* administered in the absence of acute HAE attack symptoms to prevent/reduce the likelihood of an acute HAE attack) and/or acute treatments (*i.e.* administered when acute HAE attack symptoms are noticed to try to stop or reduce the severity of the acute HAE attack).

Cinryze^{®} and Haegarda^{®} contain a C1 esterase inhibitor and are authorised to prevent acute HAE attacks (i.e. prophylactic treatment). Treatment with Cinryze^{®} requires the preparation of a solution from a powder, which is then injected every 3 or 4 days. Similarly, treatment with Haegarda^{®} requires the preparation of a solution from a powder, which is then injected twice a week. It is not always possible for a patient to self-administer these treatments, and if this is the case, the patient is required to visit a clinic for treatment. Thus, both of these prophylactic treatments suffer from high patient burden. Additionally, the FDA packet insert for Haegarda^{®} states that it "should not be used to treat an acute HAE attack", and therefore a patient may require additional therapy if a HAE attack develops.

Berinert^{®} and Ruconest^{®} contain a C1 esterase inhibitor and are authorised to treat acute HAE attacks. Both of these treatments also involve the preparation of an injectable solution followed by injection. This process can be burdensome on the patient, especially when the patient is suffering from an acute HAE attack. Self-administration of the dosage amount is also not always possible, and if it is not, administration of the drug can be substantially delayed thus increasing the severity of the acute HAE attack for the patient.

Kalbitor^{®} (active substance ecallantide) and Takhzyro^{®} (active substance lanadelumab) are selective plasma kallikrein inhibitors approved for medical use in the treatment of HAE. Both treatments are formulated as solutions for injection. Ecallantide is a large protein plasma kallikrein inhibitor that presents a risk of anaphylactic reactions. Indeed, the EU marketing authorisation application for Kalbitor^{®} has recently been withdrawn because the benefits of Kalbitor^{®} are said to not outweigh its risks. Lanadelumab is a recombinant fully human IgG1 kappa light chain monoclonal antibody. Reported adverse reactions of treatment with lanadelumab include hypersensitivity, injection site pain, injection site erythema, and injection site bruising. The authorised EMA label for Takhzyro^{®} (active substance lanadelumab) states that it "is not intended for treatment of acute HAE attacks" and that "in case of a breakthrough HAE attack, individualized treatment should be initiated with an approved rescue medication". Also, as injections, both of these treatments involve a high patient burden.

Berotralstat (BCX7353) has been approved in some countries for preventative treatment of HAE (not as an on-demand treatment), e.g. under the brand name Orladeyo^{®}. Hwang et al. (Immunotherapy (2019) 11(17), 1439-1444) states that higher doses were associated with more gastrointestinal adverse effects indicating increased toxicity at higher levels.

Furthermore, many molecules in the known art feature a highly polar and ionisable guanidine or amidine functionality. It is well known that such functionalities may be limiting to gut permeability and therefore to oral availability. For example, it has been reported by Tamie J. Chilcote and Sukanto Sinha ("ASP-634: An Oral Drug Candidate for Diabetic MacularEdema", ARVO 2012 May 6th - May 9th, 2012, Fort Lauderdale, Florida, Presentation 2240) that ASP-440, a benzamidine, suffers from poor oral availability. It is further reported that absorption may be improved by creating a prodrug such as ASP-634. However, it is well known that prodrugs can suffer from several drawbacks, for example, poor chemical stability and potential toxicity from the inert carrier or from unexpected metabolites. In another report, indole amides are claimed as compounds that might overcome problems associated with drugs possessing poor or inadequate ADME-tox and physicochemical properties although no inhibition against plasma kallikrein is presented or claimed (Griffioen et al., "Indole amide derivatives and related compounds for use in the treatment of neurodegenerative diseases", WO2010142801).

Other plasma kallikrein inhibitors known in the art are generally small molecules, some of which include highly polar and ionisable functional groups, such as guanidines or amidines. Recently, plasma kallikrein inhibitors that do not feature guanidine or amidine functionalities have been reported. For example Brandl et al. ("N-((6-amino-pyridin-3-yl)methyl)-heteroaryl-carboxamides as inhibitors of plasma kallikrein" WO2012/017020), Evans et al. ("Benzylamine derivatives as inhibitors of plasma kallikrein" WO2013/005045), Allan et al. ("Benzylamine derivatives" WO2014/108679), Davie et al. ("Heterocyclic derivates" WO2014/188211), Davie et al. ("N-((het)arylmethyl)-heteroaryl-carboxamides compounds as plasma kallikrein inhibitors" WO2016/083820).

The applicant has developed a novel series of compounds that are inhibitors of plasma kallikrein, which are disclosed in WO2016/083820 (PCT/GB2015/053615). These compounds demonstrate good selectivity for plasma kallikrein. One such compound is N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide. Certain dosage forms comprising this compound are disclosed in Collett et al. WO2019/106361. The name N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide denotes the structure depicted below, which is known by its codename "KVD900". The INN for KVD900 is sebetralstat.

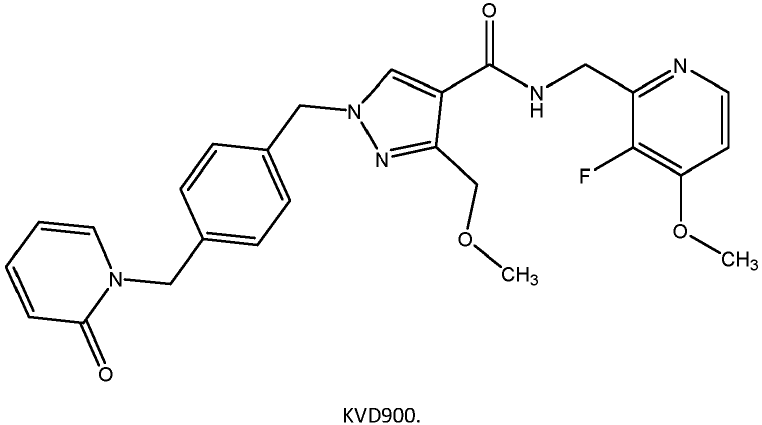

As noted, hereditary angioedema (HAE) is a rare inherited condition characterised by recurrent attacks of severe swelling. The swelling is typically of the face, hands, throat, gastro-intestinal tract and/or genitals. Attacks of swelling can be very unpleasant and dangerous if untreated, sometimes causing abdominal pain, diarrhoea, vomiting and even death. Existing treatments of HAE are either administered on-demand or as a prophylactic. On-demand treatment is administered in response to a specific attack to lessen its severity e.g. at the first sign of attack symptoms. Prophylactic treatment involves continuously and regularly taking treatment to reduce the likelihood of an attack occurring i.e. not in response to one specific attack. Prophylactic treatments are generally not completely effective, with patients receiving prophylactic treatment often suffering from breakthrough attacks. For this reason, it is common for HAE patients on prophylactic treatment to have an on-demand treatment as a backup for such breakthrough attacks (such treatment is often called a "rescue treatment"). Accordingly, all HAE patients, regardless of their current treatment regime (or lack thereof) would benefit from having effective on-demand treatments of HAE, which is a need addressed by the present invention.

There is therefore a need for a treatment of bradykinin-mediated angioedema (e.g. HAE or BK-AEnH) that is less burdensome on the patient to improve patient compliance. In particular, there is a need for a treatment of bradykinin-mediated angioedema (e.g. HAE or BK-AEnH) that can be administered orally. HAE attacks resolve faster and are shorter after early treatment (Maurer M et al. PLoS ONE 2013;8(2): e53773. doi:10.1371/journal.pone.0053773) and thus early intervention when an attack is expected, or ongoing, is essential to desirably manage the disease. Therefore, there is also a need for an oral treatment of acute bradykinin-mediated angioedema (e.g. HAE or BK-AEnH) attacks on-demand e.g. upon recognition of symptoms of an acute bradykinin-mediated angioedema (e.g. HAE or BK-AEnH) attack and does not require regular (or continuous) dosing e.g. a treatment that does not require injections twice a week. In particular there is a need for an effective oral treatment in young patients (e.g. between the ages of 2 and less than 18). Existing injectable treatments can cause pain at the injection site, and the injection itself can cause the child stress. Moreover, there is a need for specific oral dosage forms that are more suitable for patients who may struggle to swallow tablets (e.g. children, the elderly, patients suffering from a laryngeal attack or from conditions such as dysphagia).

To date, there is a lack of approved oral treatments for bradykinin-mediated angioedema (e.g. HAE or BK-AEnH) (see e.g. Craig et al. Int Arch Allergy Immunol. 2014;165(2):119-27. doi: 10.1159/000368404; Mager et al., Immunol Allergy Clin North Am. 2017 Aug;37(3):571-584). There are no approved oral on-demand treatments for bradykinin-mediated angioedema disorders (e.g. HAE or BK-AEnH). Instead, the majority of available treatments are injectables. Injectables suffer from many disadvantages compared to oral treatments, largely relating to patient compliance. For example, injectables are more inconvenient than oral treatments e.g. needing to be in a suitable environment to formulate and then administer the injection, and also causing pain at the injection site. Reduced patient compliance can lead to patients delaying treatment, which generally leads to attack symptoms getting worse, or even avoiding treatment altogether. Indeed, many patients suffering from HAE simply hope that any attack will dissipate on its own to avoid administering an injection. Injectable treatments suffer from late dosing because the patient may need to prepare the dosage form or even travel to hospital for treatment. Therefore, HAE treatment is often undermined by late dosing caused by the high burden on the patient. Indeed, Maurer M et al. (PLoS ONE 2013;8(2): e53773. doi:10.1371/journal.pone.0053773) explains that more than 60% of patients administer their HAE injectable more than one hour after the onset of an attack. HAE drugs are often used off-label to treat some types of BK-AEnH, so the problems associated with HAE treatments apply similarly to treatment of BK-AEnH.

Icatibant (marketed as Firazyr^{®}) is the predominant on-demand treatment for HAE, and is administered by subcutaneous injection. In a real-world study of HAE patients with access to icatibant (Maurer M et al. PLoS ONE 2013;8(2): e53773. doi:10.1371/journal.pone.0053773), less than 40% of treated attacks were dosed within an hour of onset, with 30% not being treated until over 5 hours after onset. Further, 45% of attacks in this study were not treated at all. Therefore, there is an unmet need to provide patients with improved treatment options to treat HAE attacks on-demand.

As noted above, there is a lack of any approved oral on-demand treatments for bradykinin-mediated angioedema disorders such as HAE and BK-AEnH. KVD900 is currently in phase 3 clinical trials (known as the KONFIDENT study, https://konfidentstudy.com/), having met all of its phase 2 efficacy and safety endpoints (see clinical trial NCT04208412). KVD900 was administered as a swallowable tablet in these clinical studies.

Nevertheless, there is a continued need to provide treatment options that provide early intervention, which is known to shorten the duration of the bradykinin-mediated attacks (e.g. HAE attacks) and the lead to faster attack resolution. Further, there is a continued need to provide treatment options for people that may have difficulty swallowing, e.g. due to young or old age, during a laryngeal attack, where the patient does not have access to water and/or where the patient is suffering from a condition such as dysphagia. Further, during abdominal attacks, there is a risk of vomiting which leads to uncertainty about how much of the dose is absorbed, and the act of drinking water itself, which is desirable for swallowing tablets, can often exacerbate the feeling of needing to vomit.

WO 2022/079446 A1 relates to treatment of bradykinin-mediated angioedema with a plasma kallikrein inhibitor, oral modified release pharmaceutical dosage forms comprising a plasma kallikrein inhibitor, and the uses of such dosage forms.

Maetzel Andreas et al., "KVD900, an oral on-demand treatment for hereditary angioedema: Phase 1 study results*"* discusses a study to evaluate the safety, tolerability and pharmacokinetics (PK) and pharmacodynamics (PD) of KVD900 in healthy adults.

Hampton Sally L et al., "KVD900 as a Single Dose, Rapid, Oral Plasma Kallikrein Inhibitor for the On-Demand Treatment of Hereditary Angioedema Attacks: Pharmacokinetic and Pharmacodynamic results from a Phase 1 Single Ascending Dose Study" discusses pharmacodynamic (PD) effects of orally administered KVD900 evaluated using *ex vivo* whole plasma assays for plasma kallikrein catalytic activity and high molecular weight kininogen (HK) cleavage.

### DESCRIPTION OF THE INVENTION

The present invention provides an orodispersible tablet comprising between 26 and 50 wt% of KVD900 (or a pharmaceutically acceptable salt and/or solvate thereof)
wherein KVD900 is,

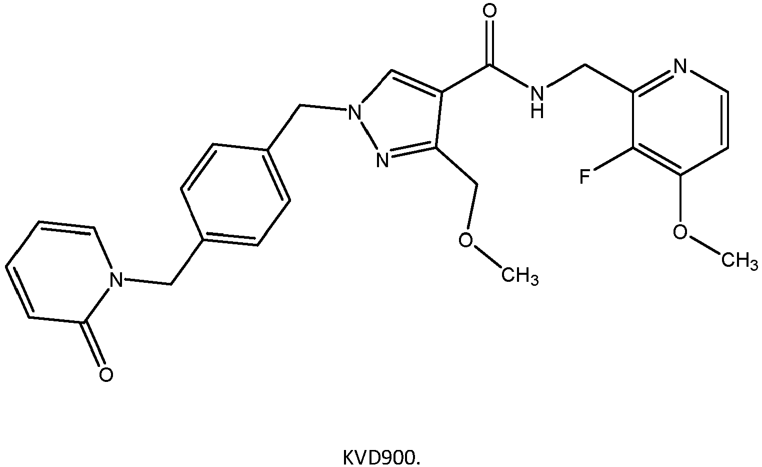

The scope of the invention is defined by the appended claims.

The present invention aims to solve the above problems, and in particular, to provide orodispersible tablets as set out in the appended claims, and their use in treatments of bradykinin-mediated angioedema disorders such as HAE and BK-AEnH that are especially suitable for patients that require a dosage form that is easy to administer orally. Such patients include young patients (e.g. those aged between 2 and less than 18, particularly those aged between 2 and less than 12), elderly patients (e.g. those aged 70 years or older), patients that often suffer from bradykinin-mediated attacks that are laryngeal attacks, patients that regularly might not have fluid (e.g. water) readily available to aid the swallowing of an oral dosage form, or patients that suffer from a condition such as dysphagia. Orodispersible tablets are also beneficial for other patients who prefer the convenience of easily swallowable dosage forms and for treatment of patients when compliance may be difficult (e.g. for psychiatric disorders).

The present invention addresses these needs by providing the first orodispersible tablet (ODT) for on-demand treatment of bradykinin-mediated angioedema disorders such as HAE and BK-AEnH. The orodispersible tablets of the present invention allow for early intervention and have been developed to reduce the barriers to treatment.

Orodispersible tablets (e.g. an orodispersible mini-tablet) are intended to be placed directly in the mouth where they rapidly disintegrate in saliva before they are swallowed. An ODT is not a swallowable tablet, for instance a conventional swallowable tablet or capsule.

There are several challenges in the development of ODT formulations, such as the need for ODTs to have sufficient mechanical strength whilst also providing rapid disintegration times. In particular, since ODTs must have a feasible tablet size and drug loading, ODTs are limited by the amount of drug that can be incorporated into each unit dose, and thus a drug with a high dose strength (>200 mg) would be less suitable for an ODT. This posed a particularly challenging development process for KVD900 because the dose of KVD900 proven to be safe and effective in phase 2 was 600 mg, and there is a need to provide this dose in as few unit dosage amounts as possible to ensure patient compliance.

Further, KVD900 was also particularly difficult to formulate into an ODT because it has been classified as a BCS class **IV** compound (in line with ICH guideline: BIOPHARMACEUTICS CLASSIFICATION SYSTEM-BASED BIOWAIVERS M9 Final version Adopted on 20 November 2019). BCS class **IV** drugs are considered to be poorly soluble and poorly permeable meaning that they are likely to have poor oral bioavailability and/or the oral absorption may be so low that they cannot be given by the oral route. Due to their numerous unfavourable characteristics, BCS class IV drugs are particularly challenging to formulate as oral dosage forms and in particular as ODTs. Indeed, ODTs of BCS class IV drugs are not generally known in the art.

The applicant has managed to overcome these difficulties and has provided an ODT of KVD900, which are the ODTs described herein as part of the invention. These ODTs have been manufactured and administered to humans in a phase 1 study.

For an ODT to be viable for clinical use, it must be manufacturable on scale. To do this, the mixture of active ingredient and excipients must be sufficiently processable and flowable, while providing an ODT that meets the requirements described above. These depend on the specific physico-chemical properties of the drug candidate including its compressibility, solubility and particle size, the desired pharmacokinetic and pharmacodynamic properties and the flow properties of the drug and excipients. These properties are unique to the active ingredient and cannot be accurately predicted before formulation development is carried out. In addition, ODTs must have a pleasant taste, must not disintegrate into larger particles in the oral cavity and should be formulated to leave minimal or no residue in mouth after oral administration.

When developing the ODTs of the present invention, the inventors encountered problems when trying to provide a suitable ODT. Initial attempts to manufacture an ODT of KVD900 were unsuccessful, with the inventors encountering a degradation problem with KVD900 as well as processability problems such as insufficient flowability. The inventors solved these problems, for instance by using the mixtures of KVD900 and excipients and the methods of manufacturing the ODTs described herein.

As explained above, KVD900 has already proven to be safe and effective, meeting all of its phase 2 safety and efficacy endpoints. It is advantageous for a subsequent ODT formulation to have a pharmacokinetic and pharmacodynamic profile that is comparable to formulations of KVD900 that have already been tested in clinical trials (e.g. the immediate release (IR) formulations of KVD900 used in the phase 2 and phase 3 studies described herein).

### Definitions

As used herein, and unless explicitly specified otherwise, the term "compound" means "KVD900". KVD900 may be present as a pharmaceutically acceptable salt and/or solvate thereof. The structure of KVD900 is shown below and is named: N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide.

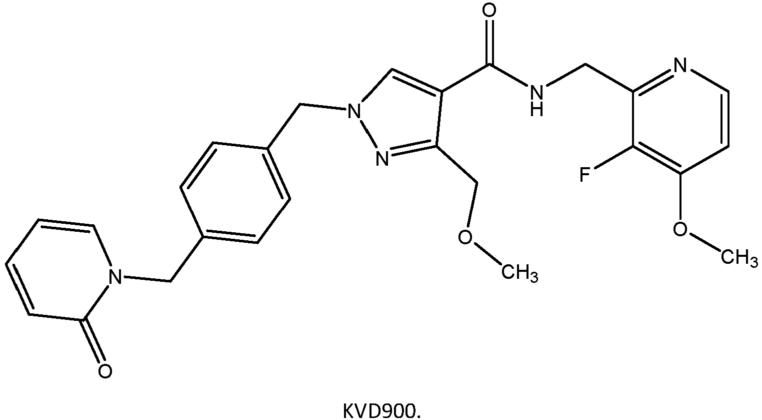

KVD900 used in the present invention can also be in the preferred crystalline form (Form 1), which exhibits at least the following characteristic X-ray powder diffraction peaks (Cu Kα radiation, expressed in degrees 2θ) at approximately:
(1) 11.2, 12.5, 13.2, 14.5 and 16.3; or
(2) 11.2, 12.5, 13.2, 14.5, 16.3, 17.4 and 17.9; or
(3) 11.2, 12.5, 13.2, 14.5, 16.3, 17.4, 17.9, 21.2 and 22.0.

The term "approximately" means in this context that there is an uncertainty in the measurements of the degrees 2θ of ± 0.3 (expressed in degrees 2θ), preferably ± 0.2 (expressed in degrees 2θ).

Form 1 can also have an X ray powder diffraction pattern comprising characteristic peaks (expressed in degrees 2θ) at approximately 4.4, 11.2, 12.5, 13.2, 14.5, 16.3, 17.4, 17.9, 21.2, 22.0 and 22.6.

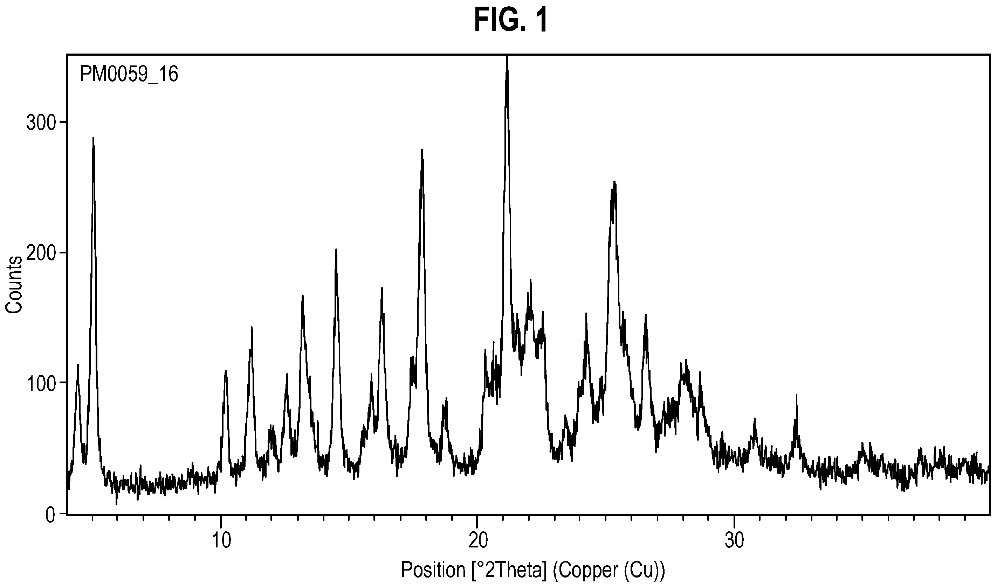

Form 1 can also have an X-ray powder diffraction pattern substantially the same as that shown in Figure 1.

It will be understood that "pharmaceutically acceptable salts and/or solvates thereof" means "pharmaceutically acceptable salts thereof", "pharmaceutically acceptable solvates thereof", and "pharmaceutically acceptable solvates of salts thereof". The preferred form of KVD900 is the free base form, specifically the anhydrous free base form (when it is possible to keep the free base anhydrous).

The term "pharmaceutically acceptable salt" means a physiologically or toxicologically tolerable salt, which can be, for example, pharmaceutically acceptable acid addition salts. For instance, as KVD900 contains a basic group, e.g. a basic nitrogen, pharmaceutically acceptable acid addition salts that can be formed include hydrochlorides, hydrobromides, sulfates, phosphates, acetates, citrates, lactates, tartrates, mesylates, succinates, oxalates, phosphates, esylates, tosylates, benzenesulfonates, naphthalenedisulphonates, maleates, adipates, fumarates, hippurates, camphorates, xinafoates, pacetamidobenzoates, dihydroxybenzoates, hydroxynaphthoates, succinates, ascorbates, oleates, bisulfates and the like. The preferred pharmaceutically acceptable salt of the compound is the hydrochloride salt.

Hemisalts of acids can also be formed, for example, hemisulfate salts.

For a review of suitable salts, see "Handbook of Pharmaceutical Salts: Properties, Selection and Use" by Stahl and Wermuth (Wiley-VCH, Weinheim, Germany, 2002).

The term "solvate" is used herein to describe a molecular complex comprising the compound of the invention and a one or more pharmaceutically acceptable solvent molecules, for example, ethanol or water. The term "hydrate" is employed when the solvent is water and for the avoidance of any doubt, the term "hydrate" is encompassed by the term "solvate".

"KVD900" may include compounds that differ only in the presence of one or more isotopically enriched atoms. For example, compounds wherein hydrogen is replaced by deuterium or tritium, or wherein carbon is replaced by ¹³C or ¹⁴C, are within the scope of the present invention.

As used herein, the term "orodispersible tablet" (ODT) means a tablet that is intended to be placed directly in the mouth where they rapidly disintegrate in saliva before they are swallowed. An ODT is not a swallowable tablet, for instance a conventional swallowable tablet or capsule.

The term "mixture" used herein in the context of the embodiments of the invention, relates to the mixtures of KVD900 and excipients used in the manufacturing process to provide ODTs of the invention. For example, the mixture of KVD900 and excipients can include a blend of KVD900 and its excipients produced prior to compression of the blend into the ODT. The "mixture" may be a powder mixture.

The terms "acute attack of bradykinin-mediated angioedema", "acute bradykinin-mediated angioedema attack", "bradykinin-mediated angioedema attack", or "attack of bradykinin-mediated angioedema" are used interchangeably herein. The "bradykinin-mediated angioedema" can be HAE or BK-AEnH. Preferably, the bradykinin-mediated angioedema is HAE, and when this is the case, the "acute attack of bradykinin-mediated angioedema" will be the "acute attack of HAE", which can be used interchangeably with "acute HAE attack", "HAE attack", or "attack of HAE".

The term "hereditary angioedema" (shortened to HAE) means any bradykinin-mediated angioedema caused by an inherited genetic dysfunction, fault, or mutation. As a result, the term "HAE" includes at least HAE type 1, HAE type 2, and normal C1 inhibitor HAE (normal C1-Inh HAE).

The term "bradykinin-mediated angioedema nonhereditary" or "BK-AEnH" is a bradykinin-mediated angioedema not caused by an inherited genetic dysfunction/fault/mutation i.e. it is not a hereditary angioedema (HAE). The underlying cause of the BK-AEnH can be unknown and/or undefined, but the signs and symptoms of BK-AEnH are similar to those of HAE, which without being bound by theory, is thought to be on account of the shared bradykinin-mediated pathway between HAE and BK-AEnH. Specific BK-AEnH that can be treated in accordance with the invention are selected from: non-hereditary angioedema with normal C1 Inhibitor (AE-nC1 Inh), which can be environmental, hormonal, or drug-induced; acquired angioedema; anaphylaxis associated angioedema; angiotensin converting enzyme (ACE or ace) inhibitor-induced angioedema; dipeptidyl peptidase-4inhibitor-induced angioedema; and tPA-induced angioedema (tissue plasminogen activator-induced angioedema).

The skilled person would understand "on-demand" treatment, in the context of bradykinin-mediated angioedema (BK-AEnH or preferably HAE), to mean that KVD900 is administered upon need of therapy in connection with one specific bradykinin-mediated angioedema attack (a BK-AEnH attack or preferably an HAE attack). As described herein, this one specific bradykinin-mediated angioedema attack (a BK-AEnH attack or preferably an HAE attack) can be ongoing (e.g. treatment is initiated upon recognition of a symptom of a bradykinin-mediated angioedema attack) or likely to occur (e.g. when the patient anticipates that a bradykinin-mediated angioedema attack might be induced or triggered). Multiple dosage amounts of KVD900 may be administered as part of the on-demand treatment, but these multiple dosages will be administered in connection with the same single bradykinin-mediated angioedema attack (a BK-AEnH attack or preferably an HAE attack). In other words, "on-demand" does not require the administration of KVD900 continuously at regular intervals (e.g. once a week, twice a week, etc.) irrespective of an instance of a bradykinin-mediated angioedema attack (a BK-AEnH attack or preferably an HAE attack). When the orodispersible tablets of the present invention are used in treatments of the invention, KVD900 is taken when the patient requires fast-acting therapeutic effects. The orodispersible tablets of the present invention can be used in particular "on-demand" treatments of the invention including: (i) treating a bradykinin-mediated angioedema attack (a BK-AEnH attack or preferably an HAE attack) on-demand, when KVD900 is administered upon recognition of a symptom of a bradykinin-mediated angioedema attack (a BK-AEnH attack or preferably an HAE attack), and (ii) prophylactically reducing the likelihood of a bradykinin-mediated angioedema attack (a BK-AEnH attack or preferably an HAE attack) on-demand, e.g. when it is anticipated that a bradykinin-mediated angioedema attack (a BK-AEnH attack or preferably an HAE attack) attack might be induced (or triggered). These are discussed below in more detail.

When the orodispersible tablets of the present invention are used in any of the treatments of the invention described herein, the patient is preferably a human. Bradykinin-mediated angioedema (e.g. BK-AEnH or preferably HAE) can affect patients of all ages. Accordingly, the human patient can be a child (ages 0 to less than 18 years) or an adult (18 years old or older). In any of the treatments described herein, the patient can have a predisposition to angioedema. Specifically, the patient can be aged 12 years and above. The patient can also be aged 2 years and above.

The human patient can be aged between 2 and less than 18. The human patient can be aged between 2 and less than 12. The human patient can be aged between 12 and less than 18. The patient can be at least 70 years old.

Accordingly, when the orodispersible tablets of the present invention are used in any of the treatments of the invention disclosed herein, particularly following a dosage amount of KVD900 of at least about 30 mg (more specifically, at least about 40 mg, at least about 50 mg, at least about 60 mg, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg or about 400 mg to about 500 mg, specifically 500 mg), in addition to inhibiting plasma kallikrein, the treatments can also reduce the cleavage of plasma prekallikrein to generate plasma kallikrein and/or reduce the generation of Factor Xlla (FXlla) following administration. Thus, in some embodiments, particularly following a dosage amount of KVD900 of at least about 30 mg (more specifically, at least about 40 mg, at least about 50 mg, at least about 60 mg, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg or about 400 mg to about 500 mg, specifically 500 mg), the treatments can block the cleavage of plasma prekallikrein to generate plasma kallikrein and/or block the cleavage of FXII to generate FXlla.

### Orodispersible tablets according to the invention

The orodispersible tablets of the present invention are advantageous because they allow for early intervention and have been developed to reduce the barriers to treatment of attacks of bradykinin-mediated angioedema (e.g. HAE). Further, the orodispersible tablets of the present invention can be made at scale because the underlying mixture/blend used to manufacture the ODTs has excellent flowability and compressibility properties.

The KVD900 in the orodispersible tablets of the present invention is administered in a dosage amount that is safe and effective, having a comparable pharmacokinetic profile to the swallowable immediate release tablets used in the phase 2 study.

The invention provides an orodispersible tablet comprising KVD900 (or a pharmaceutically acceptable salt and/or solvate thereof).

The orodispersible tablets of the invention can comprise between about 25 mg and about 1000 mg of KVD900 (or a pharmaceutically acceptable salt and/or solvate thereof). The orodispersible tablets of the invention can comprise between about 100 mg and about 1000 mg of KVD900 (or a pharmaceutically acceptable salt and/or solvate thereof). Unless explicitly stated to the contrary, the amount of KVD900 in mg in the orodispersible tablets and mixtures of the present invention is to be understood as meaning the amount of KVD900 in its free base form (i.e. not accounting for the weight of any counterion, if present). The orodispersible tablets of the invention can comprise between about 150 mg and about 1000 mg of KVD900 (or a pharmaceutically acceptable salt and/or solvate thereof). The orodispersible tablets of the invention can comprise between about 100 mg and about 700 mg of KVD900 (or a pharmaceutically acceptable salt and/or solvate thereof). The orodispersible tablets of the invention can comprise between about 200 mg and about 600 mg of KVD900 (or a pharmaceutically acceptable salt and/or solvate thereof). The orodispersible tablets of the invention can comprise between about 200 mg and about 350 mg of KVD900 (or a pharmaceutically acceptable salt and/or solvate thereof). The orodispersible tablets of the invention can comprise between about 250 mg and about 300 mg of KVD900 (or a pharmaceutically acceptable salt and/or solvate thereof). The orodispersible tablets of the invention can comprise about 250 mg of KVD900 (or a pharmaceutically acceptable salt and/or solvate thereof). Alternatively, the orodispersible tablets of the invention can comprise about 275 mg of KVD900 (or a pharmaceutically acceptable salt and/or solvate thereof). Alternatively, the orodispersible tablets of the invention can comprise about 300 mg of KVD900 (or a pharmaceutically acceptable salt and/or solvate thereof).

The orodispersible tablets of the invention can comprise between about 25 mg and about 500 mg of KVD900 (or a pharmaceutically acceptable salt and/or solvate thereof). The orodispersible tablets of the invention can comprise between about 25 mg and about 200 mg of KVD900 (or a pharmaceutically acceptable salt and/or solvate thereof). The orodispersible tablets of the invention can comprise between about 25 mg and about 150 mg of KVD900 (or a pharmaceutically acceptable salt and/or solvate thereof). The orodispersible tablets of the invention can comprise about 150 mg of KVD900 (or a pharmaceutically acceptable salt and/or solvate thereof).

The orodispersible tablets of the invention can comprise between about 25 mg and about 100 mg of KVD900 (or a pharmaceutically acceptable salt and/or solvate thereof). The orodispersible tablets of the invention can comprise between about 25 mg and about 80 mg of KVD900 (or a pharmaceutically acceptable salt and/or solvate thereof). The orodispersible tablets of the invention can comprise between about 25 mg and about 75 mg of KVD900 (or a pharmaceutically acceptable salt and/or solvate thereof). The orodispersible tablets of the invention can comprise about 75 mg of KVD900 (or a pharmaceutically acceptable salt and/or solvate thereof). Alternatively, the orodispersible tablets of the invention can comprise about 50 mg of KVD900 (or a pharmaceutically acceptable salt and/or solvate thereof). Alternatively, the orodispersible tablets of the invention can comprise about 25 mg of KVD900 (or a pharmaceutically acceptable salt and/or solvate thereof).

The orodispersible tablets of the invention comprise between 26 and 50 wt% of KVD900 (or a pharmaceutically acceptable salt and/or solvate thereof). The orodispersible tablets of the invention can comprise between 26 and about 40 wt% of KVD900 (or a pharmaceutically acceptable salt and/or solvate thereof). The orodispersible tablets of the invention can comprise between 26 and about 35 wt% of KVD900 (or a pharmaceutically acceptable salt and/or solvate thereof). The orodispersible tablets of the invention can comprise between 26 and about 30 wt% of KVD900 (or a pharmaceutically acceptable salt and/or solvate thereof). The orodispersible tablets of the invention can comprise about 30 wt% of KVD900 (or a pharmaceutically acceptable salt and/or solvate thereof).

The orodispersible tablets of the present invention can comprise one or more of a filler, a binder, a disintegrant, a lubricant, a flow enhancer, a sweetener, and a flavouring.

The orodispersible tablets of the present invention can also comprise a filler. The orodispersible tablets of the present invention can also comprise one or more fillers. The filler can be selected from isomalt, lactose and its derivatives (e.g. lactose monohydrate, spray dried lactose, anhydrous lactose), mannitol (e.g. granulated mannitol, powdered mannitol, spray-dried mannitol (e.g. Mannogem EZ^{®})), sorbitol, sucrose, starch, pregelatinized starch, and mixtures thereof. Preferably, the filler can be selected from isomalt, lactose and its derivatives (e.g. lactose monohydrate, spray dried lactose, anhydrous lactose), mannitol (e.g. granulated mannitol, powdered mannitol, spray-dried mannitol (e.g. Mannogem EZ^{®})), sorbitol and pregelatinized starch. The one or more fillers can be mannitol (e.g. granulated mannitol, powdered mannitol, spray-dried mannitol (e.g. Mannogem EZ^{®})). Even more preferably, the one or more fillers can be selected from granulated mannitol and spray-dried mannitol (e.g. Mannogem EZ^{®})). The one or more fillers can be granulated mannitol. The one or more fillers can be spray-dried mannitol (e.g. Mannogem EZ^{®}).

The one or more fillers can be present at between about 30 and about 80 wt% of the orodispersible tablet. The one or more fillers can be present at between about 40 and about 70 wt% of the orodispersible tablet. The one or more fillers can be present at between about 40 and about 65 wt% of the orodispersible tablet. The one or more fillers can be present at between about 50 and about 65 wt% of the orodispersible tablet. The one or more fillers can be present at between about 50 and about 60 wt% of the orodispersible tablet. The one or more fillers can be present at between about 50 and about 55 wt% of the orodispersible tablet. The one or more fillers can be present at between about 53 and about 54 wt% of the orodispersible tablet. Preferably, the one or more fillers can be present at about 53.7 wt% of the orodispersible tablet.

The orodispersible tablets of the present invention can also comprise a binder. The orodispersible tablets of the present invention can also comprise one or more binders. The binder can also be selected from saccharides (such as glucose, sucrose, fructose, dextrose, lactose), sugar alcohols (such as xylitol, maltitol, erythritol, sorbitol), polysaccharides (such as crystalline cellulose, microcrystalline cellulose (e.g. silicified microcrystalline cellulose), powdered cellulose, methyl cellulose, carboxymethyl cellulose and salts, hydroxypropylmethyl cellulose), corn starch, starch 1500, acacia, other natural polymers (such as gelatin), polyethylene glycol, crosslinked acrylic acid and acrylic acid copolymers (e.g. Carbopol^{®}), inorganic compounds (such as calcium carbonate), calcium phosphates (e.g. anhydrous dibasic calcium phosphate, dibasic calcium phosphate, tribasic calcium phosphate), tricalcium citrate and mixtures thereof. More specifically, the binder can be selected from glucose, lactose, corn starch, starch 1500, gelatin, acacia, methyl cellulose, carboxymethyl cellulose and salts, hydroxypropylmethyl cellulose, microcrystalline cellulose (e.g. silicified microcrystalline cellulose) and mixtures thereof. Preferably, the binder is microcrystalline cellulose or hydroxypropylmethyl cellulose. Even more preferably, the binder is silicified microcrystalline cellulose.

The one or more binders can be present at between about 5 and about 40 wt% of the orodispersible tablet. The one or more binders can be present at between about 5 and about 30 wt% of the orodispersible tablet. The one or more binders can be present at between about 5 and about 20 wt% of the orodispersible tablet. The one or more binders can be present at between about 5 and about 15 wt% of the orodispersible tablet. The one or more binders can be present at between about 5 and about 10 wt% of the orodispersible tablet. The one or more binders can be present at between about 8 and about 10 wt% of the orodispersible tablet. Preferably, the one or more binders can be present at about 10 wt% (e.g. 10.01 wt %, 9.74 wt% or 9.69 wt%) of the orodispersible tablet.

In the orodispersible tablets of the present invention, the one or more fillers and one or more binders can be present at between about 50 and about 90 wt% of the orodispersible tablet. In the orodispersible tablets of the present invention, the one or more fillers and one or more binders can be present at between about 50 and about 70 wt% of the orodispersible tablet. In the orodispersible tablets of the present invention, the one or more fillers and one or more binders can be present at between about 50 and about 65 wt% of the orodispersible tablet. In the orodispersible tablets of the present invention, the one or more fillers and one or more binders can be present at between about 55 and about 65 wt% of the orodispersible tablet. Preferably, the one or more fillers and one or more binders can be present at about 64 wt% of the orodispersible tablet. Even more preferably, the one or more fillers and one or more binders can be present about 63.7 wt% of the of the orodispersible tablet.

In the orodispersible tablets of the present invention, the wt% ratio of the one or more fillers to the one or more binders can be between about 3:1 to about 8:1. In the orodispersible tablets of the present invention, the wt% ratio of the one or more fillers to the one or more binders can be between about 4:1 to about 7:1. In the orodispersible tablets of the present invention, the wt% ratio of the one or more fillers to the one or more binders can be between about 4:1 to about 6:1. In the orodispersible tablets of the present invention, the wt% ratio of the one or more fillers to the one or more binders can be between about 5:1 to about 6:1. In the orodispersible tablets of the present invention, the wt% ratio of the one or more fillers to the one or more binders can be between about 4:1 to about 7:1. Preferably, in the orodispersible tablets of the present invention, the wt% ratio of the one or more fillers to the one or more binders can be between about 5:1 and about 5.5:1, more specifically between about 5.3:1 to about 5.4:1 (e.g. 5.37:1).

More specifically, in the orodispersible tablets of the present invention the wt% ratio of mannitol to microcrystalline cellulose is between about 3:1 to about 8:1, between about 4:1 to about 7:1, between about 4:1 to about 6:1, between about 5:1 to about 6:1 or between about 4:1 to about 7:1. Preferably, in the orodispersible tablets of the present invention the wt% ratio of mannitol to microcrystalline cellulose is between about 5:1 and about 5.5:1, more specifically between about 5.3:1 to about 5.4:1 (e.g. 5.37:1).

The orodispersible tablets of the present invention can also comprise a disintegrant. The orodispersible tablets of the present invention can also comprise one or more disintegrants. The disintegrant can be starch USP, starch 1500, cellulose derivatives (e.g. carboxymethyl cellulose and salts, hydroxypropyl methylcellulose), guar gum, alginic acid and salts, sodium starch glycolate, crosslinked cellulose (e.g. croscarmellose sodium such as those traded under the names Croscarmellose^{®}, Ac-Di-Sol^{®}, Nymce ZSX^{®}, Primellose^{®}, Solutab^{®}, Vivasol^{®}), synthetic polymers such as cross linked polyvinylpyrrolidone (e.g. crospovidone, Crospovidon M^{®}, Kollidon^{®}, Polyplasdone^{®}), soy polysaccharides, calcium silicate, pearlitol Flash (co-processed Mannitol / Maize starch) and mixtures thereof. More specifically, the disintegrant is selected from crosslinked cellulose (e.g. Croscarmellose^{®}, Ac-Di-Sol^{®}, Nymce ZSX^{®}, Primellose^{®}, Solutab^{®}, Vivasol^{®}), synthetic polymers such as cross linked polyvinylpyrrolidone (e.g. crospovidone, Crospovidon M^{®}, Kollidon^{®}, Polyplasdone^{®}), sodium starch glycolate, and mixtures thereof. Preferably, the disintegrant is selected from polyvinylpyrrolidone (crospovidone), sodium starch glycolate, croscarmellose sodium. Even more preferably, the disintegrant is polyvinylpyrrolidone (crospovidone).

The one or more disintegrants can be present at between about 1 and about 15 wt% of the orodispersible tablet. The one or more disintegrants can be present at between about 1 and about 14 wt% of the orodispersible tablet. The one or more disintegrants can be present at between about 1 and about 10 wt% of the orodispersible tablet. The one or more disintegrants can be present at between about 5 and about 10 wt% of the orodispersible tablet. The one or more disintegrants can be present at between about 1 and about 9 wt% of the orodispersible tablet. The one or more disintegrants can be present at between about 2 and about 8 wt% of the orodispersible tablet. The one or more disintegrants can be present at between about 2 and about 7 wt% of the orodispersible tablet. The one or more disintegrants can be present at between about 3 and about 6 wt% of the orodispersible tablet. The one or more disintegrants can be present at between about 4 and about 6 wt% of the orodispersible tablet. The one or more disintegrants can be present at between about 4 and about 5 wt% of the orodispersible tablet. Preferably, the one or more disintegrants can be present at between about 5 wt% of the orodispersible tablet.

The orodispersible tablets of the present invention can also comprise a lubricant. The orodispersible tablets of the present invention can also be free of lubricant. The orodispersible tablets of the present invention can also comprise a lubricant. The lubricant can be selected from magnesium stearate, calcium stearate, stearic acid, hydrogenated vegetable oil, mineral oil, glyceryl behenate, glyceryl palmitostearate, polyethylene glycol, polyoxyethylene stearates, lauryl sulphate, talc, paraffin, sodium stearyl fumarate, and mixtures thereof. Preferably, the lubricant is selected from magnesium stearate, sodium stearyl fumarate and talc. Even more preferably, the lubricant is magnesium stearate.

The lubricant can be an internal lubricant or an external lubricant. Preferably, the lubricant is an external lubricant. As used herein, an external lubricant means a lubricant that aids flow through the manufacturing process, and as such might be present in trace amounts in the final ODT, but is not actively added to the blend/mixture. Even more preferably, the only lubricant used in the orodispersible tablet of the present invention is an external lubricant.

The one or more lubricants can be present at between about 0 and about 3 wt% of the orodispersible tablet. The one or more lubricants can be present at between about 0.5 and about 3 wt% of the orodispersible tablet. The one or more lubricants can be present at between about 0.5 and about 1 wt% of the orodispersible tablet. The one or more lubricants can be present at between about 0.5 and about 1.25 wt% of the orodispersible tablet. The one or more lubricants can be present at between about 0.75 and about 1.25 wt% of the orodispersible tablet. The one or more lubricants can be present at between about 1 and about 2 wt% of the orodispersible tablet. The one or more lubricants can be present at between about 0.5 and about 1 wt% of the orodispersible tablet. Preferably, the one or more lubricants are present in a trace amount. Even more preferably, there is one lubricant and it is present in a trace amount (e.g. less than 0.05 wt%).

In a preferred orodispersible tablet of the invention, the lubricant is an external lubricant and is present in a trace amount (e.g. less than 0.05 wt%).

The orodispersible tablets of the present invention can also comprise a flow enhancer. Preferably, the flow enhancer is silicon dioxide.

The flow enhancer can be present at between about 0.5 and about 3 wt% of the orodispersible tablet. The flow enhancer can be present at between about 0.5 and about 2 wt% of the orodispersible tablet. The flow enhancer can be present at between about 0.5 and about 1 wt% of the orodispersible tablet. The flow enhancer can be present at between about 0.6 and about 0.9 wt% of the orodispersible tablet. The flow enhancer can be present at between about 0.6 and about 0.8 wt% of the orodispersible tablet. The flow enhancer can be present at between about 0.7 and about 0.8 wt% of the orodispersible tablet. The flow enhancer can be present at between about 0.6 and about 0.75 wt% of the orodispersible tablet. The flow enhancer can be present at between about 0.75 and about 0.8 wt% of the orodispersible tablet. Preferably, the flow enhancer can be present at about 0.75 wt% of the orodispersible tablet.

The orodispersible tablets of the present invention can also comprise a sweetener. The orodispersible tablets of the present invention can also comprise one or more sweeteners. The sweetener can be selected from sucralose, aspartame, sugar derivates, dextrose, polydextrose, xylitol, fructose, sucrose, lactitol, maltitol, and sodium saccharin (and mixtures thereof). Preferably, the sweetener is selected from sucralose, aspartame, sugar derivates, dextrose, polydextrose, fructose, lactitol and maltitol. Even more preferably, the sweetener is sucralose.

The one or more sweeteners can be present at between about 0.1 and about 10 wt% of the orodispersible tablet. The one or more sweeteners can be present at between about 0.1 and about 7 wt% of the orodispersible tablet. The one or more sweeteners can be present at between about 0.1 and about 4 wt% of the orodispersible tablet. The one or more sweeteners can be present at between about 0.1 and about 3 wt% of the orodispersible tablet. The one or more sweeteners can be present at between about 0.1 and about 2 wt% of the orodispersible tablet. The one or more sweeteners can be present at between about 0.1 and about 1 wt% of the orodispersible tablet. The one or more sweeteners can be present at between about 0.2 and about 1 wt% of the orodispersible tablet. The one or more sweeteners can be present at between about 0.2 and about 0.9 wt% of the orodispersible tablet. The one or more sweeteners can be present at between about 0.2 and about 0.8 wt% of the orodispersible tablet. The one or more sweeteners can be present at between about 0.2 and about 0.7 wt% of the orodispersible tablet. The one or more sweeteners can be present at between about 0.3 and about 0.7 wt% of the orodispersible tablet. The one or more sweeteners can be present at between about 0.3 and about 0.6 wt% of the orodispersible tablet. The one or more sweeteners can be present at between about 0.4 and about 0.6 wt% of the orodispersible tablet. The one or more sweeteners can be present at between about 0.4 and about 0.5 wt% of the orodispersible tablet. The one or more sweeteners can be present at between about 0.5 and about 0.6 wt% of the orodispersible tablet. The one or more sweeteners can be present at between about 0.45 and about 0.55 wt% of the orodispersible tablet. Preferably, the one or more sweeteners can be present at about 0.5 wt% of the orodispersible tablet.

The orodispersible tablets of the present invention can also comprise a flavouring. The orodispersible tablets of the present invention can also comprise one or more flavourings. The flavouring can be selected from lemon flavouring, mixed berry flavouring, grape flavouring, peppermint natural flavouring, mint flavouring, banana flavouring, chocolate flavouring, maple flavouring, strawberry flavouring, a raspberry flavouring, a cherry flavouring, orange flavouring, and a vanilla flavouring and mixtures thereof. The flavouring can be selected from lemon flavouring, mixed berry flavouring, grape flavouring, peppermint natural flavouring, mint flavouring, banana flavouring, chocolate flavouring, maple flavouring and orange flavouring. In one embodiment, the flavouring is peppermint natural flavouring. In another embodiment, the flavouring is lemon flavouring. Preferably, the flavouring is grape flavouring. Even more preferably, the flavouring is mixed berry flavouring. Alternatively, the orodispersible tablets may be unflavoured.

The one or more flavourings can be present at between about 0.03 and about 1 wt% of the orodispersible tablet. The one or more flavourings can be present at between about 0.03 and about 0.8 wt% of the orodispersible tablet. The one or more flavourings can be present at between about 0.03 and about 0.5 wt% of the orodispersible tablet. The one or more flavourings can be present at between about 0.03 and about 0.4 wt% of the orodispersible tablet. The one or more flavourings can be present at between about 0.03 and about 0.3 wt% of the orodispersible tablet. The one or more flavourings can be present at between about 0.05 and about 0.2 wt% of the orodispersible tablet. The one or more flavourings can be present at between about 0.05 and about 0.1 wt% of the orodispersible tablet. The one or more flavourings can be present at between about 0.03 and about 0.09 wt% of the orodispersible tablet. The one or more flavourings can be present at between about 0.03 and about 0.08 wt% of the orodispersible tablet. The one or more flavourings can be present at between about 0.03 and about 0.07 wt% of the orodispersible tablet. The one or more flavourings can be present at between about 0.03 and about 0.06 wt% of the orodispersible tablet. The one or more flavourings can be present at between about 0.04 and about 0.06 wt% of the orodispersible tablet. The one or more flavourings can be present at between about 0.04 and about 0.05 wt% of the orodispersible tablet. The one or more flavourings can be present at between about 0.05 and about 0.06 wt% of the orodispersible tablet. The one or more flavourings can be present at between about 0.045 and about 0.055 wt% of the orodispersible tablet. Preferably, the one or more flavourings can be present at about 0.05 wt% of the orodispersible tablet.

For example, when the flavouring is selected from lemon flavouring or mixed berry flavouring, the flavouring can be present at between about 0.2 and about 0.5 wt% of the orodispersible tablet. The flavouring can be present at between about 0.2 and about 0.4 wt% of the orodispersible tablet. The flavouring can be present at between about 0.3 and about 0.4 wt% of the orodispersible tablet. The one or more flavourings can be present at about 0.36 wt% of the orodispersible tablet. The one or more flavourings can be present at about 0.31 wt% of the orodispersible tablet.

The orodispersible tablets of the present invention can comprise between about 0.2 and about 0.5 of grape flavouring. The orodispersible tablets of the present invention can comprise about 0.36 wt% of lemon flavouring. The orodispersible tablets of the present invention can comprise about 0.31 wt% of mixed berry flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder and polyvinylpyrrolidone (crospovidone) as a disintegrant.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder and polyvinylpyrrolidone (crospovidone) as a disintegrant.

The orodispersible tablets of the present invention can comprise three or more of mannitol (e.g. granulated mannitol and/or spray-dried mannitol (e.g. Mannogem EZ^{®})) as a filler, microcrystalline cellulose (e.g. silicified microcrystalline cellulose) as a binder and polyvinylpyrrolidone (crospovidone) as a disintegrant.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and peppermint natural flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and lemon flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and mixed berry flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and grape flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and peppermint natural flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and lemon flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and mixed berry flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and grape flavouring as a flavouring.

The orodispersible tablets of the present invention can comprise three or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and peppermint natural flavouring as a flavouring.

The orodispersible tablets of the present invention can comprise three or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and lemon flavouring as a flavouring.

The orodispersible tablets of the present invention can comprise three or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and mixed berry flavouring as a flavouring.

The orodispersible tablets of the present invention can comprise three or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, silicon dioxide as a flow enhancer, sucralose as a sweetener and grape flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and peppermint natural flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and lemon flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and mixed berry flavouring as a flavouring.

The orodispersible tablets of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and grape flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and peppermint natural flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and lemon flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and mixed berry flavouring as a flavouring.

The orodispersible tablets of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and grape flavouring as a flavouring.

The orodispersible tablets of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and peppermint natural flavouring as a flavouring.

The orodispersible tablets of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and lemon flavouring as a flavouring.

The orodispersible tablets of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and mixed berry flavouring as a flavouring.

The orodispersible tablets of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone) as a disintegrant, magnesium stearate as a lubricant, silicon dioxide as a flow enhancer, sucralose as a sweetener and grape flavouring as a flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), silicon dioxide, sucralose and peppermint natural flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), silicon dioxide, sucralose and lemon flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), silicon dioxide, sucralose and mixed berry flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), silicon dioxide, sucralose and grape flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), magnesium stearate, silicon dioxide, sucralose and peppermint natural flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), magnesium stearate, silicon dioxide, sucralose and lemon flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), magnesium stearate, silicon dioxide, sucralose and mixed berry flavouring.

The orodispersible tablets of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone), magnesium stearate, silicon dioxide, sucralose and grape flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), silicon dioxide, sucralose and peppermint natural flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), silicon dioxide, sucralose and lemon flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), silicon dioxide, sucralose and mixed berry flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), silicon dioxide, sucralose and grape flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), magnesium stearate, silicon dioxide, sucralose and peppermint natural flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), magnesium stearate, silicon dioxide, sucralose and lemon flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), magnesium stearate, silicon dioxide, sucralose and mixed berry flavouring.

The orodispersible tablets of the present invention can comprise one or more of granulated mannitol, spray-dried mannitol (e.g. Mannogem EZ^{®}), silicified microcrystalline cellulose, polyvinylpyrrolidone (crospovidone), magnesium stearate, silicon dioxide, sucralose and grape flavouring.

The orodispersible tablets of the present invention can comprise between 26 and 50 wt% of KVD900 (or a pharmaceutically acceptable salt and/or solvate thereof) and one or more fillers (e.g. granulated mannitol and/or spray-dried mannitol) present at between about 30 and 80 wt% of the orodispersible tablet.

The orodispersible tablets of the present invention can comprise between 26 and 50 wt% of KVD900 (or a pharmaceutically acceptable salt and/or solvate thereof) and one or more binders (e.g. silicified microcrystalline cellulose) present at between about 5 and about 30 wt% of the orodispersible tablet.

The orodispersible tablets of the present invention can comprise between 26 and 50 wt% of KVD900 (or a pharmaceutically acceptable salt and/or solvate thereof) and one or more disintegrants (e.g. polyvinylpyrrolidone (crospovidone)) present at between 1 and about 10 wt% of the orodispersible tablet.

The orodispersible tablets of the present invention can comprise between 26 and 50 wt% of KVD900 (or a pharmaceutically acceptable salt and/or solvate thereof), one or more fillers (e.g. granulated mannitol and/or spray-dried mannitol) present at between about 30 and 80 wt% of the orodispersible tablet, one or more binders (e.g. silicified microcrystalline cellulose) present at between about 5 and about 30 wt% of the orodispersible tablet and one or more disintegrants (e.g. polyvinylpyrrolidone (crospovidone)) present at between 1 and about 10 wt% of the orodispersible tablet.

The orodispersible tablets of the present invention can comprise between 26 and about 40 wt% of KVD900 (or a pharmaceutically acceptable salt and/or solvate thereof) and one or more fillers (e.g. granulated mannitol and/or spray-dried mannitol) present at between about 40 and about 70 wt% of the orodispersible tablet.

The orodispersible tablets of the present invention can comprise between 26 and about 40 wt% of KVD900 (or a pharmaceutically acceptable salt and/or solvate thereof) and one or more binders (e.g. silicified microcrystalline cellulose) present at between about 5 and about 20 wt% of the orodispersible tablet.

The orodispersible tablets of the present invention can comprise between 26 and about 40 wt% of KVD900 (or a pharmaceutically acceptable salt and/or solvate thereof) and one or more disintegrants (e.g. polyvinylpyrrolidone (crospovidone)) present at between 1 and about 7 wt% of the orodispersible tablet.

The orodispersible tablets of the present invention can comprise between 26 and about 40 wt% of KVD900 (or a pharmaceutically acceptable salt and/or solvate thereof), one or more fillers (e.g. granulated mannitol and/or spray-dried mannitol) present at between about 40 and 70 wt% of the orodispersible tablet, one or more binders (e.g. silicified microcrystalline cellulose) present at between about 5 and about 20 wt% of the orodispersible tablet and one or more disintegrants (e.g. polyvinylpyrrolidone (crospovidone)) present at between 1 and about 7 wt% of the orodispersible tablet.

The orodispersible tablets of the present invention can comprise about 30 wt% of KVD900 (or a pharmaceutically acceptable salt and/or solvate thereof).

The orodispersible tablets of the present invention can comprise about 30 wt% of KVD900 (or a pharmaceutically acceptable salt and/or solvate thereof) and one or more fillers (e.g. granulated mannitol and/or spray-dried mannitol) present at between about 54 wt% (e.g. 53.7 wt%) of the orodispersible tablet.

The orodispersible tablets of the present invention can comprise about 30 wt% of KVD900 (or a pharmaceutically acceptable salt and/or solvate thereof) and one or more binders (e.g. silicified microcrystalline cellulose) present at about 10 wt% of the orodispersible tablet.

The orodispersible tablets of the present invention can comprise about 30 wt% of KVD900 (or a pharmaceutically acceptable salt and/or solvate thereof) and one or more disintegrants (e.g. polyvinylpyrrolidone (crospovidone)) present at about 5 wt% of the orodispersible tablet.

The orodispersible tablets of the present invention can comprise about 30 wt% of KVD900 (or a pharmaceutically acceptable salt and/or solvate thereof), one or more fillers (e.g. granulated mannitol and/or spray-dried mannitol) present at between about 54 wt% (e.g. 53.7 wt%) of the orodispersible tablet, one or more binders (e.g. silicified microcrystalline cellulose) present at about 10 wt% of the orodispersible tablet and one or more disintegrants (e.g. polyvinylpyrrolidone (crospovidone)) present at about 5 wt% of the orodispersible tablet.

The orodispersible tablets of the present invention can comprise about 30 wt% of KVD900 (or a pharmaceutically acceptable salt and/or solvate thereof), one or more fillers (e.g. granulated mannitol and/or spray-dried mannitol) present at between about 54 wt% (e.g. 53.7 wt%) of the orodispersible tablet, one or more binders (e.g. silicified microcrystalline cellulose) present at about 10 wt% of the orodispersible tablet, one or more disintegrants (e.g. polyvinylpyrrolidone (crospovidone)) present at about 5 wt% of the orodispersible tablet, and a flow enhancer (e.g. silicon dioxide) present at about 0.75 wt% of the orodispersible tablet.

The orodispersible tablets of the present invention can comprise about 30 wt% of KVD900 (or a pharmaceutically acceptable salt and/or solvate thereof), one or more fillers (e.g. granulated mannitol and/or spray-dried mannitol) present at between about 54 wt% (e.g. 53.7 wt%) of the orodispersible tablet, one or more binders (e.g. silicified microcrystalline cellulose) present at about 10 wt% of the orodispersible tablet, one or more disintegrants (e.g. polyvinylpyrrolidone (crospovidone)) present at about 5 wt% of the orodispersible tablet, a flow enhancer (e.g. silicon dioxide) present at about 0.75 wt% of the orodispersible tablet, and one or more sweeteners (e.g. sucralose) present at about 0.5 wt% of the orodispersible tablet.

The orodispersible tablets of the present invention can comprise about 30 wt% of KVD900 (or a pharmaceutically acceptable salt and/or solvate thereof), one or more fillers (e.g. granulated mannitol and/or spray-dried mannitol) present at between about 54 wt% (e.g. 53.7 wt%) of the orodispersible tablet, one or more binders (e.g. silicified microcrystalline cellulose) present at about 10 wt% of the orodispersible tablet, one or more disintegrants (e.g. polyvinylpyrrolidone (crospovidone)) present at about 5 wt% of the orodispersible tablet, a flow enhancer (e.g. silicon dioxide) present at about 0.75 wt% of the orodispersible tablet, one or more sweeteners (e.g. sucralose) present at about 0.5 wt% of the orodispersible tablet and one or more flavourings (e.g. peppermint natural flavouring) present at about 0.05 wt% of the orodispersible tablet.

The orodispersible tablets of the present invention can comprise about 30 wt% of KVD900 (or a pharmaceutically acceptable salt and/or solvate thereof), one or more fillers (e.g. granulated mannitol and/or spray-dried mannitol) present at between about 54 wt% (e.g. 53.7 wt%) of the orodispersible tablet, one or more binders (e.g. silicified microcrystalline cellulose) present at about 10 wt% of the orodispersible tablet, one or more disintegrants (e.g. polyvinylpyrrolidone (crospovidone)) present at about 5 wt% of the orodispersible tablet, a flow enhancer (e.g. silicon dioxide) present at about 0.75 wt% of the orodispersible tablet, one or more sweeteners (e.g. sucralose) present at about 0.5 wt% of the orodispersible tablet and one or more flavourings (e.g. lemon flavouring or mixed berry flavouring) present at between about 0.3 and about 0.4 wt% of the orodispersible tablet.

The orodispersible tablets of the present invention can comprise about 27 wt% of granulated mannitol. The orodispersible tablets of the present invention can comprise about 26.87 wt% of granulated mannitol. Preferably, the orodispersible tablets of the present invention can comprise about 26.85 wt% of granulated mannitol.

The orodispersible tablets of the present invention can comprise about 27 wt% of spray-dried mannitol (e.g. Mannogem EZ^{®}). The orodispersible tablets of the present invention can comprise about 26.87 wt% of spray-dried mannitol (e.g. Mannogem EZ^{®}). Preferably, the orodispersible tablets of the present invention can comprise about 26.85 wt% of spray-dried mannitol (e.g. Mannogem EZ^{®}).

The orodispersible tablets of the present invention can comprise about 10 wt% of microcrystalline cellulose. Preferably, the orodispersible tablets of the present invention can comprise about 10 wt% (e.g. 10.01 wt%, 9.74 wt% or 9.69 wt%) of silicified microcrystalline cellulose.

The orodispersible tablets of the present invention can comprise about 5 wt% of polyvinylpyrrolidone (crospovidone).

The orodispersible tablets of the present invention can comprise about 0.75 wt% of silicon dioxide.

The orodispersible tablets of the present invention can comprise about 0.5 wt% of sucralose.

The orodispersible tablets of the present invention can comprise about 0.05 wt% of peppermint natural flavouring.

The orodispersible tablets of the present invention can comprise between about 0.2 and about 0.5 of grape flavouring.

The orodispersible tablets of the present invention can comprise about 0.36 wt% of lemon flavouring.

The orodispersible tablets of the present invention can comprise about 0.31 wt% of mixed berry flavouring.

The orodispersible tablets of the present invention can comprise a trace amount of magnesium stearate.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose and about 5 wt% of polyvinylpyrrolidone (crospovidone).

The orodispersible tablets of the present invention can comprise about 30 wt% of KVD900 (or a pharmaceutically acceptable salt and/or solvate thereof), about 26.87 wt% of granulated mannitol, about 26.87 wt% of spray-dried mannitol (e.g. Mannogem EZ^{®}), about 10 wt% (e.g. 10.01 wt%) of silicified microcrystalline cellulose, about 5 wt% of polyvinylpyrrolidone (crospovidone), about 0.75 wt% of silicon dioxide, and about 0.5 wt% of sucralose.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide, about 0.5 wt% of sucralose and about 0.05 wt% of peppermint natural flavouring.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% (e.g. 9.69 wt%) of silicified microcrystalline cellulose, about 5 wt% of polyvinylpyrrolidone (crospovidone), about 0.75 wt% of silicon dioxide, about 0.5 wt% of sucralose and about 0.36 wt% of lemon flavouring.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% (e.g. 9.74 wt%) of silicified microcrystalline cellulose, about 5 wt% of polyvinylpyrrolidone (crospovidone), about 0.75 wt% of silicon dioxide, about 0.5 wt% of sucralose and about 0.31 wt% of mixed berry flavouring.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide, about 0.5 wt% of sucralose, about 0.05 wt% of peppermint natural flavouring and a trace amount of magnesium stearate.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% (e.g. 9.69 wt%) of silicified microcrystalline cellulose, about 5 wt% of polyvinylpyrrolidone (crospovidone), about 0.75 wt% of silicon dioxide, about 0.5 wt% of sucralose, about 0.36 wt% of lemon flavouring and a trace amount of magnesium stearate.

The orodispersible tablets of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% (e.g. 9.74 wt%) of silicified microcrystalline cellulose, about 5 wt% of polyvinylpyrrolidone (crospovidone), about 0.75 wt% of silicon dioxide, about 0.5 wt% of sucralose, about 0.31 wt% of mixed berry flavouring and a trace amount of magnesium stearate.

The orodispersible tablets of the present invention can be round, oval, caplet or oblong-shaped. The orodispersible tablets of the present invention can be round. The orodispersible tablets of the present invention can be oval. The orodispersible tablets of the present invention can be caplet-shaped. The orodispersible tablets of the present invention can comprise 300 mg of KVD900 (or a pharmaceutically acceptable salt and/or solvate thereof) and can be round. The orodispersible tablets of the present invention can comprise 150 mg of KVD900 (or a pharmaceutically acceptable salt and/or solvate thereof) and can be oval. The orodispersible tablets of the present invention can comprise between about 25 mg and about 75 mg (e.g. 25 mg, 50 mg or 75 mg) of KVD900 (or a pharmaceutically acceptable salt and/or solvate thereof) and can be caplet or oblong-shaped. The orodispersible tablets of the present invention can comprise about 25 mg and can be caplet shaped. The orodispersible tablets of the present invention can comprise about 50 mg and can be caplet shaped. The orodispersible tablets of the present invention can comprise about 75 mg and can be caplet shaped.

A particular advantage of the orodispersible tablets of the present invention is that they can be debossed. The orodispersible tablets of the present invention can be debossed with a logo, the tablet strength and/or one or more score lines. The orodispersible tablets of the present invention can be debossed with a logo. The orodispersible tablets of the present invention can be debossed with the tablet strength. The orodispersible tablets of the present invention can be debossed with a logo and with the tablet strength. Preferably, the orodispersible tablets of the present invention can be debossed with one or more score lines. The orodispersible tablets of the present invention can be debossed with a logo and one or more score lines. The orodispersible tablets of the present invention can be debossed with a logo and a score line. The phrase "debossed with one or more score lines" means that the orodispersible tablets of the present invention are debossed with one or more marks across the planar surface of the orodispersible tablet that enable the orodispersible tablet to be easily, accurately and uniformly divided into multiple subdivided tablets. The orodispersible tablets of the present invention can be debossed with one score line. The orodispersible tablets of the present invention can be debossed with two score lines. The orodispersible tablets of the present invention can be debossed with two score lines to form a cross-shape.

The orodispersible tablets of the present invention can be debossed with a score line and then split. Tablet splitting is the practice of dividing a single higher strength tablet into multiple subdivided tablets in order to attain a desired dose of the active ingredient. Tablet splitting facilitates adjustment of the dose, which is particularly useful in facilitating dose administration in certain populations.

More specifically, the orodispersible tablets of the present invention can be debossed with a score line for splitting into multiple subdivided tablets. The orodispersible tablets of the present invention can be debossed with a score line for splitting into four subdivided tablets. The orodispersible tablets of the present invention can be debossed with a score line for splitting into two subdivided tablets. The orodispersible tablets of the present invention can be debossed with a score line for splitting into two subdivided tablets of approximately equal halves.

An advantage of the orodispersible tablets of the present invention that are debossed with a score line is that when they are split, the multiple subdivided tablets retain acceptable content uniformity. For example, when an orodispersible tablet of the present invention which is debossed with a score line for splitting into two subdivided tablets of approximately equal halves is split into half, each half retains acceptable content uniformity.

The present invention therefore also provides subdivided tablets that result from the splitting of the orodispersible tablets of the present invention along one or more score lines.

The orodispersible tablets of the present invention can comprise about 300 mg and can be round and can be debossed with a logo and a score line. The orodispersible tablets of the present invention can comprise about 150 mg and can be oval shaped and can be debossed with a logo and a score line. The orodispersible tablets of the present invention can comprise about 25 mg and can be caplet shaped and can be debossed with a score line. The orodispersible tablets of the present invention can comprise about 50 mg and can be caplet shaped and can be debossed with a score line. The orodispersible tablets of the present invention can comprise about 75 mg and can be caplet shaped and can be debossed with a score line.

### Disintegration and dissolution

As used here, the disintegration specification is obtained in accordance with the standard procedure in USP <701> and Ph Eur 2.9.1 (measured in purified water USP). The disintegration specification can be >80% in less than about 3 minutes. More specifically, the disintegration specification can be >80% in less than about 2 minutes. More specifically, the disintegration specification can be >80% in less than about 1 minute. The disintegration specification can be >80% in less than about 45 seconds. Preferably, the disintegration specification can be >80% in less than about 30 seconds. Even more preferably, the disintegration specification can be >80% in less than about 20 seconds. The disintegration specification can be >80% in less than about 15 to about 20 seconds. The disintegration specification can be >80% in less than about 15 seconds.

As used herein, the dissolution specification is obtained in accordance with the standard procedure in USP <711> and Ph Eur 2.9.3. The dissolution specification can be ≥99% in less than about 45 minutes. The dissolution specification can be ≥97% in less than about 45 minutes. The dissolution specification can be ≥95% in less than about 45 minutes. The dissolution specification can be ≥90% in less than about 45 minutes. The dissolution specification can be ≥80% in less than about 45 minutes. More specifically, the dissolution specification can be ≥80% in less than about 30 minutes. More specifically, the dissolution specification can be ≥80% in less than about 20 minutes. More specifically, the dissolution specification can be ≥80% in less than about 10 minutes. More specifically, the dissolution specification can be ≥80% in less than about 5 minutes.

The disintegration specification can be >80% in less than about 20 seconds and the dissolution specification can be about ≥99% in less than about 45 minutes. The disintegration specification can be >80% in less than about 15 to about 20 seconds and the dissolution specification can be about ≥97% in less than about 45 minutes. The disintegration specification can be >80% in less than about 30 seconds and the dissolution specification can be ≥95% in less than about 45 minutes. The disintegration specification can be >80% in less than about 20 seconds and the dissolution specification can be ≥90% in less than about 45 minutes. The disintegration specification can be >80% in less than about 15 seconds and the dissolution specification can be ≥80% in less than about 45 minutes. The disintegration specification can be >80% in less than about 30 seconds and the dissolution specification can be ≥80% in less than about 20 minutes. The disintegration specification can be >80% in less than about 20 seconds and the dissolution specification can be ≥80% in less than about 10 minutes. The disintegration specification can be >80% in less than about 15 seconds and the dissolution specification can be ≥80% in less than about 5 minutes.

### Mixtures of the present invention

The references to mixtures in the subsequent paragraphs are to be interpreted as references to mixtures for use in the methods of manufacturing ODTs of the invention, as set out in the appended claims.

The present invention also provides mixtures that are used in the manufacturing process to provide orodispersible tablets of the present invention. The mixtures of the present invention have improved flowability, compressibility and processibility properties, for example they can be compressed into a stable orodispersible tablet. Further, the mixtures of the present invention can be made at scale because the blend has excellent flowability and compressibility properties.

The mixtures of the present invention are particularly advantageous due to their versatility. Specifically, the mixtures of the present invention can be used to manufacture orodispersible tablets of varying size and/or strength without compromising the rapid disintegration times achieved by the orodispersible tablets of the present invention, or the uniformity of the dosage unit.

It is challenging to use the same mixture to manufacture orodispersible tablets of varying size and/or strength because, for example, the compression force tolerance can be is narrower for lower strength tablets. The applicant has overcome these difficulties and has provided mixtures of the present invention that can tolerate compression even for lower strengths (e.g. 75 mg, 50 mg or 25 mg) such that these mixtures can be used to manufacture orodispersible tablets of lower strength with a suitable compression force and resulting hardness whilst retaining the rapid disintegration times.

The invention provides mixtures comprising KVD900 (or a pharmaceutically acceptable salt and/or solvate thereof). The invention provides mixtures comprising between about 25 mg and about 1000 mg of KVD900 (or a pharmaceutically acceptable salt and/or solvate thereof). The mixtures of the invention can comprise between about 100 mg and about 1000 mg of KVD900 (or a pharmaceutically acceptable salt and/or solvate thereof). The mixtures of the invention can comprise between about 150 mg and about 1000 mg of KVD900 (or a pharmaceutically acceptable salt and/or solvate thereof). The mixtures of the invention can comprise between about 100 mg and about 700 mg of KVD900 (or a pharmaceutically acceptable salt and/or solvate thereof). The mixtures of the invention can comprise between about 200 mg and about 600 mg of KVD900 (or a pharmaceutically acceptable salt and/or solvate thereof). The mixtures of the invention can comprise about 250 mg of KVD900 (or a pharmaceutically acceptable salt and/or solvate thereof). Alternatively, the mixtures of the invention can comprise about 275 mg of KVD900 (or a pharmaceutically acceptable salt and/or solvate thereof). Alternatively, the mixtures of the invention can comprise about 300 mg of KVD900 (or a pharmaceutically acceptable salt and/or solvate thereof).

The mixtures of the invention can comprise between about 25 mg and about 500 mg of KVD900 (or a pharmaceutically acceptable salt and/or solvate thereof). The mixtures of the invention can comprise between about 25 mg and about 200 mg of KVD900 (or a pharmaceutically acceptable salt and/or solvate thereof). The mixtures of the invention can comprise between about 25 mg and about 150 mg of KVD900 (or a pharmaceutically acceptable salt and/or solvate thereof). The mixtures of the invention can comprise about 150 mg of KVD900 (or a pharmaceutically acceptable salt and/or solvate thereof).

The mixtures of the invention can comprise between about 25 mg and about 100 mg of KVD900 (or a pharmaceutically acceptable salt and/or solvate thereof). The mixtures of the invention can comprise between about 25 mg and about 80 mg of KVD900 (or a pharmaceutically acceptable salt and/or solvate thereof). The mixtures of the invention can comprise between about 25 mg and about 75 mg of KVD900 (or a pharmaceutically acceptable salt and/or solvate thereof). The mixtures of the invention can comprise about 75 mg of KVD900 (or a pharmaceutically acceptable salt and/or solvate thereof). Alternatively, the mixtures of the invention can comprise about 50 mg of KVD900 (or a pharmaceutically acceptable salt and/or solvate thereof). Alternatively, the mixtures of the invention can comprise about 25 mg of KVD900 (or a pharmaceutically acceptable salt and/or solvate thereof).

The mixtures of the invention comprise between 26 and 40 wt% of KVD900 (or a pharmaceutically acceptable salt and/or solvate thereof). The mixtures of the invention can comprise between 26 and about 35 wt% of KVD900 (or a pharmaceutically acceptable salt and/or solvate thereof). The mixtures of the invention can comprise between 26 and about 30 wt% of KVD900 (or a pharmaceutically acceptable salt and/or solvate thereof). The mixtures of the invention can comprise about 30 wt% of KVD900 (or a pharmaceutically acceptable salt and/or solvate thereof).

The mixtures of the present invention can comprise one or more of a filler, a binder, a disintegrant, a lubricant, a flow enhancer, a sweetener, and a flavouring.

The mixtures of the present invention can also comprise a filler. The mixtures of the present invention can also comprise one or more fillers.

The filler can be selected from isomalt, lactose and its derivatives (e.g. lactose monohydrate, spray dried lactose, anhydrous lactose), sorbitol, mannitol (e.g. granulated mannitol, powdered mannitol, spray-dried mannitol (e.g. Mannogem EZ^{®})), sorbitol, sucrose, starch, pregelatinized starch, and mixtures thereof. Preferably, the filler can be selected from isomalt, lactose and its derivatives (e.g. lactose monohydrate, spray dried lactose, anhydrous lactose), mannitol (e.g. granulated mannitol, powdered mannitol, spray-dried mannitol (e.g. Mannogem EZ^{®})), sorbitol and pregelatinized starch. The one or more fillers can be mannitol (e.g. granulated mannitol, powdered mannitol, spray-dried mannitol (e.g. Mannogem EZ^{®})). Even more preferably, the one or more fillers can be selected from granulated mannitol and spray-dried mannitol (e.g. Mannogem EZ^{®}). The one or more fillers can be granulated mannitol. The one or more fillers can be spray-dried mannitol (e.g. Mannogem EZ^{®}).

The one or more fillers are present at between 30 and 80 wt% of the mixture. The one or more fillers can be present at between about 40 and about 70 wt% of the mixture. The one or more fillers can be present at between about 40 and about 65 wt% of the mixture. The one or more fillers can be present at between about 50 and about 65 wt% of the mixture. The one or more fillers can be present at between about 50 and about 60 wt% of the mixture. The one or more fillers can be present at between about 50 and about 55 wt% of the mixture. The one or more fillers can be present at between about 53 and about 54 wt% of the mixture. Preferably, the one or more fillers can be present at about 53.7 wt% of the mixture.

The mixtures of the present invention can also comprise a binder. The mixtures of the present invention can also comprise one or more binders. The binder can also be selected from saccharides (such as glucose, sucrose, fructose, dextrose, lactose), sugar alcohols (such as xylitol, maltitol, erythritol, sorbitol), polysaccharides (such as crystalline cellulose, microcrystalline cellulose (e.g. silicified microcrystalline cellulose), powdered cellulose, methyl cellulose, carboxymethyl cellulose and salts, hydroxypropylmethyl cellulose), corn starch, starch 1500, acacia, other natural polymers (such as gelatin), polyethylene glycol, crosslinked acrylic acid and acrylic acid copolymers (e.g. Carbopol^{®}), inorganic compounds (such as calcium carbonate), calcium phosphates (e.g. anhydrous dibasic calcium phosphate, dibasic calcium phosphate, tribasic calcium phosphate), tricalcium citrate and mixtures thereof. More specifically, the binder can be selected from glucose, lactose, corn starch, starch 1500, gelatin, acacia, methyl cellulose, carboxymethyl cellulose and salts, hydroxypropylmethyl cellulose, microcrystalline cellulose, silicified microcrystalline cellulose and mixtures thereof. Preferably, the binder is microcrystalline cellulose or hydroxypropylmethyl cellulose. Even more preferably, the binder is silicified microcrystalline cellulose.

The one or more binders are present at between 5 and 40 wt% of the mixture. The one or more binders can be present at between 5 and about 20 wt% of the mixture. The one or more binders can be present at between 5 and about 15 wt% of the mixture. The one or more binders can be present at between 5 and about 14 wt% of the mixture. The one or more binders can be present at between 5 and about 10 wt% of the mixture. The one or more binders can be present at between about 8 and about 10 wt% of the mixture. The one or more binders can be present at about 10 wt% (e.g. 10.01 wt%, 9.74 wt% or 9.69 wt%) of the orodispersible tablet. Preferably, the one or more binders can be present at about 10 wt% of the mixture.

In the mixtures of the present invention, the one or more fillers and one or more binders can be present at between about 50 and about 90 wt% of the mixture. In the mixtures of the present invention, the one or more fillers and one or more binders can be present at between about 50 and about 70 wt% of the mixture. In the mixtures of the present invention, the one or more fillers and one or more binders can be present at between about 50 and about 65 wt% of the mixture. In the mixtures of the present invention, the one or more fillers and one or more binders can be present at between about 55 and about 65 wt% of the mixture. Preferably, the one or more fillers and one or more binders can be present at about 64 wt% of the mixture. Even more preferably, the one or more fillers and one or more binders can be present about 63.7 wt% of the of the mixture.

In the mixtures of the present invention, the wt% ratio of the one or more fillers to the one or more binders can be between about 3:1 to about 8:1. In the mixtures of the present invention, the wt% ratio of the one or more fillers to the one or more binders can be between about 4:1 to about 7:1. In the mixtures of the present invention, the wt% ratio of the one or more fillers to the one or more binders can be between about 4:1 to about 6:1. In the mixtures of the present invention, the wt% ratio of the one or more fillers to the one or more binders can be between about 5:1 to about 6:1. In the mixtures of the present invention, the wt% ratio of the one or more fillers to the one or more binders can be between about 4:1 to about 7:1. Preferably, in the mixtures of the present invention, the wt% ratio of the one or more fillers to the one or more binders can be between about 5:1 and about 5.5:1, more specifically between about 5.3:1 to about 5.4:1 (e.g. 5.37:1).

More specifically, in the mixtures of the present invention the wt% ratio of mannitol to microcrystalline cellulose is between about 3:1 to about 8:1, between about 4:1 to about 7:1, between about 4:1 to about 6:1, between about 5:1 to about 6:1 or between about 4:1 to about 7:1. Preferably, in the mixtures of the present invention the wt% ratio of mannitol to microcrystalline cellulose is between about 5:1 and about 5.5:1, more specifically between about 5.3:1 to about 5.4:1 (e.g. 5.37:1).

The mixtures of the present invention can also comprise a disintegrant. The mixtures of the present invention can also comprise one or more disintegrants. The disintegrant is selected from crosslinked cellulose (e.g. Croscarmellose^{®}, Ac-Di-Sol^{®}, Nymce ZSX^{®}, Primellose^{®}, Solutab^{®}, Vivasol^{®}), synthetic polymers such as cross linked polyvinylpyrrolidone (e.g. crospovidone, Crospovidon M^{®}, Kollidon^{®}, Polyplasdone^{®}), sodium starch glycolate, and mixtures thereof. More specifically, the disintegrant can be starch USP, starch 1500, cellulose derivatives (e.g. carboxymethyl cellulose and salts, hydroxypropyl methylcellulose), guar gum, alginic acid and salts, sodium starch glycolate, crosslinked cellulose (e.g. croscarmellose sodium such as those traded under the names Croscarmellose^{®}, Ac-Di-Sol^{®}, Nymce ZSX^{®}, Primellose^{®}, Solutab^{®}, Vivasol^{®}), cross linked polyvinylpyrrolidone (e.g. crospovidone, Crospovidon M^{®}, Kollidon^{®}, Polyplasdone^{®}), soy polysaccharides, calcium silicate, pearlitol Flash (co-processed Mannitol / Maize starch) and mixtures thereof. Preferably, the disintegrant is selected from polyvinylpyrrolidone (crospovidone), sodium starch glycolate, croscarmellose sodium. Even more preferably, the disintegrant is polyvinylpyrrolidone (crospovidone).

The one or more disintegrants can be present at between 1 and 10 wt% of the mixture. The one or more disintegrants can be present at between 1 and about 9 wt% of the mixture. The one or more disintegrants can be present at between about 2 and about 8 wt% of the mixture. The one or more disintegrants can be present at between 1 and about 7 wt% of the mixture. The one or more disintegrants can be present at between about 2 and about 7 wt% of the mixture. The one or more disintegrants can be present at between about 3 and about 6 wt% of the mixture. The one or more disintegrants can be present at between about 4 and about 6 wt% of the mixture. The one or more disintegrants can be present at between about 4 and about 5 wt% of the mixture. Preferably, the one or more disintegrants can be present at between about 5 wt% of the mixture.

The mixtures of the present invention can also comprise a flow enhancer. Preferably, the flow enhancer is silicon dioxide.

The flow enhancer can be present at between about 0.5 and about 3 wt% of the mixture. The flow enhancer can be present at between about 0.5 and about 2 wt% of the mixture. The flow enhancer can be present at between about 0.5 and about 1 wt% of the mixture. The flow enhancer can be present at between about 0.6 and about 0.9 wt% of the mixture. The flow enhancer can be present at between about 0.6 and about 0.8 wt% of the mixture. The flow enhancer can be present at between about 0.7 and about 0.8 wt% of the mixture. The flow enhancer can be present at between about 0.6 and about 0.75 wt% of the mixture. The flow enhancer can be present at between about 0.75 and about 0.8 wt% of the mixture. Preferably, the flow enhancer can be present at about 0.75 wt% of the mixture.

The mixtures of the present invention can also comprise a sweetener. The mixtures of the present invention can also comprise one or more sweeteners. The sweetener can be selected from sucralose, aspartame, sugar derivates, dextrose, polydextrose, xylitol, fructose, sucrose, lactitol, maltitol, and sodium saccharin (and mixtures thereof). Preferably, the sweetener is selected from sucralose, aspartame, sugar derivates, dextrose, polydextrose, fructose, lactitol and maltitol. Even more preferably, the sweetener is sucralose.

The one or more sweeteners can be present at between about 0.1 and about 10 wt% of the mixture. The one or more sweeteners can be present at between about 0.1 and about 7 wt% of the mixture. The one or more sweeteners can be present at between about 0.1 and about 4 wt% of the mixture. The one or more sweeteners can be present at between about 0.1 and about 3 wt% of the mixture. The one or more sweeteners can be present at between about 0.1 and about 2 wt% of the mixture. The one or more sweeteners can be present at between about 0.1 and about 1 wt% of the mixture. The one or more sweeteners can be present at between about 0.2 and about 1 wt% of the mixture. The one or more sweeteners can be present at between about 0.2 and about 0.9 wt% of the mixture. The one or more sweeteners can be present at between about 0.2 and about 0.8 wt% of the mixture. The one or more sweeteners can be present at between about 0.2 and about 0.7 wt% of the mixture. The one or more sweeteners can be present at between about 0.3 and about 0.7 wt% of the mixture. The one or more sweeteners can be present at between about 0.3 and about 0.6 wt% of the mixture. The one or more sweeteners can be present at between about 0.4 and about 0.6 wt% of the mixture. The one or more sweeteners can be present at between about 0.4 and about 0.5 wt% of the mixture. The one or more sweeteners can be present at between about 0.5 and about 0.6 wt% of the mixture. The one or more sweeteners can be present at between about 0.45 and about 0.55 wt% of the mixture. Preferably, the one or more sweeteners can be present at about 0.5 wt% of the mixture.

The mixtures of the present invention can also comprise a flavouring. The mixtures of the present invention can also comprise one or more flavourings.

The flavouring can be selected from lemon flavouring, mixed berry flavouring, grape flavouring, peppermint natural flavouring, mint flavouring, banana flavouring, chocolate flavouring, maple flavouring, strawberry flavouring, a raspberry flavouring, a cherry flavouring, orange flavouring, and a vanilla flavouring and mixtures thereof. The flavouring can be selected from lemon flavouring, mixed berry flavouring, grape flavouring, peppermint natural flavouring, mint flavouring, banana flavouring, chocolate flavouring, maple flavouring and orange flavouring. In an embodiment, the flavouring is peppermint natural flavouring. In another embodiment, the flavouring is lemon flavouring. Preferably, the flavouring is grape flavouring. Even more preferably, the flavouring is mixed berry flavouring. Alternatively, the orodispersible tablets may be unflavoured.

The one or more flavourings can be present at between about 0.03 and about 1 wt% of the mixture. The one or more flavourings can be present at between about 0.03 and about 0.8 wt% of the mixture. The one or more flavourings can be present at between about 0.03 and about 0.5 wt% of the mixture. The one or more flavourings can be present at between about 0.03 and about 0.4 wt% of the mixture. The one or more flavourings can be present at between about 0.03 and about 0.3 wt% of the mixture. The one or more flavourings can be present at between about 0.05 and about 0.2 wt% of the mixture. The one or more flavourings can be present at between about 0.05 and about 0.1 wt% of the mixture. The one or more flavourings can be present at between about 0.03 and about 0.09 wt% of the mixture. The one or more flavourings can be present at between about 0.03 and about 0.08 wt% of the mixture. The one or more flavourings can be present at between about 0.03 and about 0.07 wt% of the mixture. The one or more flavourings can be present at between about 0.03 and about 0.06 wt% of the mixture. The one or more flavourings can be present at between about 0.04 and about 0.06 wt% of the mixture. The one or more flavourings can be present at between about 0.04 and about 0.05 wt% of the mixture. The one or more flavourings can be present at between about 0.05 and about 0.06 wt% of the mixture. The one or more flavourings can be present at between about 0.045 and about 0.055 wt% of the mixture. Preferably, the one or more flavourings can be present at about 0.05 wt% of the mixture.

For example, when the flavouring is selected from lemon flavouring or mixed berry flavouring, the flavouring can be present at between about 0.2 and 0.5 wt% of the orodispersible tablet. The flavouring can be present at between about 0.2 and 0.4 wt% of the orodispersible tablet. The flavouring can be present at between about 0.3 and 0.4 wt% of the orodispersible tablet. The one or more flavourings can be present at about 0.36 wt% of the orodispersible tablet. The one or more flavourings can be present at about 0.31 wt% of the orodispersible tablet.

The mixtures of the present invention can comprise between about 0.2 and about 0.5 of grape flavouring. The mixtures of the present invention can comprise about 0.36 wt% of lemon flavouring. The mixtures of the present invention can comprise about 0.31 wt% of mixed berry flavouring.

The mixtures of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and peppermint natural flavouring as a flavouring.

The mixtures of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and lemon flavouring as a flavouring.

The mixtures of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and mixed berry flavouring as a flavouring.

The mixtures of the present invention can comprise one or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and grape flavouring as a flavouring.

The mixtures of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and peppermint natural flavouring as a flavouring.

The mixtures of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and lemon flavouring as a flavouring.

The mixtures of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and mixed berry flavouring as a flavouring.

The mixtures of the present invention can comprise two or more of mannitol (e.g. granulated mannitol and/or spray-dried mannitol (e.g. Mannogem EZ^{®})) as a filler, microcrystalline cellulose (e.g. silicified microcrystalline cellulose) as a binder, polyvinylpyrrolidone (crospovidone)) as a disintegrant, silicon dioxide as a flow enhancer, sucralose as a sweetener and grape flavouring as a flavouring.

The mixtures of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone)) as a disintegrant, silicon dioxide as a flow enhancer, sucralose as a sweetener and peppermint natural flavouring as a flavouring.

The mixtures of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone)) as a disintegrant, silicon dioxide as a flow enhancer, sucralose as a sweetener and lemon flavouring as a flavouring.

The mixtures of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone)) as a disintegrant, silicon dioxide as a flow enhancer, sucralose as a sweetener and mixed berry flavouring as a flavouring.

The mixtures of the present invention can comprise three or more of mannitol (e.g. granulated mannitol and/or spray-dried mannitol (e.g. Mannogem EZ^{®})) as a filler and microcrystalline cellulose (e.g. silicified microcrystalline cellulose) as a binder, polyvinylpyrrolidone (crospovidone)) as a disintegrant, silicon dioxide as a flow enhancer, sucralose as a sweetener and grape flavouring as a flavouring.

The mixtures of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone)), silicon dioxide, sucralose and peppermint natural flavouring.

The mixtures of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone)), silicon dioxide, sucralose and lemon flavouring.

The mixtures of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone)), silicon dioxide, sucralose and mixed berry flavouring.

The mixtures of the present invention can comprise one or more of granulated mannitol, spray-dried mannitol (e.g. Mannogem EZ^{®}), microcrystalline cellulose (e.g. silicified microcrystalline cellulose), polyvinylpyrrolidone (crospovidone)), silicon dioxide, sucralose and grape flavouring.

The mixtures of the present invention can comprise one or more of granulated mannitol, spray-dried mannitol (e.g. Mannogem EZ^{®}), silicified microcrystalline cellulose, polyvinylpyrrolidone (crospovidone)), silicon dioxide, sucralose and peppermint natural flavouring.

The mixtures of the present invention can comprise one or more of granulated mannitol, spray-dried mannitol (e.g. Mannogem EZ^{®}), silicified microcrystalline cellulose, polyvinylpyrrolidone (crospovidone)), silicon dioxide, sucralose and lemon flavouring.

The mixtures of the present invention can comprise one or more of granulated mannitol, spray-dried mannitol (e.g. Mannogem EZ^{®}), silicified microcrystalline cellulose, polyvinylpyrrolidone (crospovidone)), silicon dioxide, sucralose and mixed berry flavouring.

The mixtures of the present invention can comprise one or more of granulated mannitol, spray-dried mannitol (e.g. Mannogem EZ^{®}), silicified microcrystalline cellulose, polyvinylpyrrolidone (crospovidone)), silicon dioxide, sucralose and grape flavouring.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture and one or more disintegrants (e.g. polyvinylpyrrolidone (crospovidone)) present at between 1 and 10 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and 10 wt% of the mixture and a flow enhancer (e.g. silicon dioxide) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and 10 wt% of the mixture, a flow enhancer (e.g. silicon dioxide) present at between about 0.1 and about 5 wt% of the mixture and one or more sweeteners (e.g. sucralose) present at between about 0.1 and about 1 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and 10 wt% of the mixture, a flow enhancer (e.g. silicon dioxide) present at between about 0.1 and about 5 wt% of the mixture, one or more sweeteners (e.g. sucralose) present at between about 0.1 and about 5 wt% of the mixture and one or more flavourings (e.g. lemon flavouring, mixed berry flavouring, grape flavouring or peppermint natural flavouring) present at between 0.01 and about 1 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture and one or more disintegrants (e.g. polyvinylpyrrolidone (crospovidone)) present at between 1 and 10 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and 10 wt% of the mixture and a flow enhancer (e.g. silicon dioxide) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and 10 wt% of the mixture, a flow enhancer (e.g. silicon dioxide) present at between about 0.1 and about 5 wt% of the mixture and one or more sweeteners (e.g. sucralose) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and 10 wt% of the mixture, a flow enhancer (e.g. silicon dioxide) present at between about 0.1 and about 5 wt% of the mixture, one or more sweeteners (e.g. sucralose) present at between about 0.1 and about 5 wt% of the mixture and one or more flavourings (e.g. lemon flavouring, mixed berry flavouring, grape flavouring or peppermint natural flavouring) present at between 0.01 and about 1 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture and one or more disintegrants (e.g. polyvinylpyrrolidone (crospovidone)) present at between 1 and about 7 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and about 7 wt% of the mixture and a flow enhancer (e.g. silicon dioxide) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and about 7 wt% of the mixture, a flow enhancer (e.g. silicon dioxide) present at between about 0.1 and about 5 wt% of the mixture and one or more sweeteners (e.g. sucralose) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 30 wt% of the mixture, one or more disintegrants (e.g. polyvinylpyrrolidone (crospovidone)) present at between 1 and about 7 wt% of the mixture, a flow enhancer (e.g. silicon dioxide) present at between about 0.1 and about 5 wt% of the mixture, one or more sweeteners (e.g. sucralose) present at between about 0.1 and about 5 wt% of the mixture and one or more flavourings (e.g. lemon flavouring, mixed berry flavouring, grape flavouring or peppermint natural flavouring) present at between 0.01 and about 1 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture and one or more disintegrants (e.g. polyvinylpyrrolidone (crospovidone)) present at between 1 and about 7 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and about 7 wt% of the mixture and a flow enhancer (e.g. silicon dioxide) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and about 7 wt% of the mixture, a flow enhancer (e.g. silicon dioxide) present at between about 0.1 and about 5 wt% of the mixture and one or more sweeteners (e.g. sucralose) present at between about 0.1 and about 5 wt% of the mixture.

The mixtures of the present invention can comprise between 26 and 40 wt% of KVD900 (or a pharmaceutically acceptable salt and/or solvate thereof), one or more fillers (e.g. granulated mannitol and/or spray-dried mannitol) present at between 30 and 80 wt% of the mixture, one or more binders (e.g. silicified microcrystalline cellulose) present at between 5 and about 14 wt% of the mixture, one or more disintegrants (e.g. polyvinylpyrrolidone (crospovidone)) present at between 1 and about 7 wt% of the mixture, a flow enhancer (e.g. silicon dioxide) present at between about 0.1 and about 5 wt% of the mixture, one or more sweeteners (e.g. sucralose) present at between about 0.1 and about 5 wt% of the mixture and one or more flavourings (e.g. lemon flavouring, mixed berry flavouring, grape flavouring or peppermint natural flavouring) present at between 0.01 and about 1 wt% of the mixture.

The mixtures of the present invention can comprise about 27 wt% of granulated mannitol. The mixtures of the present invention can comprise about 26.87 wt% of granulated mannitol. Preferably, the mixtures of the present invention can comprise about 26.85 wt% of granulated mannitol.

The mixtures of the present invention can comprise about 27 wt% of spray-dried mannitol (e.g. Mannogem EZ^{®}). The mixtures of the present invention can comprise about 26.87 wt% of spray-dried mannitol (e.g. Mannogem EZ^{®}). Preferably, the mixtures of the present invention can comprise about 26.85 wt% of spray-dried mannitol (e.g. Mannogem EZ^{®}).

The mixtures of the present invention can comprise about 10 wt% of microcrystalline cellulose. Preferably, the mixtures of the present invention can comprise about 10 wt% of silicified microcrystalline cellulose.

The mixtures of the present invention can comprise about 5 wt% of polyvinylpyrrolidone (crospovidone).

The mixtures of the present invention can comprise about 0.75 wt% of silicon dioxide.

The mixtures of the present invention can comprise about 0.5 wt% of sucralose.

The mixtures of the present invention can comprise about 0.36 wt% of lemon flavouring.

The mixtures of the present invention can comprise about 0.31 wt% of mixed berry flavouring.

The mixtures of the present invention can comprise about 0.05 wt% of peppermint natural flavouring.

The mixtures of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose and about 5 wt% of polyvinylpyrrolidone (crospovidone).

The mixtures of the present invention can comprise about 30 wt% of KVD900 (or a pharmaceutically acceptable salt and/or solvate thereof), about 26.87 wt% of granulated mannitol, about 26.87 wt% of spray-dried mannitol (e.g. Mannogem EZ^{®}), about 10 wt% of silicified microcrystalline cellulose and about 5 wt% of polyvinylpyrrolidone (crospovidone).

The mixtures of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide, about 0.5 wt% of sucralose and about 0.05 wt% of peppermint natural flavouring.

The mixtures of the present invention can comprise about 30 wt% of KVD900 (or a pharmaceutically acceptable salt and/or solvate thereof), about 26.87 wt% of granulated mannitol, about 26.87 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide and about 0.5 wt% of sucralose.

The mixtures of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide, about 0.5 wt% of sucralose and about 0.36 wt% of lemon flavouring.

The mixtures of the present invention can comprise about 30 wt% of KVD900 (or a pharmaceutically acceptable salt and/or solvate thereof), about 26.85 wt% of granulated mannitol, about 26.85 wt% of spray-dried mannitol (e.g. Mannogem EZ^{®}), about 10 wt% of silicified microcrystalline cellulose, about 5 wt% of polyvinylpyrrolidone (crospovidone), about 0.75 wt% of silicon dioxide, about 0.5 wt% of sucralose and about 0.31 wt% of mixed berry flavouring.

### Methods of manufacturing of the present invention

The present invention provides a method of manufacturing an orodispersible tablet of the present invention from a mixture comprising,
a) between 26 and 40 wt% of KVD900;
b) between 30 and 80 wt% of one or more fillers;
c) between 5 and 40 wt% of one or more binders;
d) between 1 and 10 wt% of one or more disintegrants.
the method comprising blending the components of the mixture to form a powder mixture and compressing into the orodispersible tablet.

As noted above, the methods of manufacturing the ODTs described herein are ways to overcome instability and degradation problems initially seen when the inventors attempted to produce ODTs of KVD900.

The present invention provides a method of manufacturing the ODTs of the present invention from the mixtures of the present invention comprising blending the components of the mixture to form a powder mixture and compressing into the orodispersible tablet.

Specifically, the present invention provides a method of manufacturing an orodispersible tablet according to the present invention, from the mixtures of the present invention comprising:
a) combining the KVD900, the one or more binders, the one or more disintegrants and blending to form a powder mixture;
b) adding the one or more fillers to the powder mixture from step a) and blending to form a final blend;
c) compressing into the orodispersible tablet.

More specifically, the method of manufacturing comprises:
a) combining between 26 and 40 wt% of KVD900; between 5 and about 30 wt% of one or more binders selected from microcrystalline cellulose or hydroxypropylmethyl cellulose between 1 and 10 wt% of one or more disintegrants selected from polyvinylpyrrolidone (crospovidone), sodium starch glycolate, croscarmellose sodium and blending to form a powder mixture;
b) adding between 30 and 80 wt% of one or more fillers selected from mannitol (e.g. granulated mannitol and spray-dried mannitol (e.g. Mannogem EZ^{®})) to the powder mixture from step a) and blending to form a final blend;
c) compressing into the orodispersible tablet.

More specifically, the method of manufacturing comprises:
a) combining about 30 wt% of KVD900; about 10 wt% of silicified microcrystalline cellulose about 5 wt% of polyvinylpyrrolidone (crospovidone), and blending to form a powder mixture;
b) adding about 26.85 wt% of granulated mannitol and 26.85 wt% of spray-dried mannitol (e.g. Mannogem EZ^{®}) to the powder mixture from step a) and blending to form a final blend;
c) compressing into the orodispersible tablet.

Alternatively, the method of manufacturing comprises:
a) combining about 30 wt% of KVD900; about 10 wt% of silicified microcrystalline cellulose about 5 wt% of polyvinylpyrrolidone (crospovidone), and blending to form a powder mixture;
b) adding about 26.87 wt% of granulated mannitol and 26.87 wt% of spray-dried mannitol (e.g. Mannogem EZ^{®}) to the powder mixture from step a) and blending to form a final blend;
c) compressing into the orodispersible tablet.

In another embodiment of the invention, the method of manufacturing comprises:
a) combining the KVD900, the one or more binders, the one or more disintegrants, the flow enhancer; the one or more sweeteners and the one or more flavourings and blending to form a powder mixture;
b) adding the one or more fillers to the powder mixture from step a) and blending to form a final blend;
c) compressing into the orodispersible tablet.

More specifically, the method of manufacturing comprises:
a) combining between 26 and 40 wt% of KVD900, between 5 and about 30 wt% of one or more binders selected from microcrystalline cellulose or hydroxypropylmethyl cellulose, between 1 and 10 wt% of one or more disintegrants selected from polyvinylpyrrolidone (crospovidone), sodium starch glycolate, croscarmellose sodium, between about 0.5 to about 1 wt% of a flow enhancer, between about 0.1 and about 1 wt% of one or more sweeteners and between about 0.01 to about 1 wt% of one or more flavourings and blending to form a powder mixture;
b) adding between 30 and 80 wt% of one or more fillers selected from mannitol (e.g. granulated mannitol and spray-dried mannitol (e.g. Mannogem EZ^{®})) to the powder mixture from step a) and blending to form a final blend;
c) compressing into the orodispersible tablet.

More specifically, the method of manufacturing comprises:
a) combining about 30 wt% of KVD900, about 10 wt% of silicified microcrystalline cellulose, about 5 wt% of polyvinylpyrrolidone (crospovidone), about 0.75% silicon dioxide, about 0.5% sucralose and about 0.05 wt% peppermint natural flavouring and blending to form a powder mixture;
b) adding about 26.85 wt% of granulated mannitol and 26.85 wt% of spray-dried mannitol (e.g. Mannogem EZ^{®}) to the powder mixture from step a) and blending to form a final blend;
c) compressing into the orodispersible tablet.

Alternatively, the method of manufacturing comprises:
a) combining about 30 wt% of KVD900, about 10 wt% of silicified microcrystalline cellulose, about 5 wt% of polyvinylpyrrolidone (crospovidone), about 0.75% silicon dioxide, about 0.5% sucralose and blending to form a powder mixture;
b) adding about 26.87 wt% of granulated mannitol and 26.87 wt% of spray-dried mannitol (e.g. Mannogem EZ^{®}) to the powder mixture from step a) and blending to form a final blend;
c) compressing into the orodispersible tablet.

In another embodiment, the method of manufacturing comprises:
a) combining about 30 wt% of KVD900, about 10 wt% of silicified microcrystalline cellulose, about 5 wt% of polyvinylpyrrolidone (crospovidone), about 0.75% silicon dioxide, about 0.5% sucralose and about 0.36 wt% lemon flavouring and blending to form a powder mixture;
b) adding about 26.85 wt% of granulated mannitol and 26.85 wt% of spray-dried mannitol (e.g. Mannogem EZ^{®}) to the powder mixture from step a) and blending to form a final blend;
c) compressing into the orodispersible tablet.

In another embodiment, the method of manufacturing comprises:
a) combining about 30 wt% of KVD900, about 10 wt% of silicified microcrystalline cellulose, about 5 wt% of polyvinylpyrrolidone (crospovidone), about 0.75% silicon dioxide, about 0.5% sucralose and about 0.31 wt% mixed berry flavouring and blending to form a powder mixture;
b) adding about 26.85 wt% of granulated mannitol and 26.85 wt% of spray-dried mannitol (e.g. Mannogem EZ^{®}) to the powder mixture from step a) and blending to form a final blend;
c) compressing into the orodispersible tablet.

In some embodiments, the method of manufacturing comprises using an external lubricant. An external lubricant means a lubricant that aids flow through the manufacturing process, and as such might be present in trace amounts in the final ODT, but is not actively added to the blend/mixture. Preferably, the external lubricant is magnesium stearate.

The methods of manufacturing of the present invention can further comprise the step of debossing the orodispersible tablet. The methods of manufacturing of the present invention can further comprise the step of debossing the orodispersible tablet with a logo, the tablet strength and/or one or more score lines. In one embodiment, the methods of manufacturing of the present invention further comprise the step of debossing the orodispersible tablet with a logo. In another embodiment, the methods of manufacturing of the present invention further comprise the step of debossing the orodispersible tablet with the tablet strength. Preferably, the methods of manufacturing of the present invention further comprise the step of debossing the orodispersible tablet with one or more score lines. The methods of manufacturing of the present invention further comprise the step of debossing the orodispersible tablet with one score line. The methods of manufacturing of the present invention further comprise the step of debossing the orodispersible tablet with two score lines. The methods of manufacturing of the present invention further comprise the step of debossing the orodispersible tablet with two score lines to form a cross-shape.

### Kits of the present invention

The present invention also provides a kit for providing a dose of KVD900 (or a pharmaceutically acceptable salt and/or solvate thereof) comprising more than one orodispersible tablet according to the present invention.

In some embodiments of the present invention, the kit for providing a dose of KVD900 (or a pharmaceutically acceptable salt and/or solvate thereof) comprises two orodispersible tablet according to the present invention.

The total dose of the more than one orodispersible tablets in the kits of the present invention can be about 500 mg, e.g. by providing a kit comprising two orodispersible tablets as described here each comprising about 250 mg of KVD900.

The total dose of the more than one orodispersible tablets in the kits of the present invention can be about 550 mg, e.g. by providing a kit comprising two orodispersible tablets as described here each comprising about 275 mg of KVD900.

The total dose of the more than one orodispersible tablets in the kits of the present invention can be about 600 mg, e.g. by providing a kit comprising two orodispersible tablets as described here each comprising about 300 mg of KVD900.

In the kits of the present invention, the more than one orodispersible tablets can be packaged in a blister pack (e.g. Alu/Alu blisters, Aclar / Alu blisters, Amcor AmSky blisters). The blister packs may be in peel-back or push-through format. The more than one orodispersible tablets can also be packaged in other configurations that are known in the art.

When the kits comprise orodispersible tablets that are debossed with score line(s) as described herein, the kits of the present invention can also comprise instructions for splitting the orodispersible tablet into subdivided tablets.

### Orodispersible tablets of the present invention for use in treatments of bradykinin-mediated angioedema (e.g. BK AEnH attacks, preferably HAE attacks) attacks on-demand

As noted above, "bradykinin-mediated angioedema" can be selected from HAE and BK-AEnH. The bradykinin-mediated angioedema can be BK-AEnH. Preferably, the bradykinin-mediated angioedema is HAE.

The present invention provides orodispersible tablets as set out in the appended claims, and their use in a treatment of bradykinin-mediated angioedema that is improved compared to any bradykinin-mediated angioedema treatment currently available. The present invention provides an oral treatment of HAE that is particularly useful as an on-demand treatment of bradykinin-mediated angioedema attacks, and/or as an on-demand treatment to reduce the likelihood of bradykinin-mediated angioedema attacks. Specifically, as described herein, the treatments according to the invention (i) have a rapid onset of action, (ii) are potent, (iii) have a good safety profile, and (iv) have prolonged pharmacodynamic effects.

Thus, in accordance with the present invention, there is provided an orodispersible tablet as set out in the appended claims for use in a method for treating bradykinin-mediated angioedema on-demand, said method comprising: administering one or more of the orodispersible tablets comprising KVD900 according to the invention to a patient in need thereof on-demand.

In accordance with an aspect of the invention there is provided a method for treating an attack of bradykinin-mediated angioedema on-demand comprising: administering one or more orodispersible tablets comprising KVD900 according to the invention to a patient in need thereof, wherein the one or more orodispersible tablets are administered on-demand upon recognition of a symptom of a bradykinin-mediated angioedema attack.

When the bradykinin-mediated angioedema is HAE, the "bradykinin-mediated angioedema attack" is an "HAE attack". When the bradykinin-mediated angioedema is BK-AEnH, the "bradykinin-mediated angioedema attack" is a "BK-AEnH attack" As noted above, the bradykinin-mediated angioedema is preferably HAE.

Although each bradykinin-mediated angioedema attack (e.g. a BK-AEnH attack, preferably an HAE attack) can be different in severity and in terms of the area affected, patients who suffer from bradykinin-mediated angioedema (e.g. BK-AEnH, preferably HAE), medical professionals with knowledge of bradykinin-mediated angioedema (e.g. BK-AEnH, preferably HAE), and carers of bradykinin-mediated angioedema (e.g. BK-AEnH, preferably HAE) patients are astute in identifying symptoms of a bradykinin-mediated angioedema attack (e.g. a BK-AEnH attack, preferably an HAE attack). These symptoms include, but are not limited to: swelling of tissues such as in the hands, feet, limbs, face, intestinal tract, and/or airway; fatigue; headache; muscle aches; skin tingling; abdominal pain; nausea; vomiting; diarrhoea; difficulty swallowing; hoarseness; shortness of breath; and/or mood changes. Thus, in some embodiments, administration of KVD900 can occur upon recognition of at least one of the above symptoms.

The orodispersible tablets as set out in the appended claims for use in treatments of the invention can treat patients suffering from a laryngeal attack.

The orodispersible tablets as set out in the appended claims for use in treatments of the invention can treat patients suffering from an abdominal attack, and in particular, suffering from an abdominal attack and is experiencing vomiting episodes.

The orodispersible tablets as set out in the appended claims for use in treatments of the invention can treat patients also suffering from dysphagia.

The skilled person would also understand that "administered upon recognition of a symptom of a bradykinin-mediated angioedema attack (e.g. a BK-AEnH attack, preferably an HAE attack)" means that administration occurs as quickly as feasibly possible after the symptom of a bradykinin-mediated angioedema attack (e.g. a BK-AEnH attack, preferably an HAE attack) is recognised. For example, patients are expected to have KVD900 easily and readily available at all times (most likely in the form of a pharmaceutically acceptable composition) to ensure that treatment can occur upon recognition of a symptom of a bradykinin-mediated angioedema attack (e.g. a BK-AEnH attack, preferably an HAE attack). In other words, the treatment occurs on-demand. For example, in some embodiments, KVD900 can be administered within 1 hour of the symptom of a bradykinin-mediated angioedema attack (e.g. a BK-AEnH attack, preferably an HAE attack) being recognised, preferably within 30 minutes, within 20 minutes, within 10 minutes, or within 5 minutes of the symptom of a bradykinin-mediated angioedema attack (e.g. a BK-AEnH attack, preferably an HAE attack) being recognised.

If the symptom of a bradykinin-mediated angioedema attack (e.g. a BK-AEnH attack, preferably an HAE attack) is recognised in the prodromal phase, an embodiment of the invention is that KVD900 can be administered in the prodromal phase of a bradykinin-mediated angioedema attack (e.g. a BK-AEnH attack, preferably an HAE attack). In these circumstances, the symptom recognised can be a slight swelling, in particular, a slight swelling affecting the face and neck. In addition, or in the alternative, the symptom can be abdominal pain, in particular, abdominal pain is considered to be characteristic of a bradykinin-mediated angioedema attack (e.g. a BK-AEnH attack, preferably an HAE attack). In addition, or in the alternative, the symptom can be a reddening of the skin such as erythema marginatum.

The orodispersible tablets as set out in the appended claims for use in treatment in accordance with the invention can prevent a bradykinin-mediated angioedema attack (e.g. a BK-AEnH attack, preferably an HAE attack) from increasing in severity. In some circumstances, treatment can shorten the attack duration, and sometimes even halt the attack in its entirety. For instance, treatment can halt the progression of a peripheral bradykinin-mediated angioedema attack (e.g. a BK-AEnH attack, preferably an HAE attack) or an abdominal bradykinin-mediated angioedema attack (e.g. a BK-AEnH attack, preferably an HAE attack). In some embodiments, the orodispersible tablets as set out in the appended claims for use in treatment according to the invention can suppress the subsequent onset of swelling, sometimes completely, and in particular when treatment is initiated in the prodromal phase. In particular, in some embodiments, the bradykinin-mediated angioedema attack (e.g. a BK-AEnH attack, preferably an HAE attack) can be prevented from progressing into the swelling stage when the treatment is initiated in the prodromal phase.

KVD900 can be sufficient for treating the bradykinin-mediated angioedema attack (e.g. the BK-AEnH attack, preferably the HAE attack) alone i.e. without the patient being administered any active pharmaceutical ingredient other than KVD900. Thus, in some embodiments of the invention, no active pharmaceutical ingredient other than KVD900 is administered to the patient in order to treat the bradykinin-mediated angioedema attack (e.g. the BK-AEnH attack, preferably the HAE attack). In particular, in some embodiments, the orodispersible tablets as set out in the appended claims for use in the treatments of the invention do not require administering any active pharmaceutical ingredient for treating a bradykinin-mediated angioedema attack (e.g. a BK-AEnH attack, preferably an HAE attack) other than KVD900 (e.g. a rescue medication such as pdC1INH, rhC1INH, or icatibant is not required). More specifically, in some embodiments, no active pharmaceutical ingredient for treating a bradykinin-mediated angioedema attack (e.g. a BK-AEnH attack, preferably an HAE attack), other than KVD900 is administered to the patient (e.g. a rescue medication such as pdC1INH, rhC1INH, or icatibant is not required).

Alternatively, in some embodiments, the orodispersible tablets as set out in the appended claims for use in the treatments of the invention may be used in combination with other treatments of bradykinin-mediated angioedema (e.g. BK-AEnH, preferably HAE). For example, in some embodiments, the on-demand acute therapy described herein can be used as a "top-up" to another treatment of bradykinin-mediated angioedema (e.g. BK-AEnH, preferably HAE). In some embodiments, the patient may be taking a prophylactic treatment of bradykinin-mediated angioedema (e.g. BK-AEnH, preferably HAE) and might use the on-demand treatments described herein to treat a bradykinin-mediated angioedema attack (e.g. a BK-AEnH attack, preferably an HAE attack) that was not prevented by the other prophylactic treatment of bradykinin-mediated angioedema attack (e.g. a BK-AEnH attack, preferably an HAE attack).

For instance, in some embodiments, an orodispersible tablet as set out in the appended claims for use in a method for treating bradykinin-mediated angioedema (e.g. BK-AEnH, preferably HAE) in a patient already taking a C1 inhibitor (such as Cinryze^{®}, Haegarda^{®}, Berinert^{®}) for prophylaxis is provided, said method comprising: orally administering KVD900 to the patient on-demand upon recognition of a symptom of a bradykinin-mediated angioedema attack (e.g. a BK-AEnH attack, preferably an HAE attack). In another embodiment, an orodispersible tablet as set out in the appended claims for use in a method for treating bradykinin-mediated angioedema (e.g. BK-AEnH, preferably HAE) in a patient already taking lanadelumab for prophylaxis is provided, said method comprising: orally administering KVD900 to the patient on-demand upon recognition of a symptom of a bradykinin-mediated angioedema attack (e.g. a BK-AEnH attack, preferably an HAE attack). In another embodiment, an orodispersible tablet as set out in the appended claims for use in a method for treating bradykinin-mediated angioedema (e.g. BK-AEnH, preferably HAE) in a patient already taking berotralstat for prophylaxis is provided, said method comprising: orally administering KVD900 to the patient on-demand upon recognition of a symptom of a bradykinin-mediated angioedema attack (e.g. a BK-AEnH attack, preferably an HAE attack).

In any of the above treatments, the symptom can be recognised by the patient. In any of the above treatments, the symptom can be recognised by a medical professional such as a medical professional with knowledge of bradykinin-mediated angioedema (e.g. BK-AEnH, preferably HAE). In any of the above treatments, the symptom can be recognised by a carer of the patient.

The orodispersible tablets as set out in the appended claims for use in treatments according to the invention can reduce the proportion of bradykinin-mediated angioedema attacks (e.g. BK-AEnH attacks, preferably HAE attacks) that progress by one level or more on a 5-point Likert scale (5LS). The orodispersible tablets as set out in the appended claims for use in treatments according to the invention can reduce the proportion of bradykinin-mediated angioedema attacks (e.g. BK-AEnH attacks, preferably HAE attacks) that progress by one level or more on a 5LS within 12 hours of administering the compound. The orodispersible tablets as set out in the appended claims for use in treatments according to the invention can improve the resolution time of a bradykinin-mediated angioedema attack (e.g. a BK-AEnH attack, preferably a HAE attack) to "none" on a 5LS. 5LS is a known scale in the art (see e.g. Allergy Asthma Proc. 2018 Jan 1;39(1):74-80. doi: 10.2500/aap.2018.39.4095) that can be used to report the severity of bradykinin-mediated angioedema attacks (e.g. BK-AEnH attacks, preferably HAE attacks) and for example can be used to report attacks as "none", "mild", "moderate", "severe" or "very severe".

The orodispersible tablets as set out in the appended claims for use in treatments according to the invention can reduce the proportion of bradykinin-mediated angioedema attacks (e.g. BK-AEnH attacks, preferably HAE attacks) that are rated "worse" or "much worse" on a 7-point transition question (7TQ). The orodispersible tablets as set out in the appended claims for use in treatments according to the invention can increase the proportion of bradykinin-mediated angioedema attacks (e.g. BK-AEnH attacks, preferably HAE attacks) that are rated as "better" or "much better". 7TQ is a known index in the art that can be used to score the progression of a bradykinin-mediated angioedema attack (e.g. a BK-AEnH attack, preferably a HAE attack) and to report attacks as "much better", "better", "a little better", "no change", "a little worse", "worse", or "much worse".

In some embodiments of any of the orodispersible tablets as set out in the appended claims for use in the on-demand treatments of a bradykinin-mediated angioedema attack (e.g. a BK-AEnH attack, preferably an HAE attack) of the invention, the patient can be administered a single dosage amount of KVD900 to treat the bradykinin-mediated angioedema attack (e.g. a BK-AEnH attack, preferably an HAE attack). In some other embodiments of any of the orodispersible tablets as set out in the appended claims for use in the on-demand treatments of bradykinin-mediated angioedema attacks (e.g. BK-AEnH attacks, preferably HAE attacks) of the invention, the patient can be administered multiple dosage amounts of KVD900 to treat the acute bradykinin-mediated angioedema attack (e.g. a BK-AEnH attack, preferably an HAE attack). For example, the on-demand treatment can comprise administering two dosage amounts of KVD900 within a 24 hour period starting from the time of taking the first dosage amount. Alternatively, the on-demand treatment can comprise administering three dosage amounts of KVD900 within a 24 hour period starting from the time of taking the first dosage amount. Alternatively, the on-demand treatment can comprise administering four dosage amounts of KVD900 within a 24 hour period starting from the time of taking the first dosage amount. When multiple dosage amounts are taken, the dosage amount can be evenly spaced apart such that there is an approximately equal time period between each dosage amount e.g. taking the subsequent dosage amount at 8 hours, 16 hours and 24 hours following the first dosage amount.

In some embodiments of any of the orodispersible tablets as set out in the appended claims for use in the on-demand treatments of a bradykinin-mediated angioedema attack (e.g. a BK-AEnH attack, preferably an HAE attack) of the invention, the patient can be administered the daily dosage amount in two dosage amounts per day. These two dosage amounts can be administered simultaneously, separately or sequentially. In some embodiments, the two dosage amounts can be administered at any time within the day, with the interval between the two dosage amounts being specific to the patient, and the severity of the bradykinin-mediated angioedema attack (e.g. the BK-AEnH attack, preferably the HAE attack). In some embodiments, the second dosage amount can be administered within about 2 hours of the first (more specifically, between about 1 and 2 hours following the first dosage amount). In some embodiments, the second dosage amount can be administered between about 1 and about 4 hours of the first (more specifically, between about 1 and 3 hours, about 2 and 3 hours, or between 3 hours and about 4 hours, following the first dosage amount). In some embodiments, the second dosage amount can be administered between about 4 and about 12 hours of the first (more specifically, between about 4 and about 8 hours, or at about 6 hours, following the first dosage amount). In some embodiments, the second dosage amount can be administered between about 2 and about 6 hours of the first (more specifically, between about 3 and about 6 hours, following the first dosage amount). In some embodiments, the second dosage amount can be administered within about 8 hours of the first (more specifically, between about 4 and about 8 hours following the first dosage amount). In some embodiments, the second dosage amount can be administered within about 12 hours of the first (more specifically, between about 8 and about 12 hours following the first dosage amount). In some embodiments, the second dosage amount can be administered within about 16 hours of the first (more specifically, between about 12 and about 16 hours following the first dosage amount). In some embodiments, the second dosage amount can be administered within about 20 hours of the first (more specifically, between about 16 and about 20 hours following the first dosage amount). In some embodiments, the second dosage amount can be administered within about 24 hours of the first (more specifically, between about 20 and about 24 hours following the first dosage amount). In some embodiments, each of the two dosage amounts can be 500 mg of KVD900. Alternatively, each of the two dosage amounts can be 550 mg of KVD900. Alternatively, each of the two dosage amounts can be 600 mg of KVD900.

In any of the orodispersible tablets as set out in the appended claims for use in the on-demand treatments of a bradykinin-mediated angioedema attack (e.g. a BK-AEnH attack, preferably an HAE attack) of the invention, the patient can be administered the daily dosage amount in two dosage amounts per day, wherein the second dosage amount can be administered at least about 6 hours after the first dosage amount. The patient can be administered the daily dosage amount in two dosage amounts per day, wherein the second dosage amount can be administered between about 5 and about 7 hours after the first dosage amount. More specifically, the patient can be administered the daily dosage amount in two dosage amounts per day, wherein the second dosage amount can be administered about 6 hours after the first dosage amount. In these embodiments, each of the two dosage amounts can be 500 mg of KVD900. Each of these 500 mg dosage amounts can be two orodispersible tablets comprising 250 mg of KVD900. In these embodiments, each of the two dosage amounts can be 550 mg of KVD900. Each of these 550 mg dosage amounts can be two orodispersible tablets comprising 275 mg of KVD900. In these embodiments, each of the two dosage amounts can be 600 mg of KVD900. Each of these 600 mg dosage amounts can be two orodispersible tablets comprising 300 mg of KVD900.

In some embodiments of any of the orodispersible tablets as set out in the appended claims for use in the on-demand treatments of a bradykinin-mediated angioedema attack (e.g. a BK-AEnH attack, preferably an HAE attack) of the invention, the patient can be administered the daily dosage amount in three dosage amounts per day. These three dosage amounts can be administered simultaneously, separately or sequentially. In some embodiments, the three dosage amounts can be administered at any time within the day, with the interval between the three dosage amounts being specific to the patient, and the severity of the bradykinin-mediated angioedema attack (e.g. the BK-AEnH attack, preferably the HAE attack). In some embodiments, the second and third dosage amounts can be both administered within about 4 hours of the first. More specifically, the second dosage amount can be administered between about 1 and 3 hours following the first dosage amount and the third dosage amount can be administered between about 3 and about 4 hours following the first dosage amount. The second dosage amount can be administered between about 4 and about 12 hours of the first (more specifically, between about 4 and about 8 hours, or at about 6 hours, following the first dosage amount), and the third dosage amount can be administered between about 4 and about 12 hours of the second (more specifically, between about 4 and about 8 hours, or at about 6 hours, following the second dosage amount). Even more specifically, the second dosage amount can be administered about 2 hours following the first dosage amount and the third dosage amount can be administered about 4 hours following the first dosage amount. In some embodiments, the second and third dosage amounts can both be administered within about 8 hours of the first. More specifically, the second dosage amount can be administered between about 3 and 5 hours of the first dosage amount and the third dosage amount can be administered between about 7 and about 8 hours following the first dosage amount. Even more specifically, the second dosage amount can be administered about 4 hours following the first dosage amount and the third dosage amount can be administered about 8 hours following the first dosage amount. In some embodiments, the second and third dosage amounts can both be administered within about 16 hours of the first. More specifically, the second dosage amount can be administered between about 7 and 9 hours of the first dosage amount and the third dosage amount can be administered between about 15 and about 16 hours following the first dosage amount. Even more specifically, the second dosage amount can be administered about 8 hours following the first dosage amount and the third dosage amount can be administered about 16 hours following the first dosage amount. In some embodiments, each of the three dosage amounts can be 500 mg of KVD900. Alternatively, each of the three dosage amounts can be 550 mg of KVD900. Alternatively, each of the three dosage amounts can be 600 mg of KVD900.

In any of the orodispersible tablets as set out in the appended claims for use in the on-demand treatments of bradykinin-mediated angioedema attacks (e.g. BK-AEnH attacks, preferably HAE attacks) of the invention, the patient can be administered the daily dosage amount in three dosage amounts per day, wherein the second and third dosage amounts can be administered at least about 6 hours after the preceding dosage amount. The patient can be administered the daily dosage amount in three dosage amounts per day, wherein the second dosage amount can be administered between about 5 and about 7 hours after the first dosage amount, and the third dosage amount can be administered between about 11 and about 13 hours after the first dosage amount. More specifically, the patient can be administered the daily dosage amount in three dosage amounts per day, wherein the second dosage amount can be administered about 6 hours after the first dosage amount and the third dosage amount can be administered about 12 hours after the first dosage amount. In these embodiments, each of the three dosage amounts can be 500 mg of KVD900. Each of these 500 mg dosage amounts can be two orodispersible tablets comprising 250 mg of KVD900. In these embodiments, each of the three dosage amounts can be 550 mg of KVD900. Each of these 550 mg dosage amounts can be two orodispersible tablets comprising 275 mg of KVD900. In these embodiments, each of the three dosage amounts can be 600 mg of KVD900. Each of these 600 mg dosage amounts can be two orodispersible tablets comprising 300 mg of KVD900.

Multiple dosage amounts can be administered if, for example, a bradykinin-mediated angioedema attack (e.g. a BK-AEnH attack, preferably an HAE attack) persists after administration of the first dosage amount. When used in this context, "persists" can mean that, e.g., the first dosage amount does not prevent a bradykinin-mediated angioedema attack (e.g. a BK-AEnH attack, preferably an HAE attack) from increasing in severity, or that the first dosage amount does not halt the bradykinin-mediated angioedema attack (e.g. the BK-AEnH attack, preferably the HAE attack) in its entirety, or that the first dosage amount does not decrease the severity of the bradykinin-mediated angioedema attack (e.g. the BK-AEnH attack, preferably the HAE attack) . Accordingly, orodispersible tablets as set out in the appended claims for use in the on-demand treatments of a bradykinin-mediated angioedema attack (e.g. a BK-AEnH attack, preferably an HAE attack) of the invention can comprise administering a first dosage amount, and then administering a second dosage amount if the bradykinin-mediated angioedema attack (e.g. the BK-AEnH attack, preferably the HAE attack) persists after administering the first dosage amount. Orodispersible tablets as set out in the appended claims for use in on-demand treatments of a bradykinin-mediated angioedema attack (e.g. a BK-AEnH attack, preferably an HAE attack) of the invention can also comprise administering a first dosage amount, and then administering a second dosage amount if the bradykinin-mediated angioedema attack (e.g. the BK-AEnH attack, preferably the HAE attack) persists after administering the first dosage amount, and then administering a third dosage amount if the bradykinin-mediated angioedema attack (e.g. the BK-AEnH attack, preferably the HAE attack) persists after administering the second dosage amount. In each case, each subsequent dosage amount can be administered simultaneously, separately or sequentially. In each case, each subsequent dosage amount can be administered at least about 6 hours (e.g. at about 6 hours) after the preceding dosage amount. In each case, each dosage amount can comprise 500 mg of KVD900, e.g., administered as two orodispersible tablets comprising 250 mg of KVD900. Alternatively, each dosage amount can comprise 550 mg of KVD900, e.g., administered as two orodispersible tablets comprising 275 mg of KVD900. Alternatively, each dosage amount can comprise 600 mg of KVD900, e.g., administered as two orodispersible tablets comprising 300 mg of KVD900.

Specifically, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 500 mg of the compound (e.g. as two orodispersible tablets comprising 250 mg of the compound), and then administering a second dosage amount comprising 500 mg of the compound (e.g. as two orodispersible tablets comprising 250 mg of the compound) if the bradykinin-mediated angioedema attack (e.g. the BK-AEnH attack, preferably the HAE attack) persists after administering the first dosage amount. The second dosage amount can be administered at least about 6 hours (e.g. at about 6 hours) after the first dosage amount. If the bradykinin-mediated angioedema attack (e.g. the BK-AEnH attack, preferably the HAE attack) persists after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 500 mg of the compound (e.g. as two orodispersible tablets comprising 250 mg of the compound). The third dosage amount can be administered at least about 6 hours (e.g. at about 6 hours) after the second dosage amount.

Alternatively, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 550 mg of the compound (e.g. as two orodispersible tablets comprising 275 mg of the compound), and then administering a second dosage amount comprising 550 mg of the compound (e.g. as two orodispersible tablets comprising 275 mg of the compound) if the bradykinin-mediated angioedema attack (e.g. the BK-AEnH attack, preferably the HAE attack) persists after administering the first dosage amount. The second dosage amount can be administered at least about 6 hours (e.g. at about 6 hours) after the first dosage amount. If the bradykinin-mediated angioedema attack (e.g. the BK-AEnH attack, preferably the HAE attack) persists after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 550 mg of the compound (e.g. as two orodispersible tablets comprising 275 mg of the compound). The third dosage amount can be administered at least about 6 hours (e.g. at about 6 hours) after the second dosage amount.

Alternatively, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 600 mg of the compound (e.g. as two orodispersible tablets comprising 300 mg of the compound), and then administering a second dosage amount comprising 600 mg of the compound (e.g. as two orodispersible tablets comprising 300 mg of the compound) if the bradykinin-mediated angioedema attack (e.g. the BK-AEnH attack, preferably the HAE attack) persists after administering the first dosage amount. The second dosage amount can be administered at least about 6 hours (e.g. at about 6 hours) after the first dosage amount. If the bradykinin-mediated angioedema attack (e.g. the BK-AEnH attack, preferably the HAE attack) persists after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 600 mg of the compound (e.g. as two orodispersible tablets comprising 300 mg of the compound). The third dosage amount can be administered at least about 6 hours (e.g. at about 6 hours) after the second dosage amount.

Multiple dosage amounts can be administered even if the severity of the bradykinin-mediated angioedema attack (e.g. the BK-AEnH attack, preferably the HAE attack) appears to have been reduced (or even halted in its entirety) after administration of the first dosage amount to prevent the bradykinin-mediated angioedema attack (e.g. the BK-AEnH attack, preferably the HAE attack) from increasing in severity again. For example, multiple dosage amounts can be used for the peace of mind of the patient e.g. to ease anxiety of the patient. Accordingly, orodispersible tablets as set out in the appended claims for use in the on-demand treatments of a bradykinin-mediated angioedema attack (e.g. a BK-AEnH attack, preferably a HAE attack) of the invention can comprise administering a first dosage amount, and then administering a second dosage amount even if the severity of a bradykinin-mediated angioedema attack (e.g. a BK-AEnH attack, preferably a HAE attack) appears to have been reduced (or even halted in its entirety) after administration of the first dosage amount to prevent the bradykinin-mediated angioedema attack (e.g. the BK-AEnH attack, preferably the HAE attack) from increasing in severity again. Even if the severity of the bradykinin-mediated angioedema attack (e.g. the BK-AEnH attack, preferably the HAE attack) appears to have been reduced (or even halted in its entirety) after administration of the first and/or second dosage amount, the orodispersible tablets as set out in the appended claims for use in the on-demand treatments of a bradykinin-mediated angioedema attack (e.g. a BK-AEnH attack, preferably a HAE attack) of the invention can also comprise administering a third dosage amount to prevent the bradykinin-mediated angioedema attack (e.g. the BK-AEnH attack, preferably the HAE attack) from increasing in severity again. In each case, each subsequent dosage amount can be administered simultaneously, separately or sequentially. In each case, each subsequent dosage amount can be administered at least about 6 hours (e.g. at about 6 hours) after the preceding dosage amount. In each case, each dosage amount can comprise 500 mg of the compound, e.g., administered as two tablets comprising 250 mg of the compound. Alternatively, each dosage amount can comprise 550 mg of the compound, e.g., administered as two tablets comprising 275 mg of the compound. Alternatively, each dosage amount can comprise 600 mg of the compound, e.g., administered as two tablets comprising 300 mg of the compound.

Specifically, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 500 mg of the compound (e.g. as two tablets comprising 250 mg of the compound), and then administering a second dosage amount comprising 500 mg of the compound (e.g. as two tablets comprising 250 mg of the compound) even if the severity of the bradykinin-mediated angioedema attack (e.g. the BK-AEnH attack, preferably the HAE attack) appears to have been reduced (or even halted in its entirety) after administration of the first dosage amount to prevent the bradykinin-mediated angioedema attack (e.g. the BK-AEnH attack, preferably the HAE attack) from increasing in severity again. The second dosage amount can be administered at least about 6 hours (e.g. at about 6 hours) after the first dosage amount. Even if the severity of the bradykinin-mediated angioedema attack (e.g. the BK-AEnH attack, preferably the HAE attack) appears to have been reduced (or even halted in its entirety) after administration of the first and/or second dosage amount, the orodispersible tablets as set out in the appended claims for use in the on-demand treatments of a bradykinin-mediated angioedema attack (e.g. a BK-AEnH attack, preferably a HAE attack) of the invention can comprise administering a third dosage amount comprising 500 mg of the compound (e.g. as two tablets comprising 250 mg of the compound) to prevent the bradykinin-mediated angioedema attack (e.g. the BK-AEnH attack, preferably the HAE attack) from increasing in severity again. The third dosage amount can be administered at least about 6 hours (e.g. at about 6 hours) after the second dosage amount.

Alternatively, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 550 mg of the compound (e.g. as two tablets comprising 275 mg of the compound), and then administering a second dosage amount comprising 550 mg of the compound (e.g. as two tablets comprising 275 mg of the compound) even if the severity of the bradykinin-mediated angioedema attack (e.g. the BK-AEnH attack, preferably the HAE attack) appears to have been reduced (or even halted in its entirety) after administration of the first dosage amount to prevent the bradykinin-mediated angioedema attack (e.g. the BK-AEnH attack, preferably the HAE attack) from increasing in severity again. The second dosage amount can be administered at least about 6 hours (e.g. at about 6 hours) after the first dosage amount. Even if the severity of the bradykinin-mediated angioedema attack (e.g. the BK-AEnH attack, preferably the HAE attack) appears to have been reduced (or even halted in its entirety) after administration of the first and/or second dosage amount, the orodispersible tablets as set out in the appended claims for use in the on-demand treatments of a bradykinin-mediated angioedema attack (e.g. a BK-AEnH attack, preferably a HAE attack) of the invention can comprise administering a third dosage amount comprising 550 mg of the compound (e.g. as two tablets comprising 275 mg of the compound) to prevent the bradykinin-mediated angioedema attack (e.g. the BK-AEnH attack, preferably the HAE attack) from increasing in severity again. The third dosage amount can be administered at least about 6 hours (e.g. at about 6 hours) after the second dosage amount.

Alternatively, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount comprising 600 mg of the compound (e.g. as two tablets comprising 300 mg of the compound), and then administering a second dosage amount comprising 600 mg of the compound (e.g. as two tablets comprising 300 mg of the compound) even if the severity of the bradykinin-mediated angioedema attack (e.g. the BK-AEnH attack, preferably the HAE attack) appears to have been reduced (or even halted in its entirety) after administration of the first dosage amount to prevent the bradykinin-mediated angioedema attack (e.g. the BK-AEnH attack, preferably the HAE attack) from increasing in severity again. The second dosage amount can be administered at least about 6 hours (e.g. at about 6 hours) after the first dosage amount. Even if the severity of the bradykinin-mediated angioedema attack (e.g. the BK-AEnH attack, preferably the HAE attack) appears to have been reduced (or even halted in its entirety) after administration of the first and/or second dosage amount, the orodispersible tablets as set out in the appended claims for use in the on-demand treatments of a bradykinin-mediated angioedema attack (e.g. a BK-AEnH attack, preferably a HAE attack) of the invention can comprise administering a third dosage amount comprising 600 mg of the compound (e.g. as two tablets comprising 300 mg of the compound) to prevent the bradykinin-mediated angioedema attack (e.g. the BK-AEnH attack, preferably the HAE attack) from increasing in severity again. The third dosage amount can be administered at least about 6 hours (e.g. at about 6 hours) after the second dosage amount.

The orodispersible tablets as set out in the appended claims for use in the on-demand treatments of a bradykinin-mediated angioedema attack (e.g. a BK-AEnH attack, preferably an HAE attack) of the invention can comprise not administering more than three dosage amounts in a 24 hour period (e.g. three dosage amounts comprising 500 mg of the compound, optionally as 6 tablets each comprising 250 mg of the compound). Alternatively, the orodispersible tablets as set out in the appended claims for use in the on-demand treatments of a bradykinin-mediated angioedema attack (e.g. a BK-AEnH attack, preferably an HAE attack) of the invention can comprise not administering more than three dosage amounts in a 24 hour period (e.g. three dosage amounts comprising 550 mg of the compound, optionally as 6 tablets each comprising 275 mg of the compound). Alternatively, the orodispersible tablets as set out in the appended claims for use in the on-demand treatments of a bradykinin-mediated angioedema attack (e.g. a BK-AEnH attack, preferably an HAE attack) of the invention can comprise not administering more than three dosage amounts in a 24 hour period (e.g. three dosage amounts comprising 600 mg of the compound, optionally as 6 tablets each comprising 300 mg of the compound).

### Orodispersible tablets of the present invention for use in on-demand prophylactic treatment of bradykinin mediated angioedema attack (e.g. BK AEnH attacks, preferably HAE attacks)

In accordance with an aspect of the invention, there is provided an orodispersible tablet as set out in the appended claims for use in the a method for treating bradykinin-mediated angioedema (e.g. BK-AEnH, preferably HAE) on-demand, said method comprising: administering one or more of the orodispersible tablets comprising KVD900 according to the present invention to a patient in need thereof, wherein the one or more orodispersible tablets are administered on-demand to prophylactically reduce the likelihood of a bradykinin-mediated angioedema attack (e.g. a BK-AEnH attack, preferably an HAE attack).

In some embodiments, KVD900 can be administered to prevent a bradykinin-mediated angioedema attack (e.g. a BK-AEnH attack, preferably an HAE attack). As noted above, preferably the bradykinin-mediated angioedema is HAE.

As discussed above, the orodispersible tablets as set out in the appended claims for use in the treatments in accordance with the invention do not require dosing of KVD900 at regular intervals to provide prophylactic therapy. Indeed, in some embodiments, KVD900 can be administered on-demand. For example, KVD900 can be administered on-demand to reduce the likelihood of a bradykinin-mediated angioedema attack (e.g. a BK-AEnH attack, preferably an HAE attack), e.g. to prevent a bradykinin-mediated angioedema attack (such as a BK-AEnH attack, preferably an HAE attack) when it is anticipated that a bradykinin-mediated angioedema attack (e.g. a BK-AEnH attack, preferably an HAE attack) will be induced (or triggered) i.e. it is anticipated that the patient will suffer from a bradykinin-mediated angioedema attack (e.g. a BK-AEnH attack, preferably an HAE attack). In some embodiments, the patient can anticipate than a bradykinin-mediated angioedema attack (e.g. a BK-AEnH attack, preferably an HAE attack) will be induced (or triggered). In some embodiments, a medical professional such as a medical professional with knowledge of bradykinin-mediated angioedema (e.g. BK-AEnH, preferably HAE) can anticipate than a bradykinin-mediated angioedema attack (e.g. a BK-AEnH attack, preferably an HAE attack) will be induced (or triggered). In some embodiments, a carer for the patient can anticipate than a bradykinin-mediated angioedema attack (e.g. a BK-AEnH attack, preferably an HAE attack) will be induced (or triggered). For example, a bradykinin-mediated angioedema attack (e.g. a BK-AEnH attack, preferably an HAE attack) can be induced (or triggered) by various stimuli such as physical traumata (e.g. medical, dental or surgical procedures) and/or stress (e.g. high stress situations such as mental stress, which in some instances can be associated with taking examinations or mental stress associated with a medical, dental or surgical procedure). For example, a bradykinin-mediated angioedema attack (e.g. a BK-AEnH attack, preferably an HAE attack) can be induced (or triggered) by the elevated stress/anxiety levels of the patient when the patient might expect to have a bradykinin-mediated angioedema attack (e.g. a BK-AEnH attack, preferably an HAE attack). Additionally, the frequency of bradykinin-mediated angioedema attacks (e.g. BK-AEnH attacks, preferably HAE attacks) instances can vary over time in the same patient. Patients can often suffer from periods where the frequency of instances of bradykinin-mediated angioedema attacks (e.g. BK-AEnH attacks, preferably HAE attacks) is greater than normal. So, a bradykinin-mediated angioedema attack (e.g. a BK-AEnH attack, preferably an HAE attack) can be anticipated during periods where the patient is suffering from more frequent instances of bradykinin-mediated angioedema attacks (e.g. BK-AEnH attacks, preferably HAE attacks) compared to normal. Those familiar with bradykinin-mediated angioedema (e.g. BK-AEnH, preferably HAE) will be aware that bradykinin-mediated angioedema attacks (e.g. BK-AEnH attacks, preferably HAE attacks) can be induced (or triggered) in this way. Patients, medical professionals with knowledge of bradykinin-mediated angioedema (e.g. BK-AEnH, preferably HAE), and carers of patients can also be astute in anticipating such triggers. Thus, in accordance with the invention, the treatment can be administered on-demand when it is anticipated that the patient will be subjected to one or more of these stimuli or circumstances.

As discussed above, the patient can be administered KVD900 as part of an on-demand prophylactic treatment of a bradykinin-mediated angioedema attack (e.g. a BK-AEnH attack, preferably an HAE attack). As discussed above, this treatment reduces the likelihood of a bradykinin-mediated angioedema attack (e.g. a BK-AEnH attack, preferably an HAE attack). However, in some circumstances, a patient can still suffer from a bradykinin-mediated angioedema attack (e.g. a BK-AEnH attack, preferably an HAE attack).

Thus, an embodiment of the invention is that the patient can be administered KVD900 as part of an on-demand prophylactic treatment of a bradykinin-mediated angioedema attack (e.g. a BK-AEnH attack, preferably an HAE attack), as discussed above, further comprising taking an on-demand dosage amount of KVD900 upon recognition of a symptom of a bradykinin-mediated angioedema attack (e.g. a BK-AEnH attack, preferably an HAE attack) to treat a bradykinin-mediated angioedema attack (e.g. a BK-AEnH attack, preferably an HAE attack) should it arise. These on-demand treatments of bradykinin-mediated angioedema attacks (e.g. BK-AEnH attacks, preferably HAE attacks) are discussed above.

Thus, in some embodiments, there is provided an orodispersible tablet as set out in the appended claims for use in a method for treating bradykinin-mediated angioedema (e.g. BK-AEnH, preferably HAE) on-demand, said method comprising: administered one or more of the orodispersible tablets comprising KVD900 according to the present invention to a patient in need thereof, wherein the one or more orodispersible tablets are administered on-demand to prophylactically reduce the likelihood of a bradykinin-mediated angioedema attack (e.g. a BK-AEnH attack, preferably an HAE attack), further comprising administering the one or more orodispersible tablets on-demand upon recognition of a symptom of an acute HAE attack.

In some embodiments of any of the orodispersible tablets as set out in the appended claims for use in the on-demand treatments of a bradykinin-mediated angioedema attack (e.g. a BK-AEnH attack, preferably an HAE attack) of the invention, the patient can be administered a single dosage amount of KVD900 to treat the bradykinin-mediated angioedema attack (e.g. the BK-AEnH attack, preferably the HAE attack). In some other embodiments of any of the orodispersible tablets as set out in the appended claims for use in the on-demand treatments of bradykinin-mediated angioedema attacks (e.g. BK-AEnH attacks, preferably HAE attacks) of the invention, the patient can be administered multiple dosage amounts of KVD900 to treat the bradykinin-mediated angioedema attack (e.g. the BK-AEnH attack, preferably the HAE attack). For example, the on-demand treatment can comprise administering two dosage amounts of KVD900 within a 24 hour period starting from the time of taking the first dosage amount. Alternatively, the on-demand treatment can comprise administering three dosage amounts of KVD900 within a 24 hour period starting from the time of taking the first dosage amount. Alternatively, the on-demand treatment can comprise administering four dosage amounts of KVD900 within a 24 hour period starting from the time of taking the first dosage amount. When multiple dosage amounts are taken, the dosage amount can be evenly spaced apart such that there is an approximately equal time period between each dosage amount e.g. taking the subsequent dosage amount at 8 hours, 16 hours and 24 hours following the initial dosage amount.

In some embodiments of any of the orodispersible tablets as set out in the appended claims for use in the on-demand prophylactic treatments of bradykinin-mediated angioedema attacks (e.g. BK-AEnH attacks, preferably HAE attacks) described herein, the patient can be administered two dosage amounts per day. These two dosage amounts can be administered simultaneously, separately or sequentially. In some embodiments, the two dosage amounts can be administered at any time within the day, with the interval between the two dosage amounts being specific to the patient. In some embodiments, the second dosage amount can be administered within about 2 hours of the first (more specifically, between about 1 and 2 hours following the first dosage amount). In some embodiments, the second dosage amount can be administered between about 1 and about 4 hours of the first (more specifically, between about 1 and 3 hours, about 2 and 3 hours, or between 3 hours and about 4 hours, following the first dosage amount). In some embodiments, the second dosage amount can be administered between about 4 and about 12 hours of the first (more specifically, between about 4 and about 8 hours, or at about 6 hours, following the first dosage amount). In some embodiments, the second dosage amount can be administered between about 2 and about 6 hours of the first (more specifically, between about 3 and about 6 hours, following the first dosage amount). In some embodiments, the second dosage amount can be administered within about 8 hours of the first (more specifically, between about 4 and about 8 hours following the first dosage amount). In some embodiments, the second dosage amount can be administered within about 12 hours of the first (more specifically, between about 8 and about 12 hours following the first dosage amount). In some embodiments, the second dosage amount can be administered within about 16 hours of the first (more specifically, between about 12 and about 16 hours following the first dosage amount). In some embodiments, the second dosage amount can be administered within about 20 hours of the first (more specifically, between about 16 and about 20 hours following the first dosage amount). In some embodiments, the second dosage amount can be administered within about 24 hours of the first (more specifically, between about 20 and about 24 hours following the first dosage amount). In some embodiments, each of the two dosage amounts can be 500 mg of KVD900. Alternatively, each of the two dosage amounts can be 550 mg of KVD900. Alternatively, each of the two dosage amounts can be 600 mg of KVD900.

In any of the embodiments relating to the orodispersible tablets as set out in the appended claims for use in the on-demand prophylactic treatments of bradykinin-mediated angioedema attacks (e.g. a BK-AEnH attack, preferably an HAE attack) described herein, the patient can be administered the daily dosage amount in two dosage amounts per day, wherein the second dosage amount can be administered at least about 6 hours after the first dosage amount. The patient can be administered the daily dosage amount in two dosage amounts per day, wherein the second dosage amount can be administered between about 5 and about 7 hours after the first dosage amount. More specifically, the patient can be administered the daily dosage amount in two dosage amounts per day, wherein the second dosage amount can be administered about 6 hours after the first dosage amount. In these embodiments, each of the two dosage amounts can be 500 mg of KVD900. Each of these 500 mg dosage amounts can be two orodispersible tablets comprising 250 mg of KVD900. Alternatively, in these embodiments, each of the two dosage amounts can be 550 mg of KVD900. Each of these 550 mg dosage amounts can be two orodispersible tablets comprising 275 mg of KVD900. Alternatively, in these embodiments, each of the two dosage amounts can be 600 mg of KVD900. Each of these 600 mg dosage amounts can be two orodispersible tablets comprising 300 mg of KVD900.

In some embodiments of any of the orodispersible tablets as set out in the appended claims for use in the on-demand prophylactic treatments of bradykinin-mediated angioedema attacks (e.g. BK-AEnH attacks, preferably HAE attack) described herein, the patient can be administered the daily dosage amount in three dosage amounts per day. These three dosage amounts can be administered simultaneously, separately or sequentially. In some embodiments, the three dosage amounts can be administered at any time within the day, with the interval between the three dosage amounts being specific to the patient. In some embodiments, the second and third dosage amounts can be both administered within about 4 hours of the first. More specifically, the second dosage amount can be administered between about 1 and 3 hours following the first dosage amount and the third dosage amount can be administered between about 3 and about 4 hours following the first dosage amount. The second dosage amount can be administered between about 4 and about 12 hours of the first (more specifically, between about 4 and about 8 hours, or at about 6 hours, following the first dosage amount), and the third dosage amount can be administered between about 4 and about 12 hours of the second (more specifically, between about 4 and about 8 hours, or at about 6 hours, following the second dosage amount). Even more specifically, the second dosage amount can be administered about 2 hours following the first dosage amount and the third dosage amount can be administered about 4 hours following the first dosage amount. In some embodiments, the second and third dosage amounts can both be administered within about 8 hours of the first. More specifically, the second dosage amount can be administered between about 3 and 5 hours of the first dosage amount and the third dosage amount can be administered between about 7 and about 8 hours following the first dosage amount. Even more specifically, the second dosage amount can be administered about 4 hours following the first dosage amount and the third dosage amount can be administered about 8 hours following the first dosage amount. In some embodiments, the second and third dosage amounts can both be administered within about 16 hours of the first. More specifically, the second dosage amount can be administered between about 7 and 9 hours of the first dosage amount and the third dosage amount can be administered between about 15 and about 16 hours following the first dosage amount. Even more specifically, the second dosage amount can be administered about 8 hours following the first dosage amount and the third dosage amount can be administered about 16 hours following the first dosage amount. In some embodiments, each of the three dosage amounts can be 500 mg of KVD900. Alternatively, each of the three dosage amounts can be 550 mg of KVD900. Alternatively, each of the three dosage amounts can be 600 mg of KVD900.

In any of the orodispersible tablets as set out in the appended claims for use in the on-demand prophylactic treatments of bradykinin-mediated angioedema attacks (e.g. BK-AEnH attacks, preferably HAE attacks) described herein, the patient can be administered the daily dosage amount in three dosage amounts per day, wherein the second and third dosage amounts can be administered at least about 6 hours after the preceding dosage amount. The patient can be administered the daily dosage amount in three dosage amounts per day, wherein the second dosage amount can be administered between about 5 and about 7 hours after the first dosage amount, and the third dosage amount can be administered between about 11 and about 13 hours after the first dosage amount. More specifically, the patient can be administered the daily dosage amount in three dosage amounts per day, wherein the second dosage amount can be administered about 6 hours after the first dosage amount and the third dosage amount can be administered about 12 hours after the first dosage amount. In these embodiments, each of the three dosage amounts can be 500 mg of KVD900. Each of these 500 mg dosage amounts can be two orodispersible tablets comprising 250 mg of KVD900. Alternatively, in these embodiments, each of the three dosage amounts can be 550 mg of KVD900. Each of these 550 mg dosage amounts can be two orodispersible tablets comprising 275 mg of KVD900. Alternatively, in these embodiments, each of the three dosage amounts can be 600 mg of KVD900. Each of these 600 mg dosage amounts can be two orodispersible tablets comprising 300 mg of KVD900.

Multiple dosage amounts can be administered if, for example, there is a continued need to prophylactically reduce the likelihood of a bradykinin-mediated angioedema attack (e.g. a BK-AEnH attack, preferably an HAE attack), e.g. if the patient continues to anticipate that a bradykinin-mediated angioedema attack (e.g. a BK-AEnH attack, preferably an HAE attack) might be induced, as discussed above. Accordingly, the orodispersible tablets as set out in the appended claims for use in the on-demand treatments of a bradykinin-mediated angioedema attack (e.g. a BK-AEnH attack, preferably an HAE attack) of the invention can comprise administering a first dosage amount, and then administering a second dosage amount if there is a continued need to prophylactically reduce the likelihood of a bradykinin-mediated angioedema attack (e.g. a BK-AEnH attack, preferably an HAE attack) after administering the first dosage amount. The orodispersible tablets as set out in the appended claims for use in the on-demand treatments of a bradykinin-mediated angioedema attack (e.g. a BK-AEnH attack, preferably an HAE attack) of the invention can also comprise administering a first dosage amount, and then administering a second dosage amount if there is a continued need to prophylactically reduce the likelihood of a bradykinin-mediated angioedema attack (e.g. a BK-AEnH attack, preferably an HAE attack) after administering the first dosage amount, and then administering a third dosage amount if there is a continued need to prophylactically reduce the likelihood of a bradykinin-mediated angioedema attack (e.g. a BK-AEnH attack, preferably an HAE attack) after administering the second dosage amount. In each case, each subsequent dosage amount can be administered simultaneously, separately or sequentially. In each case, each dosage amount can comprise 500 mg of KVD900, e.g., administered as two orodispersible tablets comprising 250 mg of KVD900. Alternatively, each dosage amount can comprise 550 mg of KVD900, e.g., administered as two orodispersible tablets comprising 275 mg of KVD900. Alternatively, each dosage amount can comprise 600 mg of KVD900, e.g., administered as two orodispersible tablets comprising 300 mg of KVD900.

Specifically, the on-demand prophylactic treatments of bradykinin-mediated angioedema attacks (e.g. BK-AEnH attacks, preferably HAE attacks) described herein can comprise administering a first dosage amount comprising 500 mg of the compound (e.g. as two tablets each comprising 250 mg of the compound), and then administering a second dosage amount comprising 500 mg of the compound (e.g. as two tablets each comprising 250 mg of the compound) if there is a continued need to prophylactically reduce the likelihood of an acute HAE attack after administering the first dosage amount. The second dosage amount can be administered at least about 6 hours (e.g. at about 6 hours) after the first dosage amount. If there is a continued need to prophylactically reduce the likelihood of a bradykinin-mediated angioedema attack (e.g. a BK-AEnH attack, preferably an HAE attack) after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 500 mg of the compound (e.g. as two tablets each comprising 250 mg of the compound). The third dosage amount can be administered at least about 6 hours (e.g. at about 6 hours) after the second dosage amount.

Alternatively, the on-demand prophylactic treatments of bradykinin-mediated angioedema attacks (e.g. BK-AEnH attacks, preferably HAE attacks) described herein can comprise administering a first dosage amount comprising 550 mg of the compound (e.g. as two tablets each comprising 275 mg of the compound), and then administering a second dosage amount comprising 550 mg of the compound (e.g. as two tablets each comprising 275 mg of the compound) if there is a continued need to prophylactically reduce the likelihood of an acute HAE attack after administering the first dosage amount. The second dosage amount can be administered at least about 6 hours (e.g. at about 6 hours) after the first dosage amount. If there is a continued need to prophylactically reduce the likelihood of a bradykinin-mediated angioedema attack (e.g. a BK-AEnH attack, preferably an HAE attack) after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 550 mg of the compound (e.g. as two tablets each comprising 275 mg of the compound). The third dosage amount can be administered at least about 6 hours (e.g. at about 6 hours) after the second dosage amount.

Alternatively, the on-demand prophylactic treatments of bradykinin-mediated angioedema attacks (e.g. BK-AEnH attacks, preferably HAE attacks) described herein can comprise administering a first dosage amount comprising 600 mg of the compound (e.g. as two tablets each comprising 300 mg of the compound), and then administering a second dosage amount comprising 600 mg of the compound (e.g. as two tablets each comprising 300 mg of the compound) if there is a continued need to prophylactically reduce the likelihood of an acute HAE attack after administering the first dosage amount. The second dosage amount can be administered at least about 6 hours (e.g. at about 6 hours) after the first dosage amount. If there is a continued need to prophylactically reduce the likelihood of a bradykinin-mediated angioedema attack (e.g. a BK-AEnH attack, preferably an HAE attack) after the second dosage amount, the orodispersible tablets as set out in the appended claims for use in the on-demand treatments of a bradykinin-mediated angioedema attack (e.g. a BK-AEnH attack, preferably an HAE attack) of the invention can comprise administering a third dosage amount comprising 600 mg of the compound (e.g. as two tablets each comprising 300 mg of the compound). The third dosage amount can be administered at least about 6 hours (e.g. at about 6 hours) after the second dosage amount.

The on-demand prophylactic treatments of bradykinin-mediated angioedema attacks (e.g. BK-AEnH attacks, preferably HAE attacks) described herein can comprise not administering more than three dosage amounts in a 24 hour period (e.g. three dosage amounts comprising 500 mg of the compound, optionally as 6 tablets each comprising 250 mg of the compound). Alternatively, the on-demand prophylactic treatments of bradykinin-mediated angioedema attacks (e.g. BK-AEnH attacks, preferably HAE attacks) described herein can comprise not administering more than three dosage amounts in a 24 hour period (e.g. three dosage amounts comprising 550 mg of the compound, optionally as 6 tablets each comprising 275 mg of the compound). Alternatively, the on-demand prophylactic treatments of bradykinin-mediated angioedema attacks (e.g. BK-AEnH attacks, preferably HAE attacks) described herein can comprise not administering more than three dosage amounts in a 24 hour period (e.g. three dosage amounts comprising 600 mg of the compound, optionally as 6 tablets each comprising 300 mg of the compound).

### Orodispersible tablets of the present invention for use in continuous and regular prophylactic treatment of bradykinin mediated angioedema (e.g. BK AEnH, preferably HAE)

In accordance with an aspect of the invention, there is provided an orodispersible tablet as set out in the appended claims for use in a method for treating bradykinin-mediated angioedema (e.g. BK-AEnH, preferably HAE), the method comprising: administering one or more of the orodispersible tablets comprising KVD900 according to the present invention to a patient in need thereof, wherein the one or more orodispersible tablets are administered to prophylactically reduce the likelihood of a bradykinin-mediated angioedema attack (e.g. a BK-AEnH attack, preferably an HAE attack), wherein the one or more orodispersible tablets of the present invention are administered regularly to the patient.

The term "administered regularly" is intended to mean administering KVD900 continuously at regular intervals (e.g. once a week, twice a week, etc.) to provide an effective treatment. KVD900 is administered in one or more ODTs described herein. The healthcare professional would readily understand what regular (or continuous) administration is intended to mean.

In some embodiments, KVD900 can be administered to prevent a bradykinin-mediated angioedema attack (e.g. a BK-AEnH attack, preferably an HAE attack).

In some embodiments, the one or more orodispersible tablets can be administered once daily. In another embodiment, the one or more orodispersible tablets can be administered twice daily. In another embodiment, the one or more orodispersible tablets can be administered three times daily. In another embodiment, the one or more orodispersible tablets can be administered every other day.

As discussed above, the patient can be administered KVD900 as part of a continuous and regular prophylactic treatment of bradykinin-mediated angioedema (e.g. BK-AEnH, preferably HAE). As discussed above, this treatment reduces the likelihood of a bradykinin-mediated angioedema attack (e.g. a BK-AEnH attack, preferably an HAE attack). However, in some circumstances, a patient can still suffer from a bradykinin-mediated angioedema attack (e.g. a BK-AEnH attack, preferably an HAE attack). Thus, an embodiment of the invention is that the patient can be administered KVD900 as part of a continuous and regular prophylactic treatment of bradykinin-mediated angioedema (e.g. BK-AEnH, preferably HAE), as discussed above, further comprising taking an on-demand dosage amount of KVD900 upon recognition of a symptom of a bradykinin-mediated angioedema attack (e.g. a BK-AEnH attack, preferably an HAE attack) to treat a bradykinin-mediated angioedema attack (e.g. a BK-AEnH attack, preferably an HAE attack) should it arise. These on-demand treatments of bradykinin-mediated angioedema attacks (e.g. BK-AEnH attacks, preferably an HAE attacks) are discussed above.

Thus, in some embodiments, there is provided an orodispersible tablet as set out in the appended claims for use in a method for treating bradykinin-mediated angioedema (e.g. BK-AEnH, preferably HAE), said method comprising: administering one or more of the orodispersible tablets comprising KVD900 according to the present invention to a patient in need thereof, wherein the one or more orodispersible tablets are administered to prophylactically reduce the likelihood of a bradykinin-mediated angioedema attack (e.g. a BK-AEnH attack, preferably an HAE attack), wherein the one or more orodispersible tablets of the present invention are administered regularly to the patient, further comprising administering the one or more orodispersible tablets on-demand upon recognition of a symptom of a bradykinin-mediated angioedema attack (e.g. a BK-AEnH attack, preferably an HAE attack).

### BK-AEnH

As noted above, the bradykinin-mediated angioedema can be BK-AEnH.

Where the BK-AEnH is dipeptidyl peptidase-4 inhibitor-induced angioedema, the BK-AEnH can be induced by the use of dipeptidyl peptidase-4 inhibitor as an antidiabetic drug. The BK-AEnH can be dipeptidyl peptidase-4 inhibitor-induced by sitagliptin, metformin, saxagliptin, linagliptin, empagliflozin, alogliptin, or pioglitazone.

Where the BK-AEnH is ace inhibitor-induced angioedema, the BK-AEnH can be ace inhibitor-induced by benazepril, captopril, enalapril, fosinopril, lisinopril, moexipril, perindopril, quinapril, ramipril, or trandolapril.

Where the BK-AEnH is tPA-induced angioedema, the BK-AEnH can be induced by thrombolytic therapy using a tissue plasminogen activator. The patient can be receiving thrombolytic therapy using a tissue plasminogen activator e.g. to treat an acute stroke such as an ischemic stroke.

Where the BK-AEnH is non-hereditary angioedema with normal C1 Inhibitor (AE-nC1 Inh) and is drug-induced (i.e. drug-induced AE-nC1 Inh), the BK-AEnH can be drug-induced by at least one of a nonsteroidal anti-inflammatory agent, a β-lactam antibiotic, and a non-β lactam antibiotic. The nonsteroidal anti-inflammatory agent can be at least one of aspirin, celecoxib, diclofenac, diflunisal, etodolac ibuprofen, indomethacin, ketoprofen, ketorolac, nabumetone, naproxen, oxaprozin, piroxicam, salsalate, sulindac, and tolmetin.

Where the BK-AEnH is non-hereditary angioedema with normal C1 Inhibitor (AE-nC1 Inh) and is drug-induced (i.e. drug-induced AE-nC1 Inh), the BK-AEnH can be induced by an angiotensin II receptor blocker (ARB). For instance, the BK-AEnH can be induced by azilsartan, candesartan, eprosartan, irbesartan, losartan, olmesartan, telmisartan, or valsartan.

Where the BK-AEnH is drug-induced AE-nC1 Inh, the BK-AEnH can be drug-induced by beta blockers.

Where the BK-AEnH is non-hereditary angioedema with normal C1 Inhibitor (AE-nC1 Inh) and is hormonal-induced, the AE-nC1 Inh can be hormonally-induced by a hormonal contraceptive. In some embodiments, the AE-nC1 Inh can be hormonally-induced by oestrogen. For instance, the patient can be a female and be taking oestrogen e.g. as a contraceptive.

### Dosing

In any of the embodiments of the invention relating to the orodispersible tablets as set out in the appended claims for use in the treatments of the invention described herein, KVD900 is orally administered in the form of an orodispersible tablet in a therapeutically effective amount.

KVD900 can be administered at a daily dosage amount of between about 5 mg and about 2000 mg per day. "Daily dosage amount" means the total amount administered in one day. More specifically, the compound of Formula A can be administered at a daily dosage amount of between about 100 mg and about 1500 mg, about 300 mg to about 1800 mg, about 100 mg and about 1400 mg, about 200 mg and about 1200 mg, about 300 mg and about 1200 mg, about 600 mg and about 1200 mg, about 550 mg and about 1200 mg, about 500 mg and about 1000 mg, about 450 mg and about 900 mg, about 450 mg and about 600 mg, about 500 mg and about 700 mg (more specifically, 600 mg), about 800 mg and about 1000 mg per day, about 900 mg and about 1400 mg (more specifically 1200 mg), or about 900 mg and about 1200 mg. In a specific embodiment, the daily dosage amount is 300 mg. In another specific embodiment, the daily dosage amount is 500 mg. In another specific embodiment, the daily dosage amount is 550 mg. In another specific embodiment, the daily dosage amount is 600 mg. In another specific embodiment, the daily dosage amount is 900 mg. In another specific embodiment, the daily dosage amount is 1000 mg. In another specific embodiment, the daily dosage amount is 1100 mg. In another specific embodiment, the daily dosage amount is 1200 mg. In another specific embodiment, the daily dosage amount is 1500 mg. In another specific embodiment, the daily dosage amount is 1650 mg. In another specific embodiment the daily dosage amount is 1800 mg.

The daily dosage amount can be administered as one single dosage amount, or sub-divided into multiple dosage amounts for administration periodically during the day. In turn, each dosage amount can be administered as a single dosage form, or sub-divided into multiple dosage forms. Where multiple dosage amounts and multiple dosage forms are used, these can be administered simultaneously, separately or sequentially.

In some embodiments, each single unit dosage form comprising KVD900 comprises about 250 mg of KVD900. Alternatively, each single unit dosage form comprising KVD900 comprises about 275 mg of KVD900. Alternatively, each single unit dosage form comprising KVD900 comprises about 300 mg of KVD900.

Each dosage amount administered to the patient can comprise 500 mg of the KVD900 that may be sub-divided into two tablets comprising 250 mg of KVD900. Alternatively, each dosage amount administered to the patient can comprise 550 mg of KVD900 that may be sub-divided into two tablets comprising 275 mg of KVD900. Alternatively, each dosage amount administered to the patient can comprise 600 mg of KVD900 that may be sub-divided into two tablets comprising 300 mg of KVD900.

Alternatively, each dosage amount can comprise 250 mg of the compound that may be one tablet comprising 250 mg of the compound. Alternatively, each dosage amount can comprise 275 mg of the compound that may be one tablet comprising 275 mg of the compound. Alternatively, each dosage amount can comprise 300 mg of the compound that may be one tablet comprising 300 mg of the compound.

In a specific embodiment, the patient is administered a daily dosage amount of 500 mg, which is administered as one dosage amount. In a specific embodiment, the patient is administered a daily dosage amount of 550 mg, which is administered as one dosage amount. In another specific embodiment, the patient is administered a daily dosage amount of 600 mg, which is administered as one dosage amount.

In another specific embodiment, the patient is administered a daily dosage amount of 1000 mg, which is administered as two dosage amounts. In particular, the second dosage amount is administered between 2 and 6 hours of the first, preferably between about 3 and 6 hours of the first dosage amount. Alternatively, the patient is administered a daily dosage amount of 1100 mg, which is administered as two dosage amounts. In particular, the second dosage amount is administered between 2 and 6 hours of the first, preferably between about 3 and 6 hours of the first dosage amount. Alternatively, the patient is administered a daily dosage amount of 1200 mg, which is administered as two dosage amounts. In particular, the second dosage amount is administered between 2 and 6 hours of the first, preferably between about 3 and 6 hours of the first dosage amount.

In another specific embodiment, the patient is administered a daily dosage amount of 1500 mg, which is administered as three dosage amounts. In particular when the second dosage amount is administered between 2 and 8 hours of the first (e.g. at about 2 hours, about 4 hours, about 6 hours, or about 8 hours), and the third dosage amount is administered between about 4 and 16 hours of the first dosage amount (e.g. at about 4 hours, about 6 hours, about 8 hours, about 12 hours, or about 16 hours). Alternatively, the patient is administered a daily dosage amount of 1650 mg, which is administered as three dosage amounts. In particular when the second dosage amount is administered between 2 and 8 hours of the first (e.g. at about 2 hours, about 4 hours, about 6 hours, or about 8 hours), and the third dosage amount is administered between about 4 and 16 hours of the first dosage amount (e.g. at about 4 hours, about 6 hours, about 8 hours, about 12 hours, or about 16 hours). Alternatively, the patient is administered a daily dosage amount of 1800 mg, which is administered as three dosage amounts. In particular when the second dosage amount is administered between 2 and 8 hours of the first (e.g. at about 2 hours, about 4 hours, about 6 hours, or about 8 hours), and the third dosage amount is administered between about 4 and 16 hours of the first dosage amount (e.g. at about 4 hours, about 6 hours, about 8 hours, about 12 hours, or about 16 hours).

### Further specifics of the orodispersible tablets for use in the treatments of the invention

Thus, in the treatments of the invention, the one or more orodispersible tablets according to the present invention can be administered to patient in need thereof, wherein the patient is between the ages of 2 and less than 18, more specifically between the ages of 2 and less than 12. Alternatively, or additionally, the one or more orodispersible tablets according to the present invention can be administered to a patient in need thereof, wherein the patient is suffering from a laryngeal attack.

Alternatively, the one or more orodispersible tablets according to the present invention can be administered to patient in need thereof, wherein the patient is an elderly patient that struggles to swallow tablets (e.g. in patients older than 70 years old). Alternatively, or additionally, the one or more orodispersible tablets according to the present invention can be administered to a patient in need thereof, wherein the patient is suffering from a laryngeal attack.

As shown herein, KVD900 has a rapid onset of action. Specifically, KVD900 is a potent inhibitor of plasma kallikrein activity and is highly effective at interrupting the contact activation system's positive feedback loop between plasma kallikrein, prekallikrein, Factor XII (FXII), and Factor Xlla (FXlla). The pharmacokinetic and pharmacodynamic data provided herein (and in WO2020/249977 and WO2020/249979) demonstrate that these effects are shown quickly after oral administration of an orodispersible tablet comprising KVD900. Accordingly, the orodispersible tablets as set out in the appended claims for use in the treatments of the invention are fast acting and are thus particularly suited to treating bradykinin-mediated angioedema (e.g. BK-AEnH, preferably HAE) on-demand.

The orodispersible tablets as set out in the appended claims for use in the treatments of the invention are particularly advantageous when the concentration of KVD900 is at least 500 ng/mL in plasma. A plasma concentration of at least 500 ng/mL can be observed following administration of a dosage amount of at least about 60 mg (more specifically, at least about 70 mg or about 80 mg) of KVD900.

The orodispersible tablets as set out in the appended claims for use in the treatments according to the invention provide rapid protection from HK (high molecular weight kininogen) cleavage that are particularly suited to prophylactically reducing the chances of a bradykinin-mediated angioedema attack (e.g. a BK-AEnH attack, preferably an HAE attack) and/or to shorten the severity (or even halt) an ongoing bradykinin-mediated angioedema attack (e.g. a BK-AEnH attack, preferably an HAE attack). As described here, the orodispersible tablets as set out in the appended claims for use in the treatments according to the invention also have a prolonged pharmacodynamic effect. The pharmacodynamic effects of KVD900 that are related to treating bradykinin-mediated angioedema (e.g. BK-AEnH, preferably HAE) include providing protection from HK cleavage, which as discussed above, can cause a bradykinin-mediated angioedema attack (e.g. a BK-AEnH attack, preferably an HAE attack). For example, KVD900 can provide protection from HK cleavage by at least (i) inhibiting plasma kallikrein, (ii) reducing cleavage of plasma prekallikrein, and/or (iii) reducing the generation of Factor Xlla from Factor XII.

### Figures

In the Figures, the term "Compound" means KVD900.
- Figure 1:: X-ray powder diffraction pattern of KVD900 as generated in Example 1.
- Figure 2:: PK curve showing a comparable PK profile of the 500mg and 600mg ODT to the 600mg FCT used in the phase 2 study.
- Figure 3:: LogPK curve of the PK curve in Figure 2.
- Figure 4:: Geometric mean plasma concentrations after a single dose of 500 mg and 600 mg ODT or 600 mg FCT

Embodiments provided herein may be more fully understood by reference to the following examples. These examples are meant to be illustrative of treatments provided herein, but are not in any way limiting. Indeed, the scope of the invention is defined by the claims.

While examples of certain particular embodiments are provided herein, it will be apparent to those skilled in the art that various changes and modifications may be made. Such modifications are also intended to fall within the scope of the appended claims.

### General Experimental Details

In the following examples, the following abbreviations and definitions are used:

| | |
|---|---|
| BK-AEnH | Bradykinin-mediated angioedema nonhereditary |
| FCT | Film coated tablet |
| FXII | Factor XII |
| FXIIa | Factor XIIa |
| HAE | Hereditary angioedema |
| HK | High molecular weight kininogen |
| KVD900 | N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide |
| ODT | Orodispersible tablet |

### Example 1- Preparation of KVD900

### A. 1-(4-Hydroxymethyl-benzyl)-1H-pyridin-2-one

4-(Chloromethyl)benzylalcohol (5.0 g, 31.93 mmol) was dissolved in acetone (150 mL). 2-hydroxypyridine (3.64 g, 38.3 mmol) and potassium carbonate (13.24 g, 95.78 mmol) were added and the reaction mixture was stirred at 50 °C for 3 hrs after which time the solvent was removed *in vacuo* and the residue taken up in chloroform (100 mL). This solution was washed with water (30 mL), brine (30 mL), dried (Na₂SO₄) and evaporated *in vacuo.* The residue was purified by flash chromatography (silica), eluent 3% MeOH / 97% CHCl₃, to give a white solid identified as 1-(4-hydroxymethyl-benzyl)-1H-pyridin-2-one (5.30g, 24.62mmol, 77% yield).
[M+Na]⁺ = 238

### B. 1-(4-Chloromethyl-benzyl)-1H-pyridin-2-one

1-(4-Hydroxymethyl-benzyl)-1H-pyridin-2-one (8.45 g, 39.3 mmol), dry DCM (80 mL) and triethylamine (7.66 ml, 55.0 mmol) were cooled in an ice bath. Methanesulfonyl chloride (3.95 ml, 51.0 mmol) was added and stirred in ice bath for 15 min. The ice bath was removed and stirring continued at rt temperature overnight. The reaction mixture was partitioned between DCM (100 mL) and saturated aqueous NH₄Cl solution (100 mL). The aqueous layer was extracted with further DCM (2 x 50 mL) and the combined organics washed with brine (50 mL), dried over Na₂SO₄, filtered and concentrated to give 1-(4-chloromethyl-benzyl)-1H-pyridin-2-one (8.65 g, 36.6 mmol, 93 % yield) as a pale yellow solid.
[MH]⁺ = 234.1

### C. Methyl 3-(methoxymethyl)-1-(4-((2-oxopyridin-1(2H)-yl)methyl)benzyl)-1H-pyrazole-4-carboxylate

Potassium carbonate (519 mg, 3.76 mmol) was added to a solution of methyl 3-(methoxymethyl)-1H-pyrazole-4-carboxylate (320 mg, 1.88 mmol; CAS no. 318496-66-1 (synthesised according to the method described in WO 2012/009009)) and 1-(4-(chloromethyl)benzyl)pyridin-2(1H)-one (527 mg, 2.26 mmol) in DMF (5 mL) and heated at 60 °C overnight. The reaction mixture was diluted with EtOAc (50 mL) and washed with brine (2 x 100 mL), dried over magnesium sulfate, filtered and reduced *in vacuo.* The crude product was purified by flash chromatography (40 g column, 0-100% EtOAc in isohexanes) to afford two regioisomers. The second isomer off the column was collected to afford methyl 3-(methoxymethyl)-1-(4-((2-oxopyridin-1(2H)-yl)methyl)benzyl)-1H-pyrazole-4-carboxylate (378 mg, 1.01 mmol, 53.7 % yield) as a colourless gum.
[MH]⁺ = 368.2

### D. 3-(Methoxymethyl)-1-(4-((2-oxopyridin-1(2H)-yl)methyl)benzyl)-1H-pyrazole-4-carboxylic acid

To methyl 3-(methoxymethyl)-1-(4-((2-oxopyridin-1(2H)-yl)methyl)benzyl)-1H-pyrazole-4-carboxylate (3.77 g, 10.26 mmol) in THF (5 mL) and MeOH (5 mL) was added 2M NaOH solution (15.39 ml, 30.8 mmol) and stirred at rt overnight. 1M HCl (50 mL) was added and extracted with EtOAc (50 mL). The organic layer was washed with brine (50 mL), dried over magnesium sulfate, filtered and reduced *in vacuo* to give 3-(methoxymethyl)-1-(4-((2-oxopyridin-1(2H)-yl)methyl)benzyl)-1H-pyrazole-4-carboxylic acid (1.22 g, 3.45 mmol, 33.6 % yield) as a white powder.
[MH]⁺ = 354.2

### E. 3-Fluoro-4-methoxy-pyridine-2-carbonitrile

To a large microwave vial, copper (I) cyanide (1.304 g, 14.56 mmol) was added to a solution of 2-bromo-3-fluoro-4-methoxypyridine (1 g, 4.85 mmol) in DMF (5 mL). The reaction vial was sealed and heated to 100 °C for 16 hrs. The reaction mixture was diluted with water (20 mL) and EtOAc (20 mL). The thick suspension was sonicated and required additional water (40 mL) and EtOAc (2 x 50 mL) with sonication to break-up the solid precipitated. The combined layers were filtered through a plug of celite and the organic layer isolated, washed with brine (50 mL), dried over magnesium sulfate, filtered and the solvent removed under reduced pressure to give a pale green solid identified as the desired compound 3-fluoro-4-methoxy-pyridine-2-carbonitrile (100 mg, 0.578 mmol, 12 % yield)

### F. (3-Fluoro-4-methoxy-pyridin-2-ylmethyl)-carbamic acid tert-butyl ester

3-Fluoro-4-methoxy-pyridine-2-carbonitrile (100 mg, 0.578 mmol) was dissolved in anhydrous methanol (10 mL, 247 mmol) and nickel chloride hexahydrate (14 mg, 0.058 mmol) was added followed by di-tert-butyl dicarbonate (255 mg, 1.157 mmol). The resulting pale green solution was cooled in an ice-salt bath to -5 °C and then sodium borohydride (153 mg, 4.05 mmol) was added portion wise maintaining the reaction temperature ~0 °C. The deep brown solution was left to stir at 0 °C and slowly allowed to warm to rt and then left to stir at rt for 3 hrs. The reaction mixture was evaporated to dryness at 40 °C to afford a black residue which was diluted with DCM (10 mL) and washed with sodium hydrogen carbonate (10 mL). An emulsion formed so the organics were separated *via* a phase separating cartridge and concentrated. The crude liquid was purified by chromatography eluting with EtOAc / iso-Hexane to afford the title compound, (3-fluoro-4-methoxy-pyridin-2-ylmethyl)-carbamic acid tert-butyl ester as a clear yellow oil (108 mg, 62 % yield)
[MH]⁺= 257

### G. C-(3-Fluoro-4-methoxy-pyridin-2-yl)-methylamine hydrochloride salt

(3-Fluoro-4-methoxy-pyridin-2-ylmethyl)-carbamic acid tert-butyl ester (108mg, 0.358mmol) was taken up in iso-propyl alcohol (1 mL) and then HCl (6N in iso-propyl alcohol) (1 mL, 0.578 mmol) was added at rt and left to stir at 40 °C for 2 hrs. The reaction mixture was concentrated under reduced pressure and then triturated with ether, sonicated and then decanted to give a cream coloured solid (75 mg, 55% yield) identified as C-(3-fluoro-4-methoxy-pyridin-2-yl)-methylamine hydrochloride salt.
[MH]⁺= 157

### Example 1a - N-[(3-Fluoro-4-methoxypyridin-2-yl)methyl]-3-(methoxymethyl)-1-({4-[(2-oxopyridin-1-yl)methyllphenyl}methyl)pyrazole-4-carboxamide (Compound of Formula A)

3-(Methoxymethyl)-1-(4-((2-oxopyridin-1(2H)-yl)methyl)benzyl)-1H-pyrazole-4-carboxylic acid (825 mg, 2.34 mmol) and C-(3-fluoro-4-methoxy-pyridin-2-yl)-methylamine hydrochloride salt (450 mg, 2.34 mmol) were dissolved in DCM while cooling to 0°C. 1-Ethyl-3-(3-dimethylaminopropyl)carbodiimide hydrochloride (627.0 mg, 3.27 mmol), HOBt (378.8 mg, 2.80 mmol) and triethylamine (1.63 mL, 1182 mmol) were added while stirring, the mixture allowed to warm to rt and stirring continued for 20 hrs. Chloroform (50 mL) was added, the mixture was washed with saturated NaHCO₃(aq) and reduced in *vacuo.* The crude material was purified by chromatography eluting with methanol/DCM. The solvent was removed *in vacuo* and the resulting solid triturated with diethyl ether. The resulting solids were collected by filtration to afford the compound of Formula A.
[MH]⁺ = 492.0
NMR (CD₃OD) δ: 3.41 (3H, s), 4.03 (3H, s), 4.65 (2H, s), 4.72 (2H, d, J=2.3Hz), 5.24 (2H, s), 5.37 (2H, s), 6.44 (1H, td, J = 1.4, 6.8Hz), 6.62 (1H, d, J = 9.0Hz), 7.18-7.22 (1H, m), 7.31-7.38 (4H, m), 7.56-7.60 (1H, m), 7.75 (1H, dd, J = 1.9, 7.1Hz), 8.18 (1H, s), 8.27 (1H, d, J = 5.6Hz) ppm.

An XRPD diffractogram of KVD900 resultant from the above procedure is shown in Figure 1.

Peak position table:

| **No.** | **Pos. [°2Th.]** | **Rel. Int. [%]** |
|---|---|---|
| 1 | 4.436 | 32.36 |
| 2 | 5.0471 | 58.74 |
| 3 | 10.2255 | 43.07 |
| 4 | 11.2061 | 48.44 |
| 5 | 12.0101 | 16.4 |
| 6 | 12.5494 | 37.17 |
| 7 | 13.165 | 67.26 |
| 8 | 14.4984 | 38.94 |
| 9 | 15.8919 | 23.54 |
| 10 | 16.2983 | 34.56 |
| 11 | 17.4492 | 36.63 |
| 12 | 17.8564 | 71.49 |
| 13 | 18.6888 | 21.9 |
| 14 | 20.285 | 26.12 |
| 15 | 21.1598 | 100 |
| 16 | 22.04 | 87.76 |
| 17 | 22.5857 | 36.38 |
| 18 | 23.4408 | 14.33 |
| 19 | 24.3045 | 31.11 |
| 20 | 25.1655 | 78.97 |
| 21 | 25.3728 | 93.91 |
| 22 | 26.4946 | 56.79 |
| 23 | 27.991 | 76.91 |
| 24 | 28.7495 | 22.99 |
| 25 | 30.7611 | 13.4 |
| 26 | 32.413 | 17.2 |
| 27 | 37.2144 | 14.13 |
| 28 | 38.1171 | 14.14 |

### Example 2 - Preparation of the orodispersible tablet comprising KVD900 of the present invention

ODTs of KVD900 were prepared according to the following method.

### Method

1. Add KVD900, silicified microcrystalline cellulose, crospovidone, sucralose NF, silicon dioxide, peppermint natural flavour to the blender and blend to form a powder mixture.
2. Add granulated mannitol, spray-dried mannitol (e.g. Mannogem EZ^{®}) to the powder mixture from step 1 in the blender and blend.
3. Compress the final blend with external magnesium stearate as lubricant into tablets.

**Table 1: Composition of KVD900 250 mg and 300 mg Orodispersible Tablets (ODTs)**

| **Components** | **Function/ Standard** | **Blend composition** | **KVD900 250 mg ODT** | **KVD900 300 mg ODT** |
|---|---|---|---|---|
| | | **% w/w** | **mg** | **mg** |
| KVD900 | Active | 30.0 | 250.0 | 300.0 |
| Mannitol (Granulated) | Diluent (USP/NF, Ph Eur, JP) | 26.85 | 223.7 | 268.5 |
| Mannitol (Mannogem EZ) | Diluent (USP/NF, Ph Eur, JP) | 26.85 | 223.7 | 268.5 |
| Silicified Microcrystalline Cellulose | Diluent (USP/NF, Ph Eur, JP) | 10.00 | 83.3 | 100.0 |
| Crospovidone | Disintegrant (USP/NF, Ph Eur, JP) | 5.00 | 41.7 | 50.0 |
| Sucralose | Sweetener (USP/NF, Ph Eur) | 0.50 | 4.2 | 5.0 |
| Silicon Dioxide | Flow Enhancer (USP/NF, Ph Eur, JP) | 0.75 | 6.3 | 7.5 |
| Peppermint Natural Flavour | Flavour | 0.05 | 0.4 | 0.5 |
| **Total*** | **-**------ | **100.00** | **833.3** | **1,000** |

| | | | | |
|---|---|---|---|---|
| *Trace of Magnesium Stearate (USP/NF, Ph Eur, JP) for external lubrication only | | | | |

The orodispersible tablets of the present invention according to Table 1 have been made on a 5 kg scale.

The results provided in Examples 3-8 are for the orodispersible tablets of the present invention according to Table 1.

### Example 3 - Disintegration and dissolution of the orodispersible tablets of the present invention

### Method

### Disintegration testing

The disintegration testing can be conducted in accordance with the following protocol - USP <701> and Ph Eur 2.9.1.

Place 1 tablet in each of the six tubes of the basket and operate the apparatus using USP water maintained at 37 ± 2°C. At the end of the assigned time (see Table below), lift the basket from the fluid, and observed the tablets. If there is still palpable tablet cores in the baskets, start the test procedure again with new tablets.

When the tablets are completely disintegrated, report the value listed in the result column.

| **Time (seconds)** | **Result (seconds)** |
|---|---|
| 5 | 0-5 |
| 10 | 5-10 |
| 15 | 10-15 |
| 20 | 15-20 |
| 25 | 20-25 |
| 30 | 25-30 |

### Dissolution testing

The dissolution testing can be conducted in accordance with the following protocol - USP <711> (Type II) and Ph Eur 2.9.3.

The specific conditions are as follows:

| | |
|---|---|
| Paddle speed: | 50 rpm to 45 minutes |
| Dissolution media: | pH 4.5 sodium acetate buffer with 0.5% w/v sodium lauryl sulphate |
| Media Volume: | 1,000 mL |
| Dissolution Temp: | 37°C ± 0.5°C |
| Sample Volume: | 1.5 mL |
| Cannula Filter: | 10-micron cannula filter |
| Sampling Points: | 5, 10, 20, 30 and 45 minutes |
| Dissolution Time: | 45 minutes |
| Sample Analysis: | UPLC-UV |

The chromatographic conditions are as follows:

| | |
|---|---|
| HPLC Column: | Waters CSH C18, 2.1 x 75 mm, 1.7 micron (or validated equivalent) |
| Flow rate: | 0.7 mL/min |
| Detector wavelength: | 304 nm |
| Injection volume: | 5 µL |
| Column Temperature: | 60°C |
| Mobile Phase A: | HPLC Water + 0.05% formic acid |
| Mobile Phase B: | HPLC MeCN + 0.05% formic acid |
| Wash/Purge Solvent: | 90:10 MeCN: Water |
| Gradient: | see **Table 2** |

**Table 2: Details of Gradient Elution**

| Time (min) | %A | %B |
|---|---|---|
| 0.00 | 90 | 10 |
| 0.50 | 50 | 50 |
| 1.31 | 50 | 50 |
| 1.32 | 90 | 10 |
| 2.50 | 90 | 10 |

| | | |
|---|---|---|
| Total run time = 2.5 minutes | | |

### Results

| | **Disintegration time (sec)** | **Dissolution (%)** |
|---|---|---|
| KVD900 300 mg ODT | 15-20 | 97 |
| KVD900 250 mg ODT | < 20 | 99 |

The disintegration specification of the orodispersible tablets of the present invention is > 80% in less than about 15-20 seconds. This meets the acceptance criteria of not more than (NMT) 45 seconds based on standard procedure in USP <701> and Ph Eur 2.9.1. This also meets the FDA guidance for orodispersible tablets of a disintegration time of approximately 30 seconds of less, when based on the United States Pharmacopeia (USP) disintegration test method or alternative and the definition of orodispersible tablet in the European Pharmacopoeia of a disintegration time of not more than 3 minutes.

The dissolution specification of the orodispersible tablets of the present invention is >95% in 45 minutes. This meets the acceptance criteria of Q=80% at 45 minutes in a QC dissolution test compliant with standard procedure in USP <711> and Ph Eur 2.9.3.

### Example 4 - Uniformity and water content of the orodispersible tablets of the present invention

The uniformity of the dosage unit can be obtained using mass variation in accordance with the following protocol - USP<905> and Ph Eur 2.9.40.

The water content can be determined by Karl Fischer volumetric titration using apparatus for the determination of water content consistent with the requirements described in USP<921> and Method 1c/Ph Eur 2.5.12.

| | **Uniformity of Dosage Units** | **Water Content (% w/w)** |
|---|---|---|
| KVD900 300 mg ODT | Complies | 2.1% |
| KVD900 250 mg ODT | Complies | 1.4% |

The uniformity of dosage units of the orodispersible tablets of the present invention meet the acceptance criteria in accordance with USP<905> and Ph Eur 2.9.40.

The water content of the orodispersible tablets of the present invention meet the acceptance criteria in accordance with USP<921> and Method 1c/Ph Eur 2.5.12.

### Example 5 - Stability testing

### Stability of the KVD900 300 mg ODTs of the present invention

A sample of KVD900 300 mg ODT was packaged in a Aclar/Alu blister pack and stored at conditions of 25°C/60%RH. Tests on the sample of KVD900 300 mg ODT were carried out as described in **Table 3.**

**Table 3: Stability Data for KVD900 300 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| **Appearance** | | White to off white round tablet | White to off white round tablet | White to off white round tablet | White to off white round tablet | White to off white round tablet | White to off round tablet |
| **Disintegration (s)** | | 15-20 s | 10-15 s | 15-20 s | 15-20 s | 15-20 s | 15-20 s |
| **Dissolution (%)** | 45 min | 97% | 96% | 98% | 97% | 97% | 97% |
| **Water Content (%w/w)** | | 2.1 | 1.7 | 1.6 | 1.6 | 1.7 | 1.7 |

A second sample of KVD900 300 mg ODT was packaged in a Aclar/Alu blister pack and stored at conditions of 40°C/75%RH. Tests on the sample of KVD900 300 mg ODT were carried out as described in **Table 4.**

**Table 4: Stability Data for KVD900 300 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| **Appearance** | | White to off white round tablet | White to off white round tablet | White to off white round tablet | White to off white round tablet |
| **Disintegration (s)** | | 15-20 s | 10-15 s | 15-20 s | 15-20 s |
| **Dissolution (%)** | 45 min | 97% | 96% | 98% | 96% |
| **Water Content (%w/w)** | | 2.1 | 2.3 | 1.8 | 2.0 |

### Stability of the KVD900 250 mg ODTs of the present invention

A sample of KVD900 250 mg ODT was packaged in a Aclar/Alu blister pack and stored at conditions of 25°C/60%RH. Tests on the sample of KVD900 250 mg ODT were carried out as described in **Table 5.**

**Table 5: Stability Data for KVD900 250 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| **Appearance** | | White to off white round tablet | White to off white round tablet | White to off white round tablet | White to off white round tablet | White to off white round tablet | White to off white round tablet |
| **Disintegration (s)** | | 15-20 s | 15-20 s | 10-15 s | 10-15 s | 0-5 s | 0-5 s |
| **Dissolution (%)** | 45 min | 99% | 99% | 99% | 98% | 99% | 99% |
| **Water Content (%w/w)** | | 1.4% | 1.5% | 1.5% | 1.6% | 1.6% | 1.8% |

A second sample of KVD900 250 mg ODT was packaged in a Aclar/Alu blister pack and stored at conditions of 40°C/75%RH. Tests on the sample of KVD900 250 mg ODT were carried out as described in **Table 6.**

**Table 6: Stability Data for KVD900 250 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| **Appearance** | | White to off white round tablet | White to off white round tablet | White to off white round tablet | White to off white round tablet |
| **Disintegration (s)** | | 15-20 s | 15-20 s | 10-15 s | 15-20 s |
| **Dissolution (%)** | 45 min | 99% | 99% | 99% | 101% |
| **Water Content (%w/w)** | | 1.4% | 1.6% | 1.7% | 2.0% |

### Example 6 - Hardness testing of the orodispersible tablets of the present invention

The Hardness of the dosage unit can be obtained in accordance with USP <1217>. A single tablet is placed into tablet hardness apparatus and the hardness value recorded. The analysis is repeated 10 times and the average value reported.

### Results

Hardness data for the orodispersible tablets of the present invention are provided in **Tables 7-10.**

**Table 7: Hardness of KVD900 300 mg ODT, Storage Condition 25°C/60%RH in Aclar/Alu Blister**

| **Storage Condition 25°C/60%RH** | **Storage Time (months)** | | | | | |
|---|---|---|---|---|---|---|
| | **Initial** | **1** | **3** | **6** | **9** | **12** |
| **Hardness (N)** | 90 | 94 | 91 | 90 | 94 | 91 |

**Table 8: Hardness of KVD900 300 mg ODT, Storage Condition 40°C/75%RH in Aclar/Alu Blister**

| **Storage Condition 40°C/75%RH** | **Storage Time (months)** | | | |
|---|---|---|---|---|
| | **Initial** | **1** | **3** | **6** |
| **Hardness (N)** | 90 | 96 | 95 | 95 |

**Table 9: Hardness of KVD900 250 mg ODT, Storage Condition 25°C/60%RH in Aclar/Alu Blister**

| **Storage Condition 25°C/60%RH** | **Storage Time (months)** | | | | | |
|---|---|---|---|---|---|---|
| | **Initial** | **1** | **3** | **6** | **9** | **12** |
| **Hardness (N)** | 89 | 81 | 81 | 82 | 72 | 75 |

**Table 10: Hardness of KVD900 250 mg ODT, Storage Condition 40°C/75%RH in Aclar/Alu Blister**

| **Storage Condition 40°C/75%RH** | **Storage Time (months)** | | | |
|---|---|---|---|---|
| | **Initial** | **1** | **3** | **6** |
| **Hardness (N)** | 89 | 79 | 83 | 86 |

### Example 7 - Assay and Related Substances testing of the orodispersible tablets of the present invention

### Typical chromatographic conditions:

| | |
|---|---|
| HPLC Column: | Waters CSH C18, 2.1 x 75 mm, 1.7 micron (or equivalent) |
| Flow rate: | 0.21 mL/min |
| Detector wavelength: | 225 nm |
| PDA: | 190 nm to 400 nm PDA for Identity |
| Injection volume: | 1.0 microliter (Assay, Content Uniformity, Identity) |
| | 2.0 microliter (Related substances) |
| Column Temperature: | 25°C |
| Mobile Phase A: | HPLC Water + 0.05% formic acid |
| Mobile Phase B: | HPLC MeCN + 0.05% formic acid |
| Wash/Purge Solvent | 90:10 MeCN: Water |
| Gradient: | See Table 11 |
| Sample concentration: | ~0.12 mg/mL (Assay); ~0.24 mg/mL (Related Substances) |

**Table 11: Details of Gradient Elution**

| **Time (min)** | **%A** | **%B** |
|---|---|---|
| 0.00 | 95 | 5 |
| 7.50 | 50 | 50 |
| 10.00 | 5 | 95 |
| 10.05 | 95 | 5 |
| 18.00 | 95 | 5 |

| | | |
|---|---|---|
| Total run time = 18.0 minutes | | |

### Results

Assay and Related Substances data for the orodispersible tablets of the present invention are provided in **Tables 12-15.** These data support the excellent stability of the orodispersible tablets of the present invention.

**Table 12: Assay and Related Substances data for KVD900 300 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| **Assay by HPLC (% Label Claim)** | | 99.3% | 98.5% | 97.5% | 97.9% | 99.9% | 100.1% |
| **Related Substances by HPLC (% area)** | Individual | RRT 0.76: 0.11% | RRT 0.70: 0.07% | RRT 0.70: 0.07% | RRT 0.70: 0.06% | RRT 0.70: 0.09% | RRT 0.71: 0.11% |
| | | RRT 0.94: 0.11% | RRT 0.93: 0.08% | RRT 0.93: 0.07% | RRT 0.93: 0.05% | RRT 0.93: 0.07% | RRT 0.94: 0.08% |
| | Total | 0.2% | 0.2% | 0.1% | 0.1% | 0.2% | 0.2% |

**Table 13: Assay and Related Substances data for KVD900 300 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| **Assay by HPLC (% Label Claim)** | | 99.3% | 99.2% | 97.9% | 98.0% |
| **Related Substances by HPLC (% area)** | Individual | RRT 0.76: 0.11% | RRT 0.70: 0.07% | RRT 0.70: 0.07% | RRT 0.70: 0.06% |
| | | RRT 0.94: 0.11% | RRT 0.93: 0.08% | RRT 0.93: 0.07% | RRT 0.93: 0.05% |
| | Total | 0.2% | 0.1% | 0.1% | 0.1% |

**Table 14: Assay and Related Substances data for KVD900 250 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| **Assay by HPLC** (% **Label Claim)** | | 98.0% | 98.4% | 98.6% | 98.9% | 98.2% | 99.0% |
| **Related Substances by HPLC (% area)** | Individual | RRT 0.70: 0.13% | RRT 0.70: 0.13% | RRT 0.70: 0.13 | RRT 0.70: 0.17% | RRT 0.70: 0.12% | RRT 0.58 0.12% |
| | | | | | | | RRT 0.63 0.10% |
| | | | | | | | RRT 1.39 <LOQ |
| | Total | 0.1% | 0.1% | 0.1% | 0.2% | 0.1% | 0.2% |

**Table 15: Assay and Related Substances data for KVD900 250 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| **Appearance** | | White to off white round tablet | White to off white round tablet | White to off white round tablet | White to off white round tablet |
| **Assay by HPLC** (% **Label Claim)** | | 98.0% | 98.7% | 99.2% | 99.4% |
| **Related Substances by HPLC (% area)** | Individual | RRT 0.70: 0.13% | RRT 0.70: 0.13% | RRT 0.70: 0.13% | RRT 0.70: 0.15% |
| | Total | 0.1% | 0.1% | 0.1% | 0.2% |

The Assay of the orodispersible tablets of the present invention meet the acceptance criteria of 90-110% label claim. "Label claim" refers to the dose on the label. Accordingly, for the 300 mg ODTs of Tables 12 and 13, the acceptance limits of 90-110% equate to 270 mg - 330 mg. For the 250 mg ODTs of Tables 14 and 15, the acceptance limits of 90-110% equate to 225 mg - 275 mg.

The Related Substances analysis of the orodispersible tablets of the present invention meet the acceptance criteria of NMT 0.5% of any Individual Impurity and NMT 2.0% Total Impurities.

### Example 8 - Microbial Limits

Total viable aerobic count is performed by the Pour-Plate Method according to USP<61>/Ph Eur 2.6.12. The test for specified microorganisms on *E. coli* is performed according to USP<62>/Ph Eur 2.6.13.

### Results

Microbial Limits data for the orodispersible tablets of the present invention are provided in **Tables 16-19.**

**Table 16: Microbial Limits data for KVD900 300 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| | TAMC | <10 CFU/g | NT | NT | <10 CFU/g | NT | <10 CFU/g |
| **Micro** | TYMC | <10 CFU/g | NT | NT | <10 CFU/g | NT | <10 CFU/g |
| | *E. coli* | Absent | NT | NT | Absent | NT | Absent |

| | | | | | | | |
|---|---|---|---|---|---|---|---|
| NT = Not Tested | | | | | | | |

**Table 17: Microbial Limits data for KVD900 300 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| | TAMC | <10 CFU/g | NT | NT | <10 CFU/g |
| **Micro** | TYMC | <10 CFU/g | NT | NT | <10 CFU/g |
| | *E. coli* | Absent | NT | NT | Absent |

| | | | | | |
|---|---|---|---|---|---|
| NT = Not Tested | | | | | |

**Table 18: Microbial Limits data for KVD900 250 mg ODT, Storage Condition 25°C/60%RH in Aclar/Alu Blister**

| **Storage Condition 25°C/60%RH** | | **Storage Time (months)** | | | | | |
|---|---|---|---|---|---|---|---|
| | | **Initial** | **1** | **3** | **6** | **9** | **12** |
| | TAMC | <10 CFU/g | NT | NT | <10 CFU/g | NT | <10 CFU/g |
| **Micro** | TYMC | <10 CFU/g | NT | NT | <10 CFU/g | NT | <10 CFU/g |
| | *E. coli* | Absent | NT | NT | Absent | NT | Absent |

| | | | | | | | |
|---|---|---|---|---|---|---|---|
| NT = Not Tested | | | | | | | |

**Table 19: Microbial Limits data for KVD900 250 mg ODT, Storage Condition 40°C/75%RH in Aclar/Alu Blister**

| **Storage Condition 40°C/75%RH** | | **Storage Time (months)** | | | |
|---|---|---|---|---|---|
| | | **Initial** | **1** | **3** | **6** |
| | TAMC | <10 CFU/g | NT | NT | <10 CFU/g |
| **Micro** | TYMC | <10 CFU/g | NT | NT | <10 CFU/g |
| | *E. coli* | Absent | NT | NT | Absent |

| | | | | | |
|---|---|---|---|---|---|
| NT = Not Tested | | | | | |

The Microbial Limits data in Tables 16-19 demonstrate that the orodispersible tablets of the present invention meet the acceptance criteria of TAMC ≤10³ CFU/g, TYMC ≤10² CFU/g and *E. Coli* absent in 1 g.

### Example 9 - Phase 1 trial: Pharmacokinetic (PK) Study

### Aim

To compare the single dose PK of KVD900 following administration of orodispersible tablets (ODTs) or film-coated tablets in healthy adult subjects. A second was to determine the safety and tolerability of a single dose of KVD900 following administration of ODTs or film-coated tablets in healthy adult subjects under fasting conditions.

### Methods

This study was an open label, randomized, single-dose, 3-way crossover pharmacokinetic study.

Thirty-six (36) healthy, adult, male and female subjects were enrolled.

On Day 1 of each period, a single dose of KVD900 was administered at two different strengths as an ODT under fasting conditions (Treatment A or B) or as a film-coated tablet under fasting conditions (Treatment C) in a 3-period crossover fashion.

Treatments groups A-C were as follows:
- **Treatment A:** 600 mg KVD900 ODTs (2 x 300 mg ODTs) at Hour 0 on Day 1.
- **Treatment B:** 500 mg KVD900 ODTs (2 x 250 mg ODTs) at Hour 0 on Day 1.
- **Treatment C:** 600 mg KVD900 film-coated tablets (2 x 300 mg tablets, FCTs) at Hour 0 on Day 1.

The film coated tablets were those used in the phase 2 study described herein (NCT04208412).

There was a washout period of at least 4 days between the dose in Period 1 and Period 2 and the dose in Period 2 and Period 3.

Pharmacokinetic sampling for KVD900 was conducted pre-dose and up to 48 hours after dosing.

The following PK parameters were calculated for KVD900 in plasma: AUC₀₋ₜ, AUC_{0-inf}, AUC_{%extrap}, Cₘₐₓ, T_{lag}, Tₘₐₓ, Kel, t½, CL/F, and Vz/F.

An analysis of variance (ANOVA) was performed on the natural log (In) transformed AUC₀₋ₜ, AUC_{0-inf}, AUC_{%extrap} and Cₘₐₓ, using the appropriate statistical procedure. The 90% confidence intervals (Cis) of the ratios of least squares means (LSM) of the In transformed AUC₀₋ₜ, AUC_{0-inf}, AUC_{%extrap} and Cₘₐₓ of KVD900 for the following comparisons of interest were estimated:
- Treatment A versus Treatment C
- Treatment B versus Treatment C

A comparison of the pharmacokinetic properties of KVD900 following administration of the orodispersible tablets (ODTs) according to the present invention or film-coated tablets (FCT) is shown in **Table 20.**

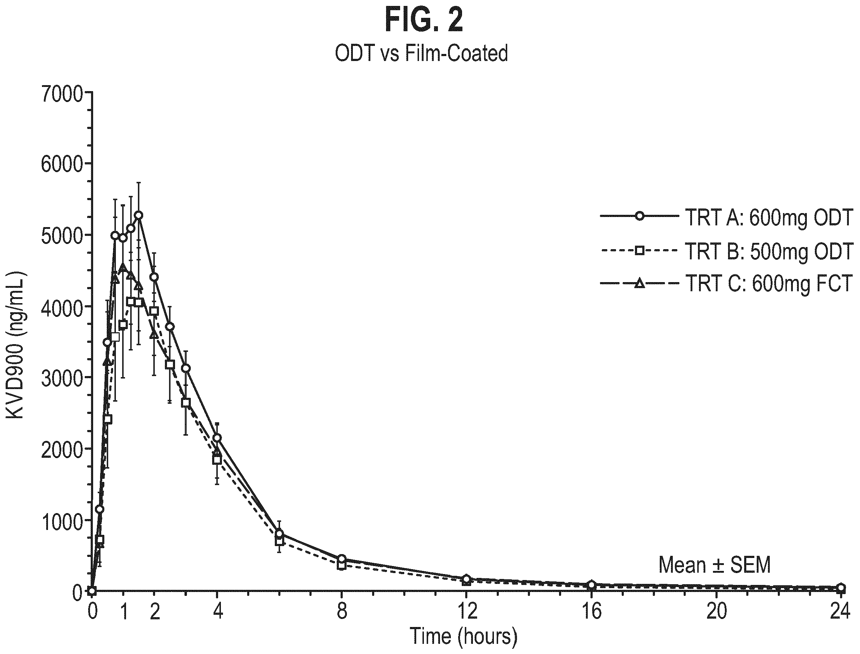

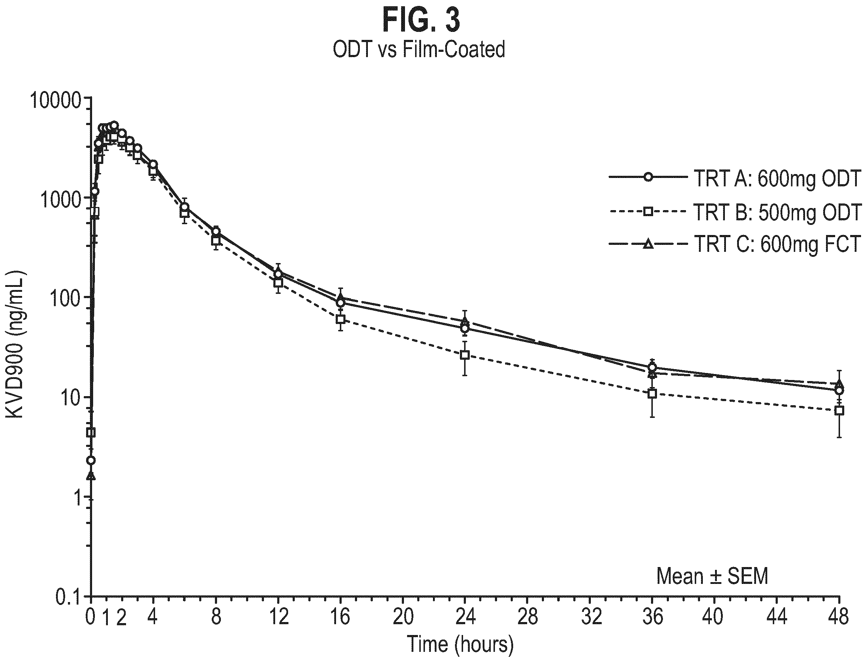

Preliminary PK data from the Phase 1 study is shown in Figures 2 and 3. Specifically, a PK curve is shown in **Figure 2** and a LogPK curve is shown in **Figure 3****.** These figures show that the ODTs of the invention have a comparable PK profile to the FCTs that have been demonstrated as safe and effective in KVD900's phase 2 study.

**Table 20: Preliminary PK data from the Phase 1 study described herein.**

| | | **600 mg ODT** | **500 mg ODT** | **600 mg FCT** |
|---|---|---|---|---|
| **Cmax** | **Mean** | 6769 | 5518 | 5636 |
| | **Geomean** | 6118 | 5066 | 5042 |
| | **Range** | [932, 12100] | [1190, 9450] | [1440, 13600] |
| | **95%CI (±)** | 877 | 665 | 904 |
| | **%CV** | 40% | 37% | 49% |
| **AUCO-inf** | **Mean** | 21158 | 16882 | 19298 |
| | **Geomean** | 19856 | 15921 | 18154 |
| | **Range** | [5991, 37204] | [6882, 28234] | [7750, 31786] |
| | **95%CI (±)** | 2345 | 1789 | 2159 |
| | **%CV** | 34% | 32% | 34% |
| **Tmax** | **Median [minimum, maximum]** | 1 [0.5, 2.5] | 1.25 [0.5, 4] | 1 [0.5, 3] |

Further PK data from the Phase 1 study is described below.

A comparison of the pharmacokinetic properties of plasma KVD900 following administration of a single oral dose of the orodispersible tablets (ODTs) according to the present invention or film-coated tablets (FCT) is shown in **Table 21.**

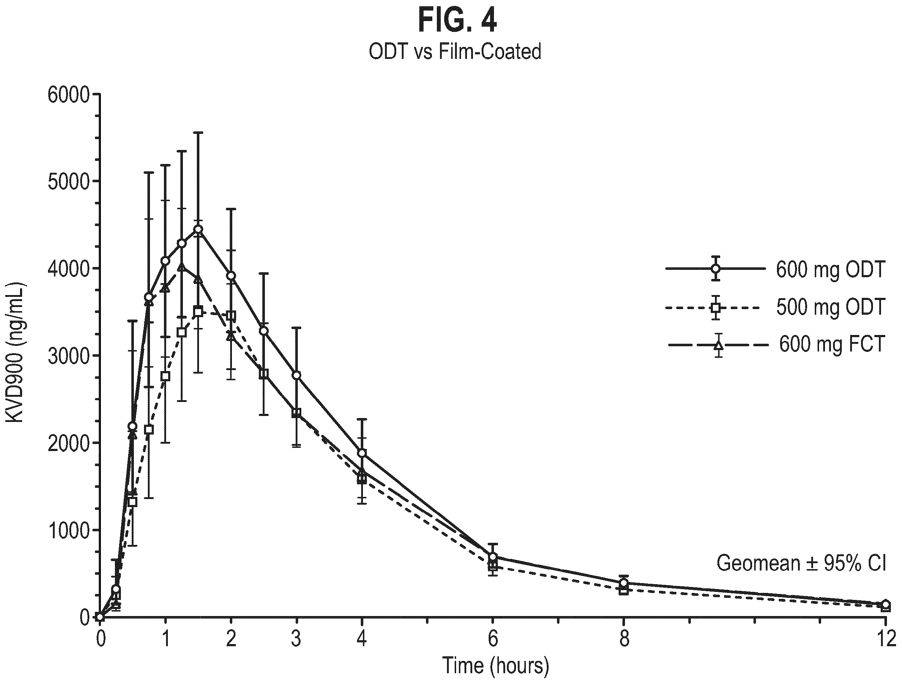

Further PK data from the Phase 1 study is shown in Figure 4. Specifically, **Figure 4** shows the geometric mean plasma concentrations after a single dose of KVD900 ODT or FCT.

**Table 21: Further PK data from the Phase 1 study described herein: plasma KVD900 PK parameters following administration of a single oral dose.**

| | | **600 mg ODT** | **500 mg ODT** | **600 mg FCT** |
|---|---|---|---|---|
| **AUC0-t (ng*hr/mL)** | **n** | 36 | 36 | 36 |
| | **Geomean** | 19600 | 15800 | 17900 |
| | **%CV** | 40.4% | 37.9% | 38.5% |
| **AUCO-inf (ng*hr/mL)** | **n** | 35 | 36 | 35 |
| | **Geomean** | 20500 | 16000 | 18300 |
| | **%CV** | 33.2% | 37.4% | 37.6% |
| **Cmax (ng/mL)** | **n** | 36 | 36 | 36 |
| | **Geomean** | 6120 | 5070 | 5040 |
| | **%CV** | 54.2% | 48.6% | 51.5% |
| **Tmax (hr)** | **n** | 36 | 36 | 36 |
| | **Median [minimum, maximum]** | 0.99 [0.50, 4.1] | 1.3 [0.50, 4.0] | 0.99 [0.50, 3.0] |

| | | | | |
|---|---|---|---|---|
| AUC0-inf, area under the concentration-time curve from time 0 extrapolated to infinity; AUC0-t, area under the concentration-time curve for time 0 to the last observed nonzero concentration; Cmax, maximum observed plasma concentration; CV%, coefficient of variation; Tmax, time to reach maximum observed plasma concentration. | | | | |

A summary of statistical comparisons of plasma KVD900 pharmacokinetic parameters following administration of a single dose of 600 mg ODT vs 600 mg FCT is shown in **Table 22.**

**Table 22: Summary of statistical comparisons of plasma KVD900 PK parameters following administration of a single dose of 600 mg ODT versus 600 mg FCT (Treatment A versus Treatment C)**

| | | **600 mg ODT** | **600 mg FCT** |
|---|---|---|---|
| **AUC0-t (ng*hr/mL)** | **n** | 36 | 36 |
| | **Geometric LSMs** | 19600 | 17900 |
| | **GMR (%)** | 109.33 | |
| | **90% CI** | 101.28-118.01 | |
| | **Intra-Participant CV%** | 19.63 | |
| **AUCO-inf (ng*hr/mL)** | **n** | 35 | 35 |
| | **Geometric LSMs** | 20200 | 18300 |
| | **GMR (%)** | 110.83 | |
| | **90% CI** | 103.03-119.22 | |
| | **Intra-Participant CV%** | 18.32 | |
| **Cmax (ng/mL)** | **n** | 36 | 36 |
| | **Geometric LSMs** | 6120 | 5040 |
| | **GMR (%)** | 121.35 | |
| | **90% CI** | 108.16-136.14 | |
| | **Intra-Participant CV%** | 29.90 | |

| | | | |
|---|---|---|---|
| ANOVA, analysis of variance; AUC0-inf, area under the concentration-time curve from time 0 extrapolated to infinity; AUC0-t, area under the concentration-time curve for time 0 to the last observed nonzero concentration; Cmax, maximum observed concentration; CV%, coefficient of variation; GMR, geometric mean ratio; LSM, least squares mean. Geometric LSMs are calculated by exponentiating the LSMs derived from the ANOVA. GMR was calculated as 100 × (test/reference). Intra-participant CV% was calculated as 100 × square root (exp[MSE]-1), where mean squared error (MSE) represents residual variance from ANOVA. | | | |

A summary of statistical comparisons of plasma KVD900 pharmacokinetic parameters following administration of a single dose of 500 mg ODT vs 600 mg FCT is shown in **Table 23.**

**Table 23: Summary of statistical comparisons of plasma KVD900 PK parameters following administration of a single dose of 500 mg ODT versus 600 mg FCT (Treatment B versus Treatment C)**

| | | **500 mg ODT** | **600 mg FCT** |
|---|---|---|---|
| **AUC0-t (ng*hr/mL)** | **n** | 36 | 36 |
| | **Geometric LSMs** | 15800 | 17900 |
| | **GMR (%)** | 88.34 | |
| | **90% CI** | 81.84-95.35 | |
| | **Intra-Participant CV%** | 19.63 | |
| **AUCO-inf (ng*hr/mL)** | **n** | 36 | 35 |
| | **Geometric LSMs** | 16000 | 18300 |
| | **GMR (%)** | 87.44 | |
| | **90% CI** | 81.35-93.99 | |
| | **Intra-Participant CV%** | 18.32 | |
| **Cmax (ng/mL)** | **n** | 36 | 36 |
| | **Geometric LSMs** | 5070 | 5040 |
| | **GMR (%)** | 100.48 | |
| | **90% CI** | 89.56-112.72 | |
| | **Intra-Participant CV%** | 29.90 | |

| | | | |
|---|---|---|---|
| ANOVA, analysis of variance; AUC0-inf, area under the concentration-time curve from time 0 extrapolated to infinity; AUC0-t, area under the concentration-time curve for time 0 to the last observed nonzero concentration; Cmax, maximum observed concentration; CV%, coefficient of variation; GMR, geometric mean ratio; LSM, least squares mean. | | | |

Singles doses of 500 mg or 600 mg KVD900 administered in ODT or FCT were well tolerated in healthy adult participants enrolled in the trial.

These further data confirm that the ODTs of the invention have a comparable PK profile to the FCTs that have been demonstrated as safe and effective in KVD900's phase 2 study.

### Example 10 - Preparation of alternative orodispersible tablets of the present invention

Alternative ODTs of KVD900 according to Tables 24-26 were prepared according to the method described in Example 2.

**Table 24: Alternative Compositions of the ODTs of the present invention (unflavoured)**

| **Components** | **Blend composition** | **KVD900 300 mg ODT** | **KVD900 150 mg ODT** |
|---|---|---|---|
| | **% w/w** | **mg** | **mg** |
| KVD900 | 30.00 | 300.00 | 150.00 |
| Mannitol (Granulated) | 26.87 | 268.70 | 134.35 |
| Mannitol (Mannogem EZ) | 26.87 | 268.70 | 134.35 |
| Silicified Microcrystalline Cellulose | 10.01 | 100.10 | 50.05 |
| Crospovidone | 5.00 | 50.00 | 25.00 |
| Sucralose | 0.50 | 5.00 | 2.50 |
| Silicon Dioxide | 0.75 | 7.50 | 3.75 |
| **Total** | **100.00** | **1000.00** | **500.00** |

**Table 25: Alternative Compositions of the ODTs of the present invention (lemon flavour)**

| **Components** | **Blend composition** | **KVD900 300 mg ODT** |
|---|---|---|
| | **% w/w** | **mg** |
| KVD900 | 30.00 | 300.00 |
| Mannitol (Granulated) | 26.85 | 268.50 |
| Mannitol (Mannogem EZ) | 26.85 | 268.50 |
| Silicified Microcrystalline Cellulose | 9.69 | 96.90 |
| Crospovidone | 5.00 | 50.00 |
| Sucralose | 0.50 | 5.00 |
| Silicon Dioxide | 0.75 | 7.50 |
| Lemon Flavour | 0.36 | 3.60 |
| **Total** | **100.00** | **1000.00** |

**Table 26: Alternative Compositions of the ODTs of the present invention (mixed berry flavour)**

| **Components** | **Blend composition** | **KVD900 300 mg ODT** | **KVD900 75 mg ODT** | **KVD900 50 mg ODT** | **KVD900 25 mg ODT** |
|---|---|---|---|---|---|
| | **% w/w** | **mg** | **mg** | **mg** | **mg** |
| KVD900 | 30.00 | 300 | 75 | 50 | 25 |
| Mannitol (Granulated) | 26.85 | 268.50 | 67.13 | 44.75 | 22.38 |
| Mannitol (Mannogem EZ) | 26.85 | 268.50 | 67.13 | 44.75 | 22.38 |
| Silicified Microcrystalline Cellulose | 9.74 | 97.40 | 24.35 | 16.23 | 8.12 |
| Crospovidone | 5.00 | 50.00 | 12.50 | 8.33 | 4.17 |
| Sucralose | 0.50 | 5.00 | 1.25 | 0.83 | 0.42 |
| Silicon Dioxide | 0.75 | 7.50 | 1.88 | 1.25 | 0.63 |
| Mixed Berry Flavour | 0.31 | 3.10 | 0.78 | 0.52 | 0.26 |
| **Total** | **100.00** | **1000.00** | **250.00** | **166.67** | **83.33** |

The orodispersible tablets (75mg, 50mg, 25mg) of the present invention according to Table 26 have been made using a single blend at a scale of 4kg. This 4kg blend was then split to provide approximately the same number of tablets for each strength:
- 75 mg: 1800g (7,200 tablets)
- 50mg: 1400g (8,400 tablets)
- 25mg: 800g (9,600 tablets)

The orodispersible tablets of the present invention according to Tables 24-26 have been prepared, demonstrating the versatility of the mixtures/blends used to manufacture orodispersible tablets of the present invention. In particular, rapid disintegration times are obtained regardless of size and/or strength of the orodispersible tablets of the present invention.

### Example 11 - Further uniformity and disintegration testing of the orodispersible tablets of the invention

### KVD900 300 mg ODTs debossed with a logo and a score line

KVD900 300 mg ODTs were made according to Table 24. These KVD900 300 mg ODTs were debossed with a logo and a score line.

Disintegration testing of these KVD900 300 mg ODTs debossed with a logo and a score line was performed according to the method in Example 3. Specifically, the disintegration testing was conducted in accordance with the following protocol - USP <701> and Ph Eur 2.9.1.

Disintegration data for KVD900 300 mg ODT debossed with a logo and a score line is provided in **Table 27.**

**Table 27: Disintegration data for KVD900 300 mg ODT debossed with a logo and a score line**

| | **Shape** | **Disintegration time (sec)** |
|---|---|---|
| | | Target: < 30 s |
| KVD900 300 mg ODT debossed with a logo and a score line | Round | < 10 |

The disintegration specification of KVD900 300 mg ODT debossed with a logo and a score line meets the FDA guidance criteria of < 30 seconds.

The uniformity of the KVD900 300 mg ODTs debossed with a logo and a score line (before and after splitting) was measured according to the method in Example 4. Specifically, the uniformity of the dosage unit was obtained using mass variation in accordance with the following protocol - USP<905>.

Content uniformity data for KVD900 300 mg ODT debossed with a logo and a score line before and after splitting is provided in **Table 28.**

**Table 28: Content uniformity data for KVD900 300 mg ODT debossed with a logo and a score line before and after splitting**

| | **Uniformity** |
|---|---|
| | Target: Complies with USP<905> |
| Before splitting | Complies |
| After splitting (halved tablets) | Complies |

The uniformity of KVD900 300 mg ODT debossed with a logo and a score line meets the acceptance criteria in accordance with USP<905>. The uniformity of the halved subdivided tablets also meets the acceptance criteria in accordance with USP<905>.

These data support that when the orodispersible tablets of the present invention that are debossed with a score line are split, the multiple subdivided tablets retain acceptable content uniformity.

### KVD900 150mg ODTs debossed with a logo and a score line

KVD900 150mg ODTs were made according to Table 24. These KVD900 150 mg ODTs were debossed with a logo and a score line.

Disintegration testing of these KVD900 150 mg ODTs debossed with a logo and a score line was performed according to the method in Example 3. Specifically, the disintegration testing was conducted in accordance with the following protocol - USP <701> and Ph Eur 2.9.1.

Disintegration data for KVD900 150 mg ODT debossed with a logo and a score line is provided in **Table 29.**

**Table 29: Disintegration data for KVD900 150 mg ODT debossed with a logo and a score line**

| | **Shape** | **Disintegration time (sec)** |
|---|---|---|
| | | Target: < 30 s |
| KVD900 150 mg ODT debossed with a logo and a score line | Oval | < 30 |

The disintegration specification of KVD900 150 mg ODT debossed with a logo and a score line meets the FDA guidance criteria of < 30 seconds.

The uniformity of the KVD900 150 mg ODTs debossed with a logo and a score line (before and after splitting) was measured according to the method in Example 4. Specifically, the uniformity of the dosage unit was obtained using mass variation in accordance with the following protocol - USP<905>.

Content uniformity data for KVD900 150 mg ODT debossed with a logo and a score line before and after splitting is provided in **Table 30.**

**Table 30: Content uniformity data for KVD900 150 mg ODT debossed with a logo and a score line before and after splitting**

| | **Uniformity** |
|---|---|
| | Target: Complies with USP<905> |
| Before splitting | Complies |
| After splitting (halved tablet) | Complies |

The uniformity of KVD900 150 mg ODT debossed with a logo and a score line meets the acceptance criteria in accordance with USP<905>. The uniformity of the halved subdivided tablets also meets the acceptance criteria in accordance with USP<905>.

These data support that when the orodispersible tablets of the present invention that are debossed with a score line are split, the multiple subdivided tablets retain acceptable content uniformity.

### KVD900 75 mg, 50 mg, 25 mg ODT debossed with tablet strength

KVD900 75 mg, 50 mg and 25mg ODTs were made according to Table 28. These KVD900 75 mg, 50 mg and 25mg ODTs were debossed with the tablet strength.

Disintegration testing of the KVD900 75 mg, 50 mg and 25mg ODTs debossed with the tablet strength was performed according to the method in Example 3. Specifically, the disintegration testing was conducted in accordance with the following protocol - USP <701> and Ph Eur 2.9.1.

The uniformity of the KVD900 75 mg, 50 mg and 25mg ODTs debossed with the tablet strength was measured according to the method in Example 4. Specifically, the uniformity of the dosage unit was obtained using mass variation in accordance with the following protocol - USP<905>.

Content uniformity data and disintegration data for KVD900 75 mg, 50 mg and 25mg ODTs debossed with tablet strength is provided in **Table 31.**

**Table 31: Content uniformity data and disintegration data for KVD900 75 mg, 50 mg and 25mg ODTs debossed with tablet strength**

| | **Shape** | **Uniformity** | **Disintegration time (sec)** |
|---|---|---|---|
| | | Target: Complies with USP<905> | Target: < 30 s |
| KVD900 75 mg ODT debossed with tablet strength ("75") | Caplet | Complies | <5 |
| KVD900 50 mg ODT debossed with tablet strength ("50") | Caplet | Complies | < 10 |
| KVD900 25 mg ODT debossed with tablet strength ("25") | Caplet | Complies | < 10 |

The uniformity of KVD900 75 mg, 50 mg and 25 mg ODTs debossed with tablet strength meets the acceptance criteria in accordance with USP<905>.

These data support that the mixtures/blends of the present invention can be used to manufacture orodispersible tablets of varying size and/or strength without compromising the uniformity of the dosage unit.

The disintegration specification of KVD900 75 mg, 50 mg and 25 mg ODTs debossed with tablet strength meets the FDA guidance criteria of < 30 seconds.

These data demonstrate the versatility of the mixtures/blends of the present invention. In particular, these data demonstrate that the mixtures/blends of the present invention can be used to manufacture orodispersible tablets of varying size and/or strength without compromising the rapid disintegration times achieved by the orodispersible tablets of the present invention.

## Claims

1. An orodispersible tablet comprising between 26 and 50 wt% of KVD900, or a pharmaceutically acceptable salt and/or solvate thereof,
wherein KVD900 is,

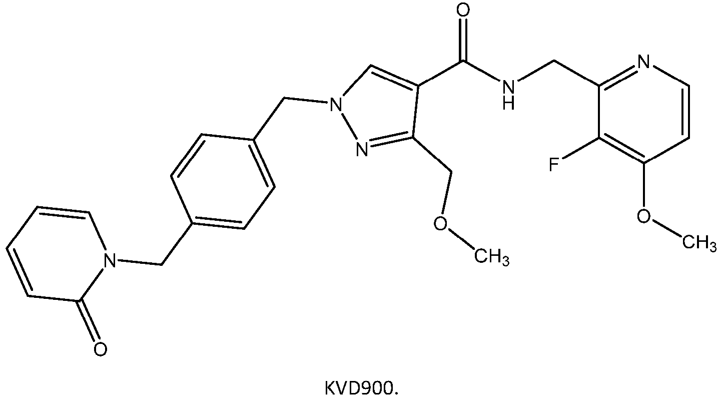

2. The orodispersible tablet according to claim 1, comprising:
between 100 mg and 1000 mg of KVD900, or a pharmaceutically acceptable salt and/or solvate thereof; or
between 200 mg and 350 mg of KVD900, or a pharmaceutically acceptable salt and/or solvate thereof; or
250 mg of KVD900, or a pharmaceutically acceptable salt and/or solvate thereof; or
275 mg of KVD900, or a pharmaceutically acceptable salt and/or solvate thereof; or
300 mg of KVD900, or a pharmaceutically acceptable salt and/or solvate thereof.

3. The orodispersible tablet according to any one of claims 1 to 2, wherein the orodispersible tablet comprises between 26 and 40 wt% of KVD900, or a pharmaceutically acceptable salt and/or solvate thereof,
preferably wherein the orodispersible tablet comprises 30 wt% of KVD900, or a pharmaceutically acceptable salt and/or solvate thereof.

4. The orodispersible tablet according to any one of claims 1 to 3, wherein the orodispersible tablet comprises:
a) one or more disintegrants present at between 1 and 15 wt% of the orodispersible tablet; or
one or more disintegrants present at between 1 and 10 wt% of the orodispersible tablet; or
one or more disintegrants present at between 4 and 5 wt% of the orodispersible tablet; or
one or more disintegrants present at 5 wt% of the orodispersible tablet;
optionally wherein the one or more disintegrants are selected from (i) polyvinylpyrrolidone e.g. crospovidone, (ii) sodium starch glycolate and (iii) croscarmellose sodium, preferably wherein the orodispersible tablet comprises polyvinylpyrrolidone e.g. crospovidone; and/or
b) one or more fillers present at between 30 and 80 wt% of the orodispersible tablet; or
wherein the orodispersible tablet comprises one or more fillers present at between 50 and 60 wt% of the orodispersible tablet; or
wherein the orodispersible tablet comprises one or more fillers present at between 53 and 54 wt% of the orodispersible tablet;
optionally wherein the one or more fillers are selected from (i) isomalt, (ii) lactose and its derivatives e.g. lactose monohydrate, spray dried lactose, anhydrous lactose, (iii) sorbitol, (iv) mannitol e.g. granulated mannitol, powdered mannitol, spray-dried mannitol, (v) sucrose, (vi) starch, (vii) pregelatinized starch, and (viii) mixtures thereof, preferably wherein the orodispersible tablet comprises one or more fillers selected from granulated mannitol and spray-dried mannitol; and/or
c) one or more binders present at between 5 and 40 wt% of the orodispersible tablet; or
wherein the orodispersible tablet comprises one or more binders present at between 5 and 30 wt% of the orodispersible tablet; or
wherein the orodispersible tablet comprises one or more binders present at between 5 and 10 wt% of the orodispersible tablet; or
wherein the orodispersible tablet comprises one or more binders present at 10 wt% of the orodispersible tablet;
optionally wherein the one or more binders selected are from microcrystalline cellulose e.g. silicified microcrystalline cellulose, and hydroxypropylmethyl cellulose, preferably wherein the orodispersible tablet comprises silicified microcrystalline cellulose.

5. The orodispersible tablet according to any one of claims 1 to 4, wherein the orodispersible tablet further comprises:
a) a flow enhancer; preferably
i) wherein the flow enhancer is present at between 0.5 to 3 wt% of the orodispersible tablet; or
ii) wherein the flow enhancer is present at 0.75 wt% of the orodispersible tablet;
optionally wherein the flow enhancer is silicon dioxide; and/or
b) one or more sweeteners, preferably
wherein the one or more sweeteners are present at between 0.1 and 1 wt% of the orodispersible tablet;
optionally wherein the one or more sweeteners selected from sucralose, aspartame, sugar derivates, dextrose, polydextrose, xylitol, fructose, sucrose, lactitol, maltitol, and sodium saccharin and mixtures thereof, preferably wherein the orodispersible tablet comprises sucralose as a sweetener;
and/or
c) one or more flavourings, preferably
wherein the one or more flavourings are present at between 0.03 and 0.4 wt% of the orodispersible tablet;
optionally wherein the one or more flavourings are selected from lemon flavouring, mixed berry flavouring, grape flavouring, peppermint natural flavouring, mint flavouring, banana flavouring, chocolate flavouring, maple flavouring, strawberry flavouring, a raspberry flavouring, a cherry flavouring, orange flavouring, and a vanilla flavouring and mixtures thereof, optionally wherein the orodispersible tablet comprises peppermint natural flavouring.

6. The orodispersible tablet according to any one of claims 1 to 5, comprising one or more of granulated mannitol, spray-dried mannitol, silicified microcrystalline cellulose, polyvinylpyrrolidone e.g. crospovidone, sucralose, silicon dioxide and peppermint natural flavour.

7. The orodispersible tablet according to any one of claims 1 to 6, wherein KVD900 is in its free base form.

8. The orodispersible tablet according to claim 7, wherein the orodispersible tablet comprises:
a) 30 wt% KVD900;
b) 26.87 wt% granulated mannitol;
c) 26.87 wt% spray-dried mannitol;
d) 10.01 wt% silicified microcrystalline cellulose;
e) 5 wt% crospovidone;
f) 0.5 wt% sucralose; and
g) 0.75 wt% silicon dioxide,
optionally further comprising a trace amount of magnesium stearate.

9. The orodispersible tablet according to claim 8, comprising 300 mg KVD900, 150 mg KVD900, or 75 mg KVD900.

10. The orodispersible tablet according to claim 9, comprising 300.00 mg KVD900, 268.70 mg granulated mannitol, 268.70 mg spray-dried mannitol, 100.10 mg silicified microcrystalline cellulose, 50.00 mg crospovidone, 5.00 mg sucralose, and 7.50 mg silicon dioxide.

11. A method of manufacturing an orodispersible tablet according to any one of claims 1 to 10, from a mixture comprising:
a) between 26 and 40 wt% of KVD900;
b) between 30 and 80 wt% of one or more fillers;
c) between 5 and 40 wt% of one or more binders;
d) between 1 and 10 wt% of one or more disintegrants,
the method comprising blending the components of the mixture to form a powder mixture and compressing into the orodispersible tablet.

12. The method of claim 11, wherein the mixture comprises:
a) 30 wt% KVD900;
b) 26.85 wt% of granulated mannitol and 26.85 wt% of spray-dried mannitol;
c) 10 wt% of silicified microcrystalline cellulose;
d) 5 wt% polyvinylpyrrolidone e.g. crospovidone.

13. An orodispersible tablet according to any one of claims 1 to 10 for use in a method for treating bradykinin mediated angioedema on-demand, said method comprising: administering one or more of the orodispersible tablets to a patient in need thereof on-demand.

14. The orodispersible tablet for use according to claim 13, wherein:
a) the bradykinin mediated angioedema is hereditary angioedema (HAE); or wherein the bradykinin mediated angioedema is bradykinin-mediated angioedema non-hereditary (BK AEnH); and/or
b) the patient is suffering from a laryngeal attack; and/or
c) the patient has difficulty swallowing whole tablets.

15. The orodispersible tablet for use according to any one of claims 13 to 14, wherein KVD900, or a pharmaceutically acceptable salt and/or solvate thereof, is administered:
a) at a dosage amount of 500 mg,
optionally wherein each dosage amount is sub-divided into two orodispersible tablets comprising 250 mg of KVD900; or
b) at a dosage amount of 550 mg,
optionally wherein each dosage amount is sub-divided into two orodispersible tablets comprising 275 mg of KVD900; or
c) at a dosage amount of 600 mg,
optionally wherein each dosage amount is sub-divided into two orodispersible tablets comprising 300 mg of KVD900.

## Patentansprüche

1. Schmelztablette, umfassend zwischen 26 und 50 Gew.-% KVD900 oder pharmazeutisch unbedenkliches Salz und/oder Solvat davon,
wobei es sich bei KVD900 um Folgendes handelt:

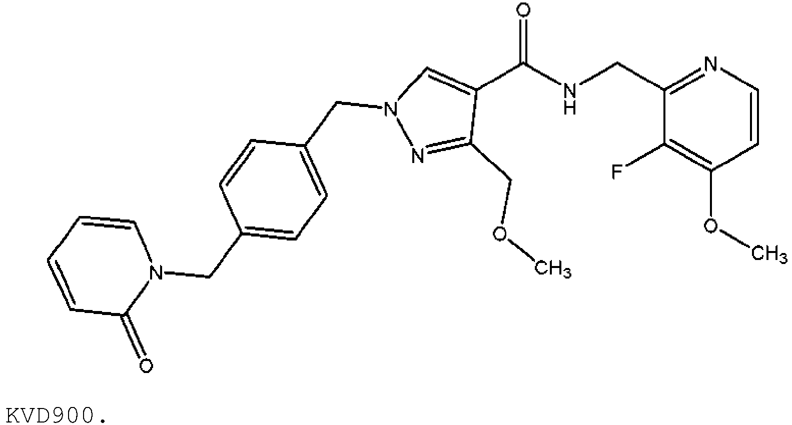

2. Schmelztablette nach Anspruch 1, umfassend:
zwischen 100 mg und 1000 mg KVD900 oder pharmazeutisch unbedenkliches Salz und/oder Solvat davon; oder
zwischen 200 mg und 350 mg KVD900 oder pharmazeutisch unbedenkliches Salz und/oder Solvat davon; oder
250 mg KVD900 oder pharmazeutisch unbedenkliches Salz und/oder Solvat davon; oder
275 mg KVD900 oder pharmazeutisch unbedenkliches Salz und/oder Solvat davon; oder
300 mg KVD900 oder pharmazeutisch unbedenkliches Salz und/oder Solvat davon.

3. Schmelztablette nach einem der Ansprüche 1 bis 2, wobei die Schmelztablette zwischen 26 und 40 Gew.-% KVD900 oder pharmazeutisch unbedenkliches Salz und/oder Solvat davon umfasst,
bevorzugt wobei die Schmelztablette 30 Gew.-% KVD900 oder pharmazeutisch unbedenkliches Salz und/oder Solvat davon umfasst.

4. Schmelztablette nach einem der Ansprüche 1 bis 3, wobei die Schmelztablette Folgendes umfasst:
a) ein oder mehrere Sprengmittel in einer Menge zwischen 1 und 15 Gew.-% der Schmelztablette; oder
ein oder mehrere Sprengmittel in einer Menge zwischen 1 und 10 Gew.-% der Schmelztablette; oder
ein oder mehrere Sprengmittel in einer Menge zwischen 4 und 5 Gew.-% der Schmelztablette; oder
ein oder mehrere Sprengmittel in einer Menge von 5 Gew.-% der Schmelztablette;
gegebenenfalls wobei das eine oder die mehreren Sprengmittel aus (i) Polyvinylpyrrolidon, z. B. Crospovidon, (ii) Natriumstärkeglykolat und (iii) Croscarmellose-Natrium ausgewählt sind, bevorzugt wobei die Schmelztablette Polyvinylpyrrolidon, z. B. Crospovidon, umfasst;
und/oder
b) einen oder mehrere Füllstoffe in einer Menge zwischen 30 und 80 Gew.-% der Schmelztablette; oder
wobei die Schmelztablette einen oder mehrere Füllstoffe in einer Menge zwischen 50 und 60 Gew.-% der Schmelztablette umfasst; oder
wobei die Schmelztablette einen oder mehrere Füllstoffe in einer Menge zwischen 53 und 54 Gew.-% der Schmelztablette umfasst;
gegebenenfalls wobei der eine oder die mehreren Füllstoffe aus (i) Isomalt, (ii) Lactose und ihren Derivaten, z. B. Lactose-Monohydrat, sprühgetrockneter Lactose, wasserfreier Lactose, (iii) Sorbit, (iv) Mannit, z. B. granuliertem Mannit, pulverförmigem Mannit, sprühgetrocknetem Mannit, (v) Saccharose, (vi) Stärke, (vii) vorgelatinisierter Stärke und (viii) Gemischen davon ausgewählt sind, bevorzugt wobei die Schmelztablette einen oder mehrere aus granuliertem Mannit und sprühgetrocknetem Mannit ausgewählte Füllstoffe umfasst;
und/oder
c) ein oder mehrere Bindemittel in einer Menge zwischen 5 und 40 Gew.-% der Schmelztablette; oder
wobei die Schmelztablette ein oder mehrere Bindemittel in einer Menge zwischen 5 und 30 Gew.-% der Schmelztablette umfasst; oder
wobei die Schmelztablette ein oder mehrere Bindemittel in einer Menge zwischen 5 und 10 Gew.-% der Schmelztablette umfasst; oder
wobei die Schmelztablette ein oder mehrere Bindemittel in einer Menge von 10 Gew.-% der Schmelztablette umfasst; gegebenenfalls wobei das eine oder die mehreren Bindemittel aus mikrokristalliner Cellulose, z. B. verkieselter mikrokristalliner Cellulose, und Hydroxypropylmethylcellulose ausgewählt sind, bevorzugt wobei die Schmelztablette verkieselte mikrokristalline Cellulose umfasst.

5. Schmelztablette nach einem der Ansprüche 1 bis 4, wobei die Schmelztablette ferner Folgendes umfasst:
a) einen Fließverbesserer; bevorzugt
i) wobei der Fließverbesserer in einer Menge zwischen 0,5 bis 3 Gew.-% der Schmelztablette vorliegt; oder
ii) wobei der Fließverbesserer in einer Menge von 0,75 Gew.-% der Schmelztablette vorliegt;
gegebenenfalls wobei es sich bei dem Fließverbesserer um Siliciumdioxid handelt; und/oder
b) einen oder mehrere Süßstoffe, bevorzugt
wobei der eine oder die mehreren Süßstoffe in einer Menge zwischen 0,1 und 1 Gew.-% der Schmelztablette vorliegen;
gegebenenfalls, wobei der eine oder die mehreren Süßstoffe aus Sucralose, Aspartam, Zuckerderivaten, Dextrose, Polydextrose, Xylit, Fructose, Saccharose, Lactitol, Maltitol und Natriumsaccharin und Gemischen davon ausgewählt sind, bevorzugt wobei die Schmelztablette Sucralose als Süßstoff umfasst; und/oder
c) einen oder mehrere Geschmacksstoffe, bevorzugt
wobei der eine oder die mehreren Geschmacksstoffe in einer Menge zwischen 0,03 und 0,4 Gew.-% der Schmelztablette vorliegen;
gegebenenfalls wobei der eine oder die mehreren Geschmacksstoffe aus Zitronenaroma, Beerenmischungsaroma, Weintraubenaroma, natürlichem Pfefferminzaroma, Minzaroma, Bananenaroma, Schokoladenaroma, Ahornaroma, Erdbeeraroma, einem Himbeeraroma, einem Kirscharoma, Orangenaroma und einem Vanillearoma und Gemischen davon ausgewählt sind, gegebenenfalls wobei die Schmelztablette natürliches Pfefferminzaroma umfasst.

6. Schmelztablette nach einem der Ansprüche 1 bis 5, umfassend eines oder mehrere von granuliertem Mannit, sprühgetrocknetem Mannit verkieselter mikrokristalliner Cellulose, Polyvinylpyrrolidon, z. B. Crospovidon, Sucralose, Siliciumdioxid und natürlichem Pfefferminzaroma.

7. Schmelztablette nach einem der Ansprüche 1 bis 6, wobei KVD900 in seiner Freie-Base-Form vorliegt.

8. Schmelztablette nach Anspruch 7, wobei die Schmelztablette Folgendes umfasst:
a) 30 Gew.-% KVD900;
b) 26,87 Gew.-% granuliertes Mannit;
c) 26,87 Gew.-% sprühgetrocknetes Mannit;
d) 10,01 Gew.-% verkieselte mikrokristalline Cellulose;
e) 5 Gew.-% Crospovidon;
f) 0,5 Gew.-% Sucralose; und
g) 0,75 Gew.-% Siliciumdioxid,
gegebenenfalls ferner umfassend eine Spurenmenge Magnesiumstearat.

9. Schmelztablette nach Anspruch 8, umfassend 300 mg KVD900, 150 mg KVD900 oder 75 mg KVD900.

10. Schmelztablette nach Anspruch 9, umfassend 300,00 mg KVD900, 268,70 mg granuliertes Mannit, 268,70 mg sprühgetrocknetes Mannit, 100,10 mg verkieselte mikrokristalline Cellulose, 50,00 mg Crospovidon, 5,00 mg Sucralose und 7,50 mg Siliciumdioxid.

11. Verfahren zur Herstellung einer Schmelztablette nach einem der Ansprüche 1 bis 10, aus einem Gemisch, das Folgendes umfasst:
a) zwischen 26 und 40 Gew.-% von KVD900;
b) zwischen 30 und 80 Gew.-% eines oder mehrerer Füllstoffe;
c) zwischen 5 und 40 Gew.-% eines oder mehrerer Bindemittel;
d) zwischen 1 und 10 Gew.-% eines oder mehrerer Sprengmittel,
wobei das Verfahren Vermischen der Komponenten des Gemischs unter Bildung eines Pulvergemischs und Verpressen zur Schmelztablette umfasst.

12. Verfahren gemäß Anspruch 11, wobei das Gemisch Folgendes umfasst:
a) 30 Gew.-% KVD900;
b) 26,85 Gew.-% granuliertes Mannit und 26,85 Gew.-% sprühgetrocknetes Mannit;
c) 10 Gew.-% verkieselte mikrokristalline Cellulose;
d) 5 Gew.-% Polyvinylpyrrolidon, z. B. Crospovidon.

13. Schmelztablette nach einem der Ansprüche 1 bis 10 zur Verwendung bei einem Verfahren zur Bedarfsbehandlung eines durch Bradykinin vermittelten Angioödems, wobei das Verfahren Folgendes umfasst: Verabreichen einer oder mehrerer der Schmelztabletten an einen dies benötigenden Patienten nach Bedarf.

14. Schmelztablette zur Verwendung nach Anspruch 13, wobei:
a) es sich bei dem durch Bradykinin vermittelten Angioödem um hereditäres Angioödem (HAE) handelt; oder wobei es sich bei dem durch Bradykinin vermittelten Angioödem um Bradykinin-vermitteltes Angioödem, nicht hereditär (BK AEnH) handelt; und/oder
b) der Patient unter einem laryngealen Anfall leidet; und/oder
c) dem Patienten das Schlucken ganzer Tabletten schwerfällt.

15. Schmelztablette zur Verwendung nach einem der Ansprüche 13 bis 14, wobei KVD900 oder ein pharmazeutisch unbedenkliches Salz und/oder Solvat davon wie folgt verabreicht wird:
a) in einer Dosierungsmenge von 500 mg,
gegebenenfalls wobei die Dosierungsmenge jeweils in zwei 250 mg KVD900 umfassende Schmelztabletten unterteilt ist; oder
b) in einer Dosierungsmenge von 550 mg,
gegebenenfalls wobei die Dosierungsmenge jeweils in zwei 275 mg KVD900 umfassende Schmelztabletten unterteilt ist; oder
c) in einer Dosierungsmenge von 600 mg,
gegebenenfalls wobei die Dosierungsmenge jeweils in zwei 300 mg KVD900 umfassende Schmelztabletten unterteilt ist.

## Revendications

1. Comprimé orodispersible comprenant entre 26 et 50 % en poids de KVD900, ou un sel et/ou solvate pharmaceutiquement acceptable de celui-ci,
où KVD900 est,

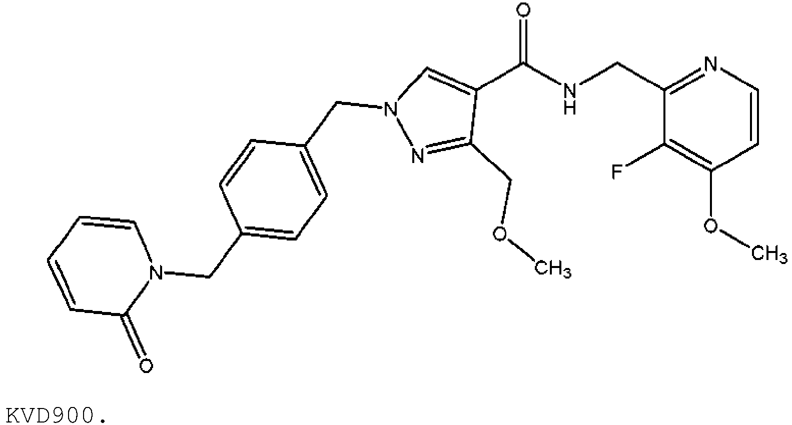

2. Comprimé orodispersible selon la revendication 1, comprenant :
entre 100 mg et 1 000 mg de KVD900, ou d'un sel et/ou solvate pharmaceutiquement acceptable de celui-ci ; ou
entre 200 mg et 350 mg de KVD900, ou d'un sel et/ou solvate pharmaceutiquement acceptable de celui-ci ; ou
250 mg de KVD900, ou d'un sel et/ou solvate pharmaceutiquement acceptable de celui-ci ; ou
275 mg de KVD900, ou d'un sel et/ou solvate pharmaceutiquement acceptable de celui-ci ; ou
300 mg de KVD900, ou d'un sel et/ou solvate pharmaceutiquement acceptable de celui-ci.

3. Comprimé orodispersible selon l'une quelconque des revendications 1 à 2, dans lequel le comprimé orodispersible comprend entre 26 et 40 % en poids de KVD900, ou d'un sel et/ou solvate pharmaceutiquement acceptable de celui-ci,
de préférence, le comprimé orodispersible comprenant 30 % en poids de KVD900, ou d'un sel et/ou solvate pharmaceutiquement acceptable de celui-ci.

4. Comprimé orodispersible selon l'une quelconque des revendications 1 à 3, le comprimé orodispersible comprenant :
a) un ou plusieurs désintégrants présents à raison d'entre 1 et 15 % en poids du comprimé orodispersible ; ou
un ou plusieurs désintégrants présents à raison d'entre 1 et 10 % en poids du comprimé orodispersible ; ou
un ou plusieurs désintégrants présents à raison d'entre 4 et 5 % en poids du comprimé orodispersible ; ou
un ou plusieurs désintégrants présents à raison de 5 % en poids du comprimé orodispersible ;
facultativement, le ou les désintégrants étant choisis parmi (i) la polyvinylpyrrolidone, par exemple la crospovidone, (ii) le glycolate d'amidon sodique et (iii) la croscarmellose sodique, le comprimé orodispersible comprenant de préférence de la polyvinylpyrrolidone, par exemple la crospovidone ;
et/ou
b) une ou plusieurs charges présentes à raison d'entre 30 et 80 % en poids du comprimé orodispersible ; ou
dans lequel le comprimé orodispersible comprend une ou plusieurs charges présentes à raison d'entre 50 et 60 % en poids du comprimé orodispersible ; ou
dans lequel le comprimé orodispersible comprend une ou plusieurs charges présentes à raison d'entre 53 et 54 % en poids du comprimé orodispersible ;
facultativement, la ou les charges étant choisies parmi (i) l'isomalt, (ii) le lactose et ses dérivés, par exemple le lactose monohydraté, le lactose séché par pulvérisation, le lactose anhydre, (iii) le sorbitol, (iv) le mannitol, par exemple le mannitol granulé, le mannitol en poudre, le mannitol séché par pulvérisation, (v) le saccharose, (vi) l'amidon, (vii) l'amidon prégélatinisé, et (viii) des mélanges de ceux-ci, de préférence dans lequel le comprimé orodispersible comprend une ou plusieurs charges choisies parmi le mannitol granulé et le mannitol séché par pulvérisation ; et/ou
c) un ou plusieurs liants présents à raison d'entre 5 et 40 % en poids du comprimé orodispersible ; ou
dans lequel le comprimé orodispersible comprend un ou plusieurs liants présents à raison d'entre 5 et 30 % en poids du comprimé orodispersible ; ou
dans lequel le comprimé orodispersible comprend un ou plusieurs liants présents à raison d'entre 5 et 10 % en poids du comprimé orodispersible ; ou
dans lequel le comprimé orodispersible comprend un ou plusieurs liants présents à raison de 10 % en poids du comprimé orodispersible ;
facultativement, le ou les liants étant choisis parmi la cellulose microcristalline, par exemple la cellulose microcristalline silicifiée, et l'hydroxypropylméthylcellulose, de préférence, le comprimé orodispersible comprenant de la cellulose microcristalline silicifiée.

5. Comprimé orodispersible selon l'une quelconque des revendications 1 à 4, dans lequel le comprimé orodispersible comprend en outre :
a) un améliorateur d'écoulement ; de préférence
i) dans lequel l'améliorateur d'écoulement est présent à raison d'entre 0,5 et 3 % en poids du comprimé orodispersible ; ou
ii) dans lequel l'améliorateur d'écoulement est présent à raison de 0,75 % en poids du comprimé orodispersible ; facultativement, dans lequel l'améliorateur d'écoulement est du dioxyde de silicium ;
et/ou
b) un ou plusieurs édulcorants, de préférence
dans lequel le ou les édulcorants sont présents à raison d'entre 0,1 et 1 % en poids du comprimé orodispersible ; facultativement, le ou les édulcorants étant choisis parmi sucralose, aspartame, dérivés de sucre, dextrose, polydextrose, xylitol, fructose, saccharose, lactitol, maltitol, et saccharine sodique et leurs mélanges, de préférence dans lequel le comprimé orodispersible comprend du sucralose comme édulcorant ;
et/ou
c) un ou plusieurs arômes, de préférence
dans lequel le ou les arômes sont présents à raison d'entre 0,03 et 0,4 % en poids du comprimé orodispersible ;
facultativement, le ou les arômes étant choisis parmi un arôme de citron, un arôme de baies mélangées, un arôme de raisin, un arôme naturel de menthe poivrée, un arôme de menthe, un arôme de banane, un arôme de chocolat, un arôme d'érable, un arôme de fraise, un arôme de framboise, un arôme de cerise, un arôme d'orange, un arôme de vanille et des mélanges de ceux-ci, le comprimé orodispersible comprenant éventuellement un arôme naturel de menthe poivrée.

6. Comprimé orodispersible selon l'une quelconque des revendications 1 à 5, comprenant un ou plusieurs parmi le mannitol granulé, le mannitol séché par pulvérisation, la cellulose microcristalline silicifiée, la polyvinylpyrrolidone, par exemple la crospovidone, le sucralose, un dioxyde de silicium et un arôme naturel de menthe poivrée.

7. Comprimé orodispersible selon l'une quelconque des revendications 1 à 6, dans lequel KVD900 est sous sa forme de base libre.

8. Comprimé orodispersible selon la revendication 7, dans lequel le comprimé orodispersible comprend :
a) 30 % en poids de KVD900 ;
b) 26,87 % en poids de mannitol granulé ;
c) 26,87 % en poids de mannitol séché par pulvérisation ;
d) 10,01 % en poids de cellulose microcristalline silicifiée ;
e) 5 % en poids de crospovidone ;
f) 0,5 % en poids de sucralose ; et
g) 0,75 % en poids de dioxyde de silicium,
éventuellement comprenant en outre une quantité trace de stéarate de magnésium.

9. Comprimé orodispersible selon la revendication 8, comprenant 300 mg de KVD900, 150 mg de KVD900 ou 75 mg de KVD900.

10. Comprimé orodispersible selon la revendication 9, comprenant 300,00 mg de KVD900, 268,70 mg de mannitol granulé, 268,70 mg de mannitol séché par pulvérisation, 100,10 mg de cellulose microcristalline silicifiée, 50,00 mg de crospovidone, 5,00 mg de sucralose et 7,50 mg de dioxyde de silicium.

11. Procédé de fabrication d'un comprimé orodispersible selon l'une quelconque des revendications 1 à 10, à partir d'un mélange comprenant :
a) entre 26 et 40 % en poids de KVD900 ;
b) entre 30 et 80 % en poids d'une ou plusieurs charges ;
c) entre 5 et 40 % en poids d'un ou plusieurs liants ;
d) entre 1 et 10 % en poids d'un ou plusieurs désintégrants,
le procédé comprenant un mélange des composants du mélange pour former un mélange pulvérulent et une compression pour obtenir le comprimé orodispersible.

12. Procédé selon la revendication 11, le mélange comprenant :
a) 30 % en poids de KVD900 ;
b) 26,85 % en poids de mannitol granulé et 26,85 % en poids de mannitol séché par pulvérisation ;
c) 10 % en poids de cellulose microcristalline silicifiée ;
d) 5 % en poids de polyvinylpyrrolidone, par exemple de crospovidone.

13. Comprimé orodispersible selon l'une quelconque des revendications 1 à 10, pour une utilisation dans un procédé de traitement de l'angiœdème induit par la bradykinine à la demande, ledit procédé comprenant : l'administration d'un ou plusieurs des comprimés orodispersibles à un patient qui en a besoin à la demande.

14. Comprimé orodispersible pour une utilisation selon la revendication 13, dans lequel :
a) l'angiœdème induit par la bradykinine est un angiœdème héréditaire (AOH) ; ou dans lequel l'angiœdème induit par la bradykinine est un angiœdème non héréditaire induit par la bradykinine (BK AEnH) ; et/ou
b) le patient souffre d'une attaque laryngée ; et/ou
c) le patient a de la difficulté à avaler des comprimés entiers.

15. Comprimé orodispersible pour une utilisation selon l'une quelconque des revendications 13 à 14, dans lequel KVD900, ou un sel et/ou solvate pharmaceutiquement acceptable de celui-ci, est administré :
a) à une dose de 500 mg,
facultativement, dans lequel chaque dose est subdivisée en deux comprimés orodispersibles comprenant 250 mg de KVD900 ; ou
b) à une dose de 550 mg,
facultativement, dans lequel chaque dose est subdivisée en deux comprimés orodispersibles comprenant 275 mg de KVD900 ; ou
c) à une dose de 600 mg,
facultativement, dans lequel chaque dose est subdivisée en deux comprimés orodispersibles comprenant 300 mg de KVD900.
